# EUROPEAN PATENT APPLICATION

(11) **EP 3 437 650 A1**
(43) Date of publication of application: **06.02.2019**
(21) Application number: 17183990.5
(22) Date of filing: 31.07.2017
(51) Int. Cl.: A61K 38/18, A61P 17/00, A61P 17/02

(54) **TREATMENT OF LOCAL SKIN HYPOTROPHY CONDITIONS**

(71) Applicant: Accanis Biotech F&E GmbH & Co KG, 1030 Vienna (AT)
(72) Inventor: MANDLER, Markus, 1030 Vienna (AT); MATTNER, Frank, 1030 Vienna (AT); SCHMIDT, Walter, 1030 Vienna (AT); SCHNEEBERGER, Achim, 1030 Vienna (AT)
(74) Representative: Sonn & Partner Patentanwälte

(57) **Abstract**

The invention discloses Fibroblast growth factor 2 (FGF2) and Fibroblast growth factor 7 (FGF7) messenger-RNA (mRNA), wherein the mRNA has a 5' CAP region, a 5' untranslated region (5'-UTR), a coding region, a 3' untranslated region (3'-UTR) and a poly-adenosine tail (poly-A tail), for use in the treatment of local skin hypotrophy conditions and kits for administrating this mRNA to a human patient in need thereof.

## Description

The present invention relates to compositions and methods for the treatment of local skin hypotrophy conditions characterized by an hypotrophic state of one or several skin layers, especially atrophic skin conditions and atrophic scars.

The present invention relates to compositions and methods for the treatment of skin disease resulting in a hypotrophic state ("hypotrohphy") of one or several skin layers (epidermis, dermis subcutaneous fat), especially atrophic scars, as medical indications, but also skin conditions that are associated with skin ageing, which are generally regarded as cosmetic problems and areas of interest (Fitzpatrick's Dermatology in General Medicine, 6th edition, Editors Freedberg et al., Chapter 144 in Fitzpatrick's Dermatology in General Medicine by Yaar et al.: Aging of Skin; Photodamaged skin, editor: Goldberg, chapter 5 by Wheeland: Nonmalignant clinical manifestations of photodamage; Skin Aging editors: Gilchrest et al., Springer, ISBN-10 3-540-24443-3; Therapie der Hautkrankheiten, Editors: Orfanos et al.).

Hypotrophy of skin and atrophic scarring are widely prevalent skin conditions. Specific diseases that are associated with hypotrophy/atrophy of the skin include cutis laxa, acrodermatitis chronica atrophicans, atrophodermia idiopathica et progressiva Pasini Pierini, scars resulting from perforating dermatoses, atrophy blanche, necrobiosis lipoidica, radiation dermatitis (skin changes resulting from exposure to ionizing radiation), striae cutis distensae (caused by pregnancy, rapid growth, alimentary obesity). The spectrum extends from small reductions in the volume of given skin layers to a complete tissue loss, its maximal variant. Tissue loss could be restricted to the epidermal compartment (i.e. erosion) or could include the dermal and/or subcutaneous compartment, then called ulcer. Causes for skin ulcers are numerous and include trauma, autoimmunological pathology, reduced perfusion (arterial or venous), psychogenic injury (self-trauma), infections. Specific diseases include ulcerative dermatitis as a result of bacterial infection, ulcerative sarcoidosis, ulcerative lichen planus, diabetic foot ulcer, ulcer associated with high blood pressure, ulcer associated with venous insufficiency, neuropathic ulcer, pressure sore, vasculitis (small and medium size vessels), pyoderma gangraenosum, rheumatioid ulceration, necrobiosis lipoidica, drug-induced necrosis (e.g. caused by warfarin, heparin), ulcers in the context of coagulopathies (e.g. caused by antiphospholipid syndrome, protein S deficiency).

Atrophic scars manifest primarily on the face, chest and back of patients. It most commonly results from acne and chick-enpox, but also from surgery, infections, drugs (e.g. steroid injection, penicillamine), autoimmunological processes (e.g. chronic discoid lupus erythematosus), trauma, nerve- and psychogenic (e.g. acne excoriée de jeunes filles) injuries. Hypotrophic/atrophic areas do not constitute an acute/active wound or active site of tissue remodeling but constitute an end-result of inefficient and aberrant repair mechanisms following a local tissue insult. In case of atrophic scars, the decreased dermal volume results in a downward pull of the affected skin area causing a sunken appearance.

Cutaneous aging falls into two categories: intrinsic (occurs with the passage of time and is predetermined by genetic predisposition) and extrinsic (largely the result of chronic sun damage (UV B and UV A); other contributing extrinsic factors include infrared radiation and tobacco smoke) aging. Clinical manifestations include dryness (roughness), irregular pigmentation, wrinkling/rhytides, dermatohelios (severe deep wrinkling), leathery skin appearance, stellate pseudoscars, fine nodularity, inelasticity, teleangiectasias, purpura (easy bruising), comedo-nes (maladies Favre et Racouchot) sebaceous hyperplasia and neo-plasias (e.g., actinic keratosis, basal cell carcinoma, squamous cell carcinoma). Pathophysiologically, skin hypo-/atrophy exhibits characteristic changes within the epidermal, dermal or subcutaneous compartment. Pathological changes in the former includes little change in epidermal thickness, keratinocyte shape and corneocyte cohesion. Major change is observed in the dermoepidermal junction, which is flattened resulting in a reduced surface contact between the epidermis and the dermis (reduced exchange of nutrients and metabolites). The dermis is hy-pocellular with reduced numbers of fibroblasts, mast cells and reduced dermal volume. Collagen fibers become loose and there is a moderate thickening of elastic fibers. In addition, there is a decrease in blood vessels with an associated shortening of capillary loops.

Early and effective treatment of hypotrophic and atrophic tissue is thus paramount for both, medical indications and cosmetic areas. An estimated 1% of the adult population has persistent atrophic/hypotrophic scars from adolescence, of which, 1 in 7 is considered to have disfiguring scars. The appearance of atrophic scars worsens with age due to the natural lipoatrophy of the skin. Facial scarring is strongly associated with psychological trauma in many patients, including loss of self-esteem, increased difficulty in obtaining employment and a diminished quality-of-life (QOL). In addition, clinical studies have revealed that facial scarring, particularly in men, has been correlated with depression and suicidal intention.

A broad array of therapeutic interventions, targeting the different skin compartments affected, have been developed over the past few decades to treat localised skin hypotrophy and atrophic scars, most notably application of ablative lasers, autologous fat transfer, and chemical peelings (reviewed in Patel et al., J. Roy. Soc. Med. Open 5 (2014), 1-13; DOI: 10.1177/2054270414540139. However, these treatments are highly-operator dependent and may have long recovery times (reviewed in Hession et al., J. Clin. Aesthet. Dermatol. 8 (2015), 50-58. Although laser therapy is considered to be the gold standard for treatment, it is usually not effective enough on its own to achieve adequate results for disfiguring scars. In reality, patients usually require the use of several different treatment techniques over multiple session in order to achieve the desired effect, increasing time and treatment costs. Therefore, there is a high unmet medical need for a potent, inexpensive atrophic scar treatment modality.

Current therapeutic interventions for hypotrophic skin areas and atrophic scars are not effective enough on their own to achieve adequate results, requiring the use of multiple treatment options, increasing time and treatment costs. In addition, most of these therapeutic interventions rely on iatrogenic wounding of the skin area to be treated, including treatment with ablative lasers and the various peeling procedures. All of them finally trigger the cascade of events generally known as wound healing processes. For obvious reasons, their efficacy is limited by the biological age of a given individual.

To increase the results of the treatment, often various approaches are combined to relatively complex treatment regimens. For example, Kang and colleagues presented a triple combination therapy combining dot peeling and subcision, done twice 2-3 months apart, and fractional laser irradiation, which was performed every 3-4 weeks, to obtain an approximately 55% improvement in scar severity. A double combination included a derma roller and 15% TCA peel performed alternatively at 2-weeks interval for 6 sessions to see an improvement of 1 grade on an international scar assessment scale. Obviously, rational combination therapies are based on the complementarity of the approaches to be combined, and, therefore, limited in their number. Moreover, their scientific and clinical evaluation is complex and their efficacy appears to be limited for technical and biological reasons.

Beside these applications, there is a long list of trials trying to topically apply different growth factors (GFs), also including fibroblast growth factors like FGF2 and FGF7, as proteins or DNA expression plasmids in order to enhance skin repair in situations with ulcers/erosions due to disease processes or following wounding or iatrogenic measures such as treatment with ablative lasers, dermabrasion, etc. These GFs (including platelet derived growth factor (PDGF), vascular endothelial growth factor (VEGF), transforming growth factor-β (TGF-β), epidermal growth factor (EGF), granulocyte colony-stimulating growth factor (G-CSF), keratinocyte growth factor (KGF), interleukin 6 (IL-6), interleukin 8 (IL-8), and hepatocyte growth factor (HGF) etc.) are normally involved in various steps of wound healing. Studies done to elucidate their potential in wound healing and tissue remodeling in experimental preclinical systems and patients demonstrated only limited success.

For example, application of growth factors as proteins showed only very limited efficacy in vivo due to various reasons. GFs are secreted proteins with short half-lives. In addition to this highly limiting factor, it has been shown by extensive analyses that only a fraction of topically applied GFs is able to penetrate the skin, possibly via sweat glands in the in-terfollicular stratum corneum. A clinical study investigated dermal remodeling following twice daily application of GF serum (NouriCel-MD, TNS recovery complex, Allergan, containing cell culture medium collected from a line of dermal fibroblasts originating from neonatal foreskin) for 60 days. It was found that 78.6% of patients showed clinical improvement after 60 days. Processing of punch biopsies demonstrated that only 37% of patients with clinical improvement were found to have new collagen formation and even less, 27% of the treated population, showed epidermal thickening (Fitzpatrick et al., J. Cosmet. Laser Ther. 5 (2003), 25-34). A second vehicle controlled, double-blind study using the same GF serum also showed very limited improvement in fine wrinkles and skin tone (Mehta et al., J. Drugs Dermatol. 7(2008), 864-71).

The preliminary effects of topically applied GFs as proteins suggest that they could have a much greater efficacy on skin remodeling in case their local availability was significantly improved.

US 2014/199367 A1 discloses a topical transdermal method for delivering nutrients (proteins, amino acids, nucleic acids) through the skin for expedited wound healing and skin rejuvenation but does not mention FGFs or any specific nucleic acids.

WO 2012/106508 A1 discloses a method for treating and/or preventing keloids or hypertrophic scars by injecting modified oligonucleotides into the affected injured skin.

WO 00/59424 A1 discloses grafting of a porous pad comprising wound healing factors such as growth factors, e.g. bFGF, for promoting wound healing in mammals.

US 2012/264690 A1 discloses a method for preventing incisional hernia formation and acute wound failure by administering a composition comprising basic fibroblast growth factor.

US 2013/0095061 A1: discloses a composition for application to the skin for the prevention or treatment of an adverse skin condition, the composition comprising elastase 2A; prostaglandin I2; prostaglandin E2; amphiregulin; fibroblast growth factor 2; fibroblast growth factor 7; G protein-coupled receptor, family C, group 5, member B; and GABA(a) receptor-associated protein like 1.

WO 2016/100820 A2 discloses a method of reducing blood glucose by administering mutated FGF2 protein or nucleic acids encoding the mutated FGF2 protein or a vector comprising the nucleic acid for the treatment of metabolic diseases e.g. diabetes.

Dou Chunqing et al. (Mol Ther. 22(4)(2014): 752-761) describes a gene delivery approach to strengthen structurally fragile skin, e.g. atrophic skin in paraplegic patients or healed chronic wounds in diabetic patients, by topical application of a DNA plasmid expressing KGF-1 (FGF7) after microdermabrasion.

Marti et al. (Gene Ther. 11(24)(2004): 1780-5) discloses improved and accelerated wound healing of cutaneous wounds in diabetic mice upon intradermal injection of KGF-1 plasmid DNA in combination with electroporation.

Lin et al. (Wound Repair Regen. 14(5)(2006): 618-24) discloses improved and accelerated wound healing of cutaneous wounds in septic rats upon intradermal injection of KGF-1 plasmid DNA in combination with electroporation.

US 2010/221232 A1 discloses compositions for osteogenic gene therapy increasing bone growth and enhancing wound healing, e.g. fracture repair, the compositions comprising recombinant nucleic acids encoding FGF-2.

None of the GFs analysed and tested so far, including FGF2 and FGF7, are currently used in clinical practice to treat wounds or, as suggested by the current invention, hypotrophic skin and atrophic scars.

These unimpressively low success rates of recombinant protein and DNA based therapies so far in wound healing as well as the lack of strategies for treatment of hypotrophic skin and atrophic scars warrant the development of novel treatment paradigms to meet the medical need in the treatment of hypotrophic skin and atrophic scars.

It is an object of the present invention to provide improved treatment methods for local skin hypotrophy, atrophic skin conditions and atrophic scars which (at least partially) overcome the limitations of current treatment options summarized above. It is a further object that such improved treatment schemes provide improved patient's compliance and improved adverse reaction events. A further object of the present invention is to provide methods which are easily reversible and do not have severe impact on the patient's body as a whole (i.e. (adverse) systemic consequences due to the treatment). Moreover, it is a desire to provide growth factor treatment without the normally accompanied burden for the patients and to increase treatment efficiency, responder rates and patient compliance.

Therefore, the present invention provides Fibroblast growth factor (FGF) messenger-RNA (mRNA), wherein the mRNA has a 5' CAP region, a 5' untranslated region (5'-UTR), a coding region, a 3' untranslated region (3'-UTR) and a poly-adenosine Tail (poly-A Tail), for use in the treatment of local skin hypotrophy conditions, especially atrophic skin conditions, wherein the FGF mRNA encodes for FGF2 and/or FGF7.

The present invention aims to treat and prevent the indications listed by activating a specific milieu that normally stimulates skin repair only in an acute remodeling process, for example due to wounding, inflammation or burns. Importantly, the tissue area aimed to be treated is devoid of these activating and remodeling stimuli before application of the intended treatment modality and is considered to be healthy skin.

The present invention allows e.g. local administration on or into the skin of patients suffering from local skin hypotrophy conditions, especially atrophic skin conditions.

The first fibroblast growth factor (FGF) ligands, FGF1 and FGF2, were initially purified from brain as mitogenic factors of fibroblasts grown in culture. Since their discovery, FGF ligands and their receptors have been implicated in numerous biological processes, and their dysregulation causes several congenital diseases (such as dwarfism) and some types of cancer. In addition to their mitogenic capacity, FGFs can also modulate cell survival, migration and differentiation in culture. Members of the FGF family of extracellular ligands are characterised by a conserved core of 140 amino acids and their strong affinity for heparin sulphate (HS). The molecular weight of FGFs range from 17 to 34 kDa in vertebrates, whereas it reaches to 84 kDa in Drosophila. In vertebrates, 22 family members have been identified and are grouped into seven subfamilies according to their sequence homology and function (Ornitz, BioEssays 22 (2000), 108-112); e.g.: FGF1 and FGF2 (FGF1 subfamily); FGF4, FGF5 and FGF6 (FGF4 subfamily); FGF3, FGF7, FGF10 and FGF22 (FGF7 subfamily); FGF8, FGF17 and FGF18 (FGF8 subfamily); FGF9, FGF16 and FGF20 (FGF9 subfamily); FGF11, FGF12, FGF13 and FGF14 (FGF11 subfamily); FGF19, FGF21, and FGF23 (FGF19 subfamily)). All FGFs, with the exception of the intracellular FGFs 11-14, signal through a family of tyrosine kinase receptors, the FGF receptors (FGFRs). Secreted FGF ligands bind the extracellular domain of the FGFRs and this leads to dimerisation of the receptor (FGF-FGFR-HS dimers) resulting in the transphosphorylation of intracellular tyrosine residues. This triggers the activation of cytoplasmic signal transduction pathways, such as the Ras/ERK/MAPK pathway (which is associated with proliferation and differentiation), the Akt pathway (associated with cell survival) or the protein kinase C (PKC) pathways (involved in cell morphology and migration). Except for one subfamily, all FGFs exhibit high-affinity binding to heparans and HS, and thus exert paracrine control in areas adjacent to their secretion. Only the FGF-19 subfamily, including FGF-21, FGF-23, and FGF-19 in humans and the mouse FGF-19 equivalent, FGF-15, acts in an endocrine fashion and require the Klotho gene family of transmembrane proteins as coreceptors to bind their cognate FGF receptors and exert their biological activities.

FGF7, also known as Keratinocyte growth factor (KGF, FGF7), was first isolated as an epithelial mitogen from the conditioned medium of human embryonic lung fibroblasts. FGF7 is primarily produced by cells of mesenchymal origin and is well known to strongly activate FGFR2b, so far, no other activity towards any other FGFR isoform has been detected. The restricted pattern of FGFR2b expression primarily in epithelial cells and the high specificity of FGF7 for this FGFR isoform support the hypothesis that they function as paracrine signals mediating mesenchymal-epithelial communication. Loss of function mutations of FGF7 in mice only leads to minor alterations in hair characteristics, kidney development and urothelial stratification in the bladder, indicating that FGF7/KGF does not play a critical role in developmental organogenesis. Experiments analysing FGF7 function rather imply, that the main role of FGF7/KGF is centered around regulation of epithelial preservation and/or repair processes. The beneficial effects described for FGF7/KGF activity in acute insults arises from multiple mechanisms that act synergistically to strengthen tissue integrity (mainly epithelium) by stimulating processes like cell proliferation, - migration, - differentiation, and - survival. Accordingly, FGF7/KGF has been the subject of intensive efforts to identify clinical applications in which the preservation or rapid restoration of epithelial tissues would be of benefit. Currently, a truncated form of recombinant FGF7/KGF (palifermin, marketed as Kepivance) has been approved for the treatment of severe oral mucositis in patients with hematologic malignancies prior to autologous blood progenitor cell transplantation.

FGF7/KGF was also shown to be upregulated following tissue damage in skin (mouse and human full-thickness wounds). Based upon such observations, experiments were performed with FGF7/KGF in animals to determine whether its topical application to the skin could stimulate epidermal wound repair. Importantly, the magnitude of these effects excerted by protein application was not considered sufficient to warrant clinical development.

Fibroblast growth factor 2 (FGF2), a prototypic member of the FGF family, is encoded by a single gene. However, alternative translation-initiation codons and polyadenylation signals produce various isoforms (Touriol et al., Biol. Cell. 95 (2003), 169-178). Low molecular weight FGF2 (Lo FGF2) is an 18 kDa protein translated from a conventional AUG start codon and its 155 amino acid sequence is common to all FGF2 isoforms (Ibrahimi et al. 2004). Alternative isoforms (e.g.: 20.5 and 21 kDa) are produced from CUG sites upstream and inframe of the AUG codon. These forms are localized in the nucleus and are responsible for the intracrine effect of FGF2. The RNA Sequence in the CDS of the 18kd form of FGF2 is 468 nucleotides and is an integral part of the naturally occurring full length mRNA sequence as disclosed in a publicly available database with the accession numbers: J04513.1 (https://www.ncbi.nlm.nih.gov/nuccore). Alternatively, the sequence is also disclosed in NM_002006.4 and M27968.1, respectively. The 21.5kd form has 591 nucleotides in the CDS and encodes 196aa and the CDS of the 22kd form has 633 nucleotides encoding for 210aa. Usage of these CDS variants would also lead to secretion of the short form of FGF2 described in this invention.

The isoforms of FGF2 are expressed in fixed molar ratios that are dependent on tissue type. These ratios are translationally regulated as overexpression of the human FGF2 gene in mice does not alter these ratios in the human FGF2 protein products. High MW FGF2 and low MW FGF2 can potentially associate reciprocally, modulating each other's biological activity depending on their relative ratios and/or localization. FGF2 isoforms are localized differentially and display different gene expression profiles. In response to ischemia/reperfusion (I/R) injury only low MW FGF2 can be released from cardiac cells further indicating the intracrine localization of high MW FGF2. Exported low MW FGF2 binds to the FGF receptor (FGFR) extracellularly and its binding is modulated by nonsignaling heparin/heparan sulphate proteoglycans that are subsequently involved in the intracellular processing of FGF2.

Alternative sequences for FGF2 which have been proposed for use also include changes of the CDS: FGF2 is lacking a standard secretion signal and export is mediated by an energy-dependent, non-ER/Golgi pathway. Hence, adding of alternative secretion signals within the FGF2 coding sequence have been suggested to increase secretion and are included: Sohn et al. (Sohn et al., Biochem Biophys Res Commun 284 (2001): 931-936.) use a classical secretion signal sequence of FGF-4, Sasada et al. (Ann NY Acad Sci 638 (1991): 149-160) use the secretion signal sequence of IL-2 (Blam et al., Oncogene 3 (1988): 129-136) use the secretion signal sequence of Growth hormone;

In addition, also mutation of the second and third of the four cysteines (i.e., cys-70 and cys-88) to serine and asparagine have been suggested to increase protein stability without affecting the biological activity of FGF2. Accordingly, also a combination of mutations with altered secretion sequences as mentioned above has been suggested as well. Along these lines: Chen et al. (J. Cell. Biochem. 100 (2007) : 1493-1508 and references therein) suggest the use of BMP, specifically BMP-2/4 hybrid secretion signal sequences along with mutation of the second and third of the four cysteines (i.e., cys-70 and cys-88) to serine and asparagine to increase stability and secretion of the mutated FGF2 protein.

FGF2 is generally considered a potent mitogen and chemoat-tractant for different cell types, including endothelial cells, fibroblasts and keratinocytes. Among other proposed functions it has been implied that FGF2 stimulates metabolism, can regulate the extracellular matrix (ECM), and also influences the movement of mesoderm-derived cells. FGF2 function is also required for limb and nervous system development and also promotes tumour growth.

Along these lines, several experiments have been performed analysing the potential applicability of FGF2 for wound healing. The administration of recombinant FGF2 to skin wounds could accelerate acute and chronic wound healing in preclinical models. In addition, bFGF-knockout mice showed a delayed healing of skin wounds, which indicates that FGF2 signalling could also be involved in normal wound healing in vertebrates. A potential function in processes other than acute wound healing and associated remodelling however remains elusive.

In general, the clinical success of growth factors administered as recombinant proteins to enhance wound repair and skin remodelling has been disappointing, despite their apparent efficacy in animal studies. While application of FGF2 and FGF7 has been shown to be effective in acute wound treatment in the preclinical arena, it never became a therapeutic option for clinical routine. The reasons are obvious.

Treatment requires frequent (daily with single or multiple applications per day) perilesional injections/applications over several weeks. Also, recombinant protein was expensive and the quality and importantly bioactivity of the preparations differed. FGFs were produced by recombinant DNA technology using genetically engineered E. coli strains requiring extensive purification and potency/bioactivity testing in order to provide a comparable level of bioactivity for treatment with varying degree of non-active and active protein in the product.

Furthermore, it has been postulated that the bioactivity of the recombinant proteins is rapidly diminished because of elevated concentrations of matrix metalloproteinases (MMPs) and other myeloid cell derived proteinases present in the wound environment. Furthermore, bolus administration does not keep the protein localized to the wound area and necessitates large amounts of the growth factor (s) that may have harmful side effects such as vascularization of non-target tissues or stimulation of tumour growth. Analysis of two pharmacologically active FGFs, FGF7 and FGF2, respectively, also supports this inability of recombinant protein to act as useful therapeutics (very high doses and limited efficacy due to very fast elimination). The pharmacokinetics of the FGF7 protein Kepivance were studied in healthy subjects and patients with hematologic malignancies. After single intravenous doses of 20-250 µg/kg in healthy subjects and 60µg/kg in cancer patients, Kepivance concentrations declined over 95% in the first 30 minutes post-dosing. The elimination half-life was similar between healthy subjects and cancer patients with an average of 4.5 hours (range: 3.3 to 5.7 hours). No accumulation of Kepivance occurred after 3 consecutive daily doses of 20 and 40 µg/kg in healthy subjects or 60 µg/kg in cancer patients. Similarly, the recombinant FGF2 preparations show a serum half-life of 2.9 min when injected intravenously (Edelman et al., PNAS 90 (1993), 1513-1517).

Alternatively, pharmaceutically active FGF2 is used as Trafermin, a recombinant form of FGF2, which is marketed as Fiblast Spray. Fiblast Spray, the world's first recombinant human bFGF product, was marketed in Japan in 2001 and has been used in wound healing applications in Japan (decubitus and skin ulcer). The usual regimen includes daily dosing for 4 weeks with 30µg FGF2/6 cm² skin area and exhibits only limited efficacy.

Treatment with DNA/cDNA encoding for growth factors has previously been assessed for its ability to promote wound healing in animal models. However, the effects that growth factors themselves have shown on improving wound repair have so far been inconsistent. In addition, treatments have required repetitive, high doses of the growth factor DNA to achieve statistically significant effects in animal models tested. This low efficacy is also uncovering an intrinsic problem of DNA based nucleic acid therapy: DNA, in order to induce protein expression, does not only need to cross the cell membrane and induce cytoplasmic protein synthesis (as functional IVT mRNA) but also needs to be transported into the nucleus to achieve gene transcription and subsequent translation. In addition to this inefficient process, development of DNA therapies also suffers from the risk of potential, uncontrolled genomic insertion of the DNA molecules and associated alterations including development of cellular abnormalities, apoptosis, mutational insertions, cancerogenesis etc. The combination of the intrinsic high risk and low efficacy profile precluded successful development into the clinical practice so far.

Along these lines, preclinical tests of cDNAs of FGF7/KGF or FGF2 in treatment of acute wounds have been performed. The different wound types analysed included for example burns, excisional wounds, dermabrasion, elevated skin flaps. These experiments were designed to analyse wound closure as well as wound healing by applying full length FGF7 and FGF2 cDNAs, naked or complexed with liposomal preparations, to porcine and rodent wound models.

Naked cDNA injections of KGF/FGF7 have been performed in wound healing experiments. Marti et al. treated full thickness excisional wounds in BALB-C mice with intradermal injections (i.d.) of 50µg KGF/FGF7 DNA in PBS at two injection sites followed by electroporation (Marti et al., Gene Ther. 11 (2004) : 1780-5). Wounds treated with KGF/FGF7 and electroporation were found to have only 38% smaller wound area 12 days after wounding. Notably, treatment with KGF cDNA without electroporation did not show significant improvements in wound healing over the vehicle controls. Similarly, Lin et al. also tested naked KGF DNA and electroporation and similarly found smaller wound area using the same dose (50µg of KGF in PBS at two injection sites) in a rat sepsis model, and also failed to observe significant effects without electroporation (Lin et al., Wound Repair Regen. 14 (2006): 618-24).

Topical application of KGF DNA plasmids has been assessed as a potential treatment for freshly iatrogenically wounded, fragile skin in a murine microdermabrasion model. Treated mice repeatedly received a high amount of plasmid DNA (i.e.: 50µg of plasmid DNA topically every 12 hours over a 4-day period) on an area of microdermal abrasion (Dou et al., Mol Ther. 22 (2014) : 752-61. doi: 10.1038/mt.2014.2). Epithelial thickness in the wounded and transfected areas were significantly increased compared to the control vector group after 48 hours and dermal thickness 120 hours after treatment.

In addition, Jeschke et al. tested a model of acute wound healing using thermal injury by hot water scalding. Here, Sprague-Dawley rats received weekly subcutaneous (s.c.) injections of 2.2µg KGF in liposomes over four weeks or a liposome only control (Jeschke et al., Gene Ther. 9 (2002), 1065-74). In this study, only a very modest acceleration in epithelization was observed as well as an increase in expression of other growth factors, including VEGF and IGF-I, compared to the controls. Additional liposomal gene transfer studies by Jeschke et al. using the same thermal injury model and dosing also showed similar results in Sprague-Dawley rats when treated with Insulin-like growth factor (IGF-I) cDNA, and combinations of KGF and IGF cDNA, respectively. However, initial studies by Jeschke et al. did not address the potential effect of the liposomal formulation itself on wound repair in these scalding models. A study by the same group in 2005 found that s.c. injection of different formulations of liposome alone increased the rate of re-epithelization (Jeschke et al., Gene Ther. 9 12 (2005), 1718-24). Indeed, weekly s.c. injection of 0.2 mL DOTAP/Chol liposomes at two injection sites accelerated epithelization by up to 200% as well as significantly increasing KGF, vascular endothelial growth factor (VEGF), and insulin-like growth factor binding protein 3 (IGFBP-3) levels, compared to saline treated controls. This is highly interesting and points towards very limited efficacy of DNA based therapeutics as liposomal KGF and IGF1 DNA application accelerated wound re-epithelization by almost 250% as reported in a previous study. A 2007 study by Pereira et al. using the same dose and schedule as the Jeschke publications also found a 22% ± 5% improvement in re-epithelization following treatment with liposomes alone, compared to an only modest increase to 40% ± 5% with KGF-I and liposomes (Pereira et al., J. Surg. Res. 139 (2007), 222-8).

For FGF2 DNA based therapeutics only very limited experiments have been described so far addressing wound healing. Fujihara et al. (2005) have shown that transfer of 300µg bFGF plasmid DNA to dorsal muscles and subsequent electroporation improves survival of ischemic skin flaps following flap elevation two days after electroporation mainly by inducing angiogenesis from muscle to overlying skin (Fujihara et al., Br. J. Plast. Surg. 58 (2005) 511-7). No clear effect on skin architecture has been analysed. Similarly, Ferraro et al. (2010) showed that intradermal injection of plasmid DNA encoding FGF-2 (pFGF) followed by non-invasive cutaneous electroporation could increase blood flow and angiogenesis in a rat model of hindlimb ischemia (Ferraro et al., Gene Ther. 17 (2010), 763-9.doi: 10.1038/gt.2010.43). Delivery of pFGF plus electroporation significantly increased FGF-2 expression for 10 days whereas delivery of pFGF without electroporation did not lead to overt improvement. Thus, the main effect associated with cutaneous overexpression of FGF2 has been angiogenesis rather than overt changes in skin architecture other than neoangiogenesis.

Yang et al. (2012) (Mol. Pharm. 9 (2012), 48-58. doi: 10.1021/mp200246b) were applying a full length FGF2 construct (6777 bp), encoding a fusion protein of recombinant murine bFGF and enhanced green fluorescent protein (bFGF/eGFP) within a cytomegalovirus promoter expression vector (C-terminal eGFP-tagged protein). In this study, diabetic rats carrying full thickness excisional wounds were treated with fibrous mats covering the wound area and containing FGF2 plasmid PEI polyplexes as component of the fibres, mats soaked in FGF2 plasmid PEI polyplexes, with fibrous mats alone or were left untreated. Wound closure studies showed that the FGF2 receiving groups developed higher healing rates as compared to non FGF2 treated groups. These effects, although statistically significant, however were modest: control animals showed a wound closure of 67% after 3 weeks when left untreated, whereas fibrous mats were increasing this rate to 78%. Loading of FGF2 plasmid on such mats was leading to an increase to 87%, and integration of the plasmid into the fibers upon production was leading to almost complete wound closure at this time point.

As already summarised above, the success rates of recombinant protein and DNA based therapies in wound healing were very low so far. Moreover, there was a lack of strategies for treatment of hypotrophic skin and atrophic scars which warrants the development of novel treatment paradigms to meet the medical need in the treatment of hypotrophic skin and atrophic scars.

The mRNA used in the present invention contains (at least) five essential elements which are all known and available to a person skilled in the art (in this order from 5' to 3'): a 5' CAP region, a 5' untranslated region (5'-UTR), a coding region for FGF2 or FGF7, a 3' untranslated region (3'-UTR) and a poly-adenosine tail (poly-A tail). The coding region should, of course encode a (human) FGF2 or FGF7, the other components may be the (native) FGF2 or FGF7 UTRs or, preferably, other UTRs. Specifically preferred UTRs according to the present invention are UTRs which improve the properties of the mRNA molecule according to the present invention, i.e. by effecting better and/or longer and/or more effective translation of the mRNA into FGF2 and/or FGF7 protein at the site of administration.

A "CAP region" ("5'CAP") refers to a structure found on the 5' end of an mRNA molecule and generally consists of a guanosine nucleotide connected to the mRNA via an unusual 5' to 5' triphosphate linkage. This guanosine nucleotide is methylated on the 7-position directly after capping in vivo by a methyl transferase ("7-methylguanylate cap" ("m7G"), "cap-0"). Further modifications include the possible methylation of the 2' hydroxy-groups of the first two ribose sugars of the 5' end of the mRNA (i.e. "CAP1" and "CAP2"): "CAP1" has a methylated 2'-hydroxy group on the first ribose sugar, while "CAP2" has methylated 2'-hydroxy groups on the first two ribose sugars. The 5' cap is chemically similar to the 3' end of an RNA molecule (the 5' carbon of the cap ribose is bonded, and the 3' unbonded). This provides significant resistance to 5' exonucleases and is therefore also providing stability in vivo. For generation of mRNAs according to the present invention also CAP analogues may be used including: monomethylated CAP analogue (mCAP), Anti-Reverse Cap Analog (ARCA CAP), m7G(5')ppp(5')A RNA CAP structure analog, G(5')ppp(5')A RNA CAP structure analog, and G(5')ppp(5')G RNA CAP structure analog.

The term "(5'- or 3'-) UTR" refers to the well-established concept of untranslated region of a mRNA in molecular genetics. There is one UTR on each side of a coding sequence on a strand of mRNA. The UTR on the 5' side, is the 5'-UTR (or leader sequence), the UTR on the 3' side, is the 3'-UTR (or trailer sequence). The 5'-UTR is upstream from the coding sequence. Within the 5'-UTR is a sequence that is recognized by the ribosome which allows the ribosome to bind and initiate translation. The mechanism of translation initiation differs in prokaryotes and eukaryotes. The 3'-UTR is found immediately following the translation stop codon. The 3'-UTR plays a critical role in translation termination as well as post-transcriptional gene expression. The UTRs as used in the present invention are usually delivering beneficial stability and expression (translation) properties to the mRNA molecules according to the present invention. The 3' end of the 3'-UTR also contains a tract of multiple adenosine monophosphates important for the nuclear export, translation, and stability of mRNA. This so-called poly-Adenosine (poly-A) tail consists of at least 60 adenosine monophosphates, preferably 100 and most preferably 120 adenosine monophosphates.

The "poly-A tail" consists of multiple adenosine monophosphates; it is a part of naturally occurring mRNA that has only adenine bases. This process called "polyadenylation" is part of the process that produces mature messenger RNA (mRNA) for translation in the course of gene expression. The natural process of polyadenylation begins as the transcription of a gene terminates. The 3'-most segment of the newly made pre-mRNA is first cleaved off by a set of proteins; these proteins then synthesize the poly (A) tail at the RNA's 3' end. In some genes, these proteins add a poly (A) tail at one of several possible sites. Therefore, polyadenylation can produce more than one transcript from a single gene (alternative polyadenylation), similar to alternative splicing. The poly(A) tail is important for the nuclear export, translation, and stability of mRNA. For the present invention, it is therefore mainly the translation and stability properties that are important for a sufficient polyadenylation of the mRNA molecules according to the present invention. During the protein generation, the tail is shortened over time, and, when it is short enough, the mRNA is enzymatically degraded. The poly-A tail according to the present invention is provided in the manner currently used and applied in the art of administering mRNA molecules in human therapy. For example, the poly-A tail may be at least 60 adenosine monophosphates long. According to a preferred embodiment, the poly-A tail is at least 100 adenosine monophosphates long, especially at least 120 adenosine monophosphates. This allows excellent stability and protein generation; however, as for the other features, the action and activity of the mRNA molecule according to the present invention can also be regulated by the poly-A tail feature.

The sequences used in the mRNA molecules according to the present invention can either be native or not. This holds true for the FGF2 or FGF7 coding region as well as for the UTRs.

The term "native" relates to the human FGF2 and FGF7 mRNA in its natural environment.

Preferably, the sequences are not native but are improved to increase various parameters of the mRNA molecule, such as efficacy, stability, deliverability, producibility, translation initiation and translation.

For example, instead of using the native FGF2 and/or FGF7 coding sequence, sequences optimised with respect to GC-content or codon usage (determined by the codon adaption index, CAI) may be used according to preferred embodiments of the present invention (see below).

The present invention, due to its mechanism, targets treatment of hypotrophic skin conditions, especially atrophic skin conditions, in general; atrophic scars and glucocorticoid (GC)-induced skin atrophy are, however, preferred therapeutic indications addressed with the present invention; whereas cosmetic treatment of the skin is - alternatively - also possible, especially for ageing skin. Atrophic scars are broadly described as exhibiting generalized cutaneous atrophy resulting in loss of cutaneous cells in the epidermis although appear clinically as a loss of normal dermis. Clinically, atrophic scars classically appear as depressions of the skin and commonly occur post acne amongst other causes. The present invention allows administration of a powerful molecule (FGF2 and/or FGF7 encoding mRNA) in a very diligent manner so as to obtain a successful clinical outcome for the patients and at least a significant amelioration of skin condition, especially for atrophic skin tissue. Amelioration of local skin hypotrophy conditions, especially atrophic skin conditions, such as atrophic scars, is measured by quantifying the size of the lesion(s). Invasive measures include the quantification of extracellular matrix components such as collagen, elastin or glycosaminoglykanes based on histological, immunohistochemical or biochemical methods. Evaluations are done at baseline and at defined time points after the treatment. Change is expressed as change from baseline.

According to a preferred embodiment, the present invention therefore preferably addresses the local skin hypotrophy conditions (i.e. the skin diseases resulting in a hypotrophic state of one or several skin layers) selected from the group consisting of cutis laxa, acrodermatitis chronica atrophicans, atrophodermia idiopathica et progressiva Pasini Pierini, scars resulting from perforating dermatoses, atrophy blanche, necrobiosis lipoidica, radiation dermatitis (skin changes resulting from exposure to ionizing radiation), striae cutis distensae (caused by pregnancy, rapid growth, alimentary obesity), atrophic skin conditions, atrophic scars, glucocorticoid (GC)-induced skin atrophy, and skin ulcer.

As already mentioned, the spectrum addressable by the present invention extends from small reductions in the volume of given skin layers to a complete tissue loss, its maximal variant. With respect to the ulcer indications to be treated according to the present invention, there are numerous causes, including trauma, autoimmunological pathology, reduced perfusion (arterial or venous), psychogenic injury (self-trauma), infections. Specific diseases include ulcerative dermatitis as a result of bacterial infection, ulcerative sarcoidosis, ulcerative lichen planus, diabetic foot ulcer, ulcer associated with high blood pressure, ulcer associated with venous insufficiency, neuropathic ulcer, pressure sore, vasculitis (small and medium size vessels), pyoderma gangraenosum, rheumatioid ulceration, necrobiosis lipoidica, drug-induced necrosis (e.g. caused by warfarin, heparin), ulcers in the context of coagulopathies (e.g. caused by antiphospholipid syndrome, protein S deficiency).

Atrophic scars manifest primarily on the face, chest and back of patients. It most commonly results from acne and chick-enpox, but also from surgery, infections, drugs (e.g. steroid injection, penicillamine), autoimmunological processes (e.g. chronic discoid lupus erythematosus), trauma, nerve- and psychogenic (e.g. acne excoriée de jeunes filles) injuries. Hypotrophic/atrophic areas do not constitute an acute/active wound or active site of tissue remodeling but constitute an end-result of inefficient and aberrant repair mechanisms following a local tissue insult. In case of atrophic scars, the decreased dermal volume results in a downward pull of the affected skin area causing a sunken appearance.

Since the major treatment area of the present invention is human medicine, the most preferred embodiment is, of course, a mRNA wherein the coding region encodes human FGF, especially human FGF2 or human FGF7 (as encoded by the various SEQ ID NOs disclosed in the example section of the present invention). These molecules are also preferred for human cosmetic use according to the present invention.

According to a preferred embodiment of the present invention, the present mRNA comprises in the 5'-UTR and/or 3'-UTR (preferably in the 3'UTR) one or more stabilization sequences that are capable of increasing the half-life of the mRNA intracellularly. These stabilization sequences may exhibit a 100% sequence homology with naturally occurring sequences that are present in viruses, bacteria and eukaryotic cells, but may however also be partly or completely synthetic. Examples for such stabilizing sequences are described in: Nucleic Acids Res. 2010; 38 (Database issue): D75-D80. UTRdb and UTRsite (RELEASE 2010): a collection of sequences and regulatory motifs of the untranslated regions of eukaryotic mRNAs and under http://utrdb.ba.itb.cnr.it/.

As a further example of stabilizing sequences that may be used in the present invention, the non-translated sequences (UTR) of the β-globin gene, for example of Homo sapiens or Xenopus laevis, may be mentioned.

Another example of a stabilization sequence has been described in Holcik et al. (Proc. Natl. Acad. Sci. USA 1997, 94: 2410 to 2414) and has the general formula:
(C/U)CCANₓCCC(U/A)PyₓUC(C/U)CC (SEQ ID NO:38),
which is contained in the 3'UTR of the very stable mRNAs that code for example for alpha(1)-collagen or for alpha globin, and ALOX15 or for tyrosine hydroxylase (and wherein "x" is (independently in Nₓ and Pyₓ) an integer of 0 to 10, preferably of 0 to 5 (Holcik et al., 1997), especially 0, 1, 2, 4 and/or 5).

Such stabilization sequences may be used individually or in combination with one another for stabilizing the inventive mRNA as well as in combination with other stabilization sequences known to the person skilled in the art. E.g.: The stabilizing effect of human β-globin 3'-UTR sequences is further augmented by using two human β-globin 3'-UTRs arranged in a head-to-tail orientation.

Accordingly, a preferred embodiment of the FGF2 or FGF7 mRNA according to the present invention is an mRNA molecule, wherein the 5'-UTR or 3'-UTR or the 5'-UTR and the 3'-UTR are different from the native FGF2 or FGF7 mRNA, preferably wherein the 5'-UTR or 3'-UTR or the 5'-UTR and the 3'-UTR contain at least one stabilization sequence, preferably a stabilization sequence with the general formula (C/U)CCANₓCCC(U/A)PyₓUC(C/U)CC (SEQ ID NO:38).

Preferably, the 5'-UTR and/or 3'-UTR are the 5'-UTR and/or 3'-UTR of a different human mRNA than FGF2 or FGF7, preferably selected from alpha Globin, beta Globin, Albumin, Lipoxygenase, ALOX15, alpha(1) Collagen, Tyrosine Hydroxylase, ribosomal protein 32L, eukaryotic elongation factor 1a (EEF1A1), 5'-UTR element present in orthopoxvirus, and mixtures thereof, especially selected from alpha Globin, beta Globin, alpha(1) Collagen, and mixtures thereof.

Accordingly, the present invention preferably relates to an mRNA which comprises in the 3'-UTR one or more stabilization sequences that are capable of increasing the half-life of the mRNA in the cytosol. These stabilization sequences may exhibit a 100% sequence homology with naturally occurring sequences that are present in viruses, bacteria and eukaryotic cells, but may, however, also be partly or completely synthetic. As an example of stabilizing sequences that may be used in the present invention, the non-translated sequences (UTR) of the ß-globin gene, for example of homo sapiens or xenopus laevis, may be mentioned. As already stated, another example of a stabilization sequence has the general formula (C/U)CCANₓCCC(U/A)PyₓUC(C/U)CC, which is contained in the 3'-UTR of the very stable mRNA that codes for alpha-globin, alpha-(1)-collagen, 15-lipoxygenase or for tyrosine hydroxylase (c.f. Holcik et al., Proc. Natl. Acad. Sci. USA 1997, 94: 2410 to 2414). Such stabilization sequences may be used individually or in combination with one another for stabilizing the inventive modified mRNA as well as in combination with other stabilization sequences known to the person skilled in the art.

Another preferred embodiment of the present invention is the 5'-TOP-UTR derived from the ribosomal protein 32L, followed by a stabilizing sequence derived from the albumin-3'-UTR.

Accordingly, a preferred embodiment of the FGF2 or FGF7 mRNA according to the present invention is an mRNA molecule containing a tract of multiple adenosine monophosphates at the 3' end of the 3'-UTR. This so-called poly-adenosine (poly-A) tail consists of at least 60 adenosine monophosphates, preferably at least 100 and most preferably at least 120 adenosine monophosphates.

In certain cases, destabilizing the mRNA might be desirable as well to limit the duration of protein production. This effect can be achieved by incorporating destabilizing sequence elements (DSE) like AU-rich elements into 3'-UTRs, thus ensuring rapid mRNA degradation and a short duration of protein expression.

Although it may be desired for certain embodiments to provide an mRNA which is devoid of destabilizing sequence elements (DSE) in the 3' and/or 5' UTR, there may be other embodiments, wherein the presence or introduction of such DSEs is advantageous. In general, a "DSE" refers to a sequence, which reduces the half-life of a transcript, e.g. the half-life of the mRNA according to the present invention inside a cell and/or organism, e.g. a human patient. Accordingly, a DSE comprises a sequence of nucleotides, which reduces the intracellular half-life of an RNA transcript.

DSE sequences are found in short-lived mRNAs such as, for example: c-fos, c-jun, c-myc, GM-CSF, IL-3, TNF-alpha, IL-2, IL-6, IL-8, IL-10, Urokinase, bcl-2, SGL T1 (Na(+)-coupled glucose transporter), Cox-2 (cyclooxygenase 2), PAI-2 (plasminogen activator inhibitor type 2), beta(1)-adrenergic receptor or GAP43 (5' -UTR and 3'-UTR).

Further DSEs are AU-rich elements (AREs) and/or U-rich elements (UREs), including single, tandem or multiple or overlapping copies of the nonamer UUAUUUA(U/A)(U/A) (where U/A is either an A or a U) and/or the pentamer AUUUA and/or the tetramer AUUU. Further DSEs are described in Nucleic Acids Res. 2010; 38 (Database issue): D75-D80. UTRdb and UTRsite (RELEASE 2010): a collection of sequences and regulatory motifs of the untranslated regions of eukaryotic mRNAs and under http://utrdb.ba.itb.cnr.it/.

Accordingly, it may also be preferred if the 5' -UTR or 3'-UTR or the 5'-UTR and the 3'-UTR contain at least one destabilization sequence element (DSE), preferably AU-rich elements (AREs) and/or U-rich elements (UREs), especially a single, tandem or multiple or overlapping copies of the nonamer UUAUUUA(U/A)(U/A), such as the pentamer AUUUA and/or the tetramer AUUU (the term "U/A" meaning either A or U).

These stabilizing and destabilizing elements can be used alone or in combination to aim at a given duration of protein production and to individualize the treatment of the present invention to the local skin hypotrophy conditions, especially atrophic skin condition, the severity of affected skin and/or the specific group of patients.

Although also nucleic acids encoding FGFs have been suggested for therapeutic applications, these proposals have either been suggested considerable time ago, were mostly related to DNA (not RNA or specifically mRNA) and were related to completely different fields and modes of administration. Moreover, the advantages revealed in the context of the present invention were not observed for these prior art uses of FGF2-/FGF7-mRNA.

The use of immunostimulatory compositions comprising adjuvant mRNA complexed with a cationic or polycationic compound in combination with free mRNA encoding a tumor antigen has previously been described in WO 2010/037408 A1 for prophylaxis, treatment and/or amelioration of tumor diseases, autoimmune, infectious and allergic diseases. This approach allows efficient translation of the administered free mRNA into the protein of interest, while the mRNA complexed with the adjuvant component induces an immune response. Another approach to stabilize nucleic acid for in vivo application is the modification of nucleic acid sequence such as the addition of a Kunitz domain, a protease inhibitor (WO 2009/030464 A2).

RNA-based therapies for the treatment of rare dermatological diseases and treatments for use in medical dermatology and aesthetic medicine have been suggested: WO 2015/117021 A1 discloses the use of a pharmaceutical composition comprising an RNA composed of one or more non-canonical nucleotides for the treatment of AK, whereby the nucleic acid encodes either for a protein of interest of the group of skin-specific structural or growth factor proteins, or for gene-editing protein targets. Similar, WO 2016/131052 A1 discusses the administration of synthetic RNA comprising canonical and non-canonical nucleotides encoding collagenase as anti-scarring treatment. In both patent applications, the administration of the pharmaceutical composition comprising the synthetic RNA can occur on multiple ways such as subcutaneous, intradermal, subdermal or intramuscular injection, as well as topical.

However, it could be shown with the present invention that a combination of modified and non-modified nucleotides is not better than an unmodified mRNA (more to the contrary, an unexpectedly high reduction by a factor of 10 lower was observed, e.g. with a FGF7 mRNA containing 5meC and pseudo-U instead of C and U). This result according to the present invention stands also in contrast with other reports wherein the use of non-canonical nucleotides was reported to be advantageous (see e.g. Thess et al., Molecular Therapy 23 (2015), 1456-1464; Kariko et al., Molecular Therapy 20 (2012), 948-953; Kormann et al. Nature Biotechnology 29 (2011), 154-157). In fact, it can be shown with the present invention that the use of exclusively native sequences in the coding region/sequence (CDS) is more effective and that this effect can further be multiplied with the amendments in the CDS. Moreover, also the implied longevity (to avoid substantial cellular toxicity) is better.

In addition, according to certain preferred embodiments, the FGF mRNAs according to the present invention are free of non-canonical nucleotides, and contain a modified UTR and an optimized CDS. Such preferred FGF mRNAs have a further surprising effect in that many more efficient and surprising effects after 24h-120h post transfection are obtained. This is counterintuitive, and non-expected if native mRNA (coding region) or the recombinant protein is the model for state of the art understanding of FGF function.

EP 2 641 614 A1 discloses a microneedle assembly formulation for prevention or treatment of skin aging or skin scars (UV-damaged skin, hypertrophic scar, atrophic scar, keloids, acne scar, hair loss, suture wound, burn wound, ulcer, bedsore, diabetic ulcer or a disease requiring angiogenesis) comprising a substance consisting e.g. of basic fibroblast growth factor (bFGF), acidic fibroblast growth factor (aFGF or FGF1), or a nucleic acid and a plasmid encoding the gene thereof. Regarding proteins, it turned out that clearance of secreted (recombinant) FGFs is extremely fast and that therefore single bolus injections are not guaranteeing successful application for the intended therapeutic use. Usually proteins need to be applied frequently (at least daily) and doses will have to be comparably high as compared to production as triggered by the mRNAs suggested (low ng vs µg).

According to a preferred embodiment, the FGF2 mRNA according to the present invention is not encoding full length FGF2 cDNA but the ORF encoding a short frame, secreted (Fibroblast growth factor 2 (FGF2), a prototypic member of the FGF family, is encoded by a single gene.

As already referred to above, alternative translation-initiation codons produce various isoforms: low molecular weight FGF2 (Lo FGF2) is an 18 kDa protein translated from a conventional AUG start codon and its 155 amino acid sequence is common to all FGF2 isoforms. The RNA sequence in the CDS is 468 nucleotides and is an integral part of the naturally occurring full length mRNA sequence as disclosed in a publicly available database (https://www.ncbi.nlm.nih.gov/nuccore) with the accession numbers: J04513.1 (for all database references herein, a date of 31 July 2017 applies). Alternatively, the sequence is also disclosed in NM_002006.4 and M27968.1, respectively. The high molecular weight (Hi FGF2) isoforms (20.5 and 21 kDa) are produced by starting translation at CUG sites upstream and inframe of the AUG codon. The 21.5kd form has 591 nucleotides in the CDS and encodes 196aa and the CDS of the 22kd form has 633 nucleotides encoding for 210aa. Usage of these CDS variants would also lead to secretion of the short form of FGF2 described in this invention.

Alternative sequences for FGF2 which have been proposed for use also include changes of the CDS: FGF2 is lacking a standard secretion signal and export is mediated by an energy-dependent, non-ER/Golgi pathway. Hence, adding of alternative secretion signals within the FGF2 coding sequence have been suggested to increase secretion and are included: Sohn et al. (2001) use a classical secretion signal sequence of FGF-4, Sasada et al., 1991 use the secretion signal sequence of IL-2, Blam et al. (1988) use the secretion signal sequence of Growth hormone; In addition also mutation of the second and third of the four cysteines (i.e., cys-70 and cys-88) to serine and asparagine have been suggested to increase protein stability without affecting the biological activity of FGF2. Accordingly, also a combination of mutations with altered secretion sequences as mentioned above has been suggested as well. Along these lines: Chen et al 2007 (and references therein) suggest the use of BMP-, specifically BMP-2/4 hybrid secretion signal sequences along with mutation of the second and third of the four cysteines (i.e., cys-70 and cys-88) to serine and asparagine to increase stability and secretion of the mutated FGF2 protein.

The other isoforms and frames in the gene are not preferred embodiments of the present invention; hence it is usually only a portion of the FGF2 gene which is used according to the present invention, not the full-length form as implied by the EP 2 641 614 A1. A person skilled in the art would - according to the teachings of EP 2 641 614 A1 - thus rather think that a full gene cDNA would lead to in situ production of different forms of FGF2 protein (Hi and Low MW forms) with distinct functions and would not lead to a comparable biological outcome as sole production of a secreted short form. DNA/plasmid also would not have the same expression kinetics and would pose the risk of integration into the genome, hence constitute a potential problem for safety. RNA mediated gene transfer is desirable especially in local applications as it avoids promoter expression uncertainty (expression plasmids, cDNA based approaches relying on external promoters), and provides a defined period for a potent biologic effect for without concerns of long-term deleterious effects. With the RNA delivery approach, target cells serve as a bioreactor for protein synthesis eliminating protein processing and modification difficulties noted with exogenously produced, recombinant products. The mRNA delivery technique allows the use of more potent cellular factors or stimulants than previously possible as it is not associated with long term mutagenic concerns and will be self-limiting due to decline after short period.

WO 2014/089486 A1 discloses compositions comprising at least one mRNA encoding a polypeptide of interest (e.g. FGF2 or FGF7) and a transfer vehicle comprising a lipid nanoparticle or a lip-idoid nanoparticle for treating diseases associated with protein or enzyme deficiencies; however, this document does not mention skin diseases such as chronic wounds, ulcers etc. US 2007/149475 A1 discloses a method of augmenting transient protein synthesis in a cell for improving wound healing by delivering eIF-4E mRNA alone or in combination with mRNAs encoding e.g. growth factors necessary for wound healing such as FGF-2. WO 2010/037408 A1 describes an immunostimulatory composition comprising a) an adjuvant component comprising of at least one complexed mRNA e.g. FGF2 or bFGF and b) at least one free mRNA for treating e.g. skin diseases. This approach is counterintuitive to the approach according to the present invention: According to the present invention, immunostimulatory events should be kept as low as possible or be avoided completely. The approach taught in WO 2010/037408 A1 is to eliminate endogenous proteins like FGFs by inducing an immune response against them. Accordingly, the use of KGF/FGF7 and FGF2 as therapeutic components to treat e.g. scars is not within the teaching of this document but to use the factors to create a response against them. The therapeutic principle in WO 2010/037408 A1 is thus elimination of the endogenous KGF/FGF7 and FGF2 protein(s) for treatment rather than use of the proteins as therapeutic agents (as in the present invention).

Moreover, preferred embodiments of the present invention are using improved UTRs and improved mRNA coding sequences (with regard to codon uses).

It is therefore evident that growth factors, such as FGFs, especially FGF2 and FGF7, have not been suggested to be applied in the context of the present invention.

General concepts for improved mRNA-based therapeutics (see e.g. Sahin et al., Nat. Rev. Drug Disc. 2014. 13(10): 759-780) are also applicable for the present invention.

Although in most cases, the use of exclusively canonical nucleotides, there may be certain occasions where the FGF2 and FGF7 mRNA according to the present invention may contain other monophosphate nucleosides than those of cytidine (C), uridine (U), adenosine (A) or guanosine (G) residues (the canonical nucleotides). There are a significant number of naturally occurring analogs of these monophosphate nucleosides and also (even more) synthetic variants of these mRNA residues. Embodiments of such variants can be found e.g. in WO 2014/153052 A2.

According to an embodiment, in the present FGF mRNA, at least 5%, preferably at least 10%, more preferably at least 30%, especially at least 50% of all
- cytidine residues are replaced by 5-methyl-cytidine residues, and/or
- cytidine residues are replaced by 2-amino-2-deoxy-cytidine residues, and/or
- cytidine residues are replaced by 2-fluoro-2-deoxy-cytidine residues, and/or
- cytidine residues are replaced by 2-thio-cytidine residues, and/or
- cytidine residues are replaced by 5-iodo-cytidine residues, and/or
- uridine residues are replaced by pseudo-uridine residues, and/or
- uridine residues are replaced by 1-methyl-pseudo-uridine residues, and/or
- uridine residues are replaced by 2-thio-uridine residues, and/or
- uridine residues are replaced by 5-methyl-uridine residues, and/or
- adenosine residues are replaced by N6-methyl-adenosine residues.

Specific embodiments are FGF2 and FGF7 mRNAs, wherein in the FGF mRNA, at least 5%, preferably at least 10%, more preferably at least 30%, especially at least 50% of all
- cytidine residues are replaced by 5-methyl-cytidine residues, and/or
- uridine residues are replaced by pseudo-uridine residues, and/or
- uridine residues are replaced by 2-thio-uridine residues.

In the course of the present invention it has been surprisingly found out that even more improved results can be obtained if the GC-content (or GC to AU ratio) of the mRNA is further increased. The reason why this was specifically surprising was because the native FGF2 and FGF7 sequences were already regarded as being optimal with respect to translation/expression efficiency. However, if the FGF2 mRNA according to the present invention is designed with a GC to AU ratio of at least 51.7% or more preferred at least 52% (e.g. at least 52.1, at least 52.2, at least 52.3), or the FGF7 mRNA according to the present invention is designed with a GC to AU ratio of at least 39.5% or more preferred at least 43%, the performance according to the present invention further increases. Accordingly, the GC to AU ratio is preferably of at least 51.7%, preferably of at least 52%, more preferred 55%, even more preferred at least 58%, especially at least 60% in case of the FGF2 mRNA and at least 39,5%, preferably of at least 43%, more preferred 45%, even more preferred at least 50%, especially at least 55% in case of the FGF7 mRNA.

In connection with the nucleoside variants disclosed above, it is important to note that the specific preferred variants described above do not influence the GC content, i.e. the variant is conservative in this respect (e.g. a cytidine variant still counts as a cytidine for the calculation of the GC content).

Another surprising observation revealed in the course of the present invention was the fact that FGF2/FGF7-mRNAs with increased Codon Adaptation Index (CAI) also showed improved performance in the present invention, especially with respect to expression capacity within the cell.

The CAI is a measurement of the relative adaptiveness of the codon usage of a gene towards the codon usage of highly expressed genes. The relative adaptiveness (w) of each codon is the ratio of the usage of each codon, to that of the most abundant codon for the same amino acid. The CAI index is defined as the geometric mean of these relative adaptiveness values. Non-synonymous codons and termination codons (dependent on genetic code) are excluded. CAI values range from 0 to 1, with higher values indicating a higher proportion of the most abundant codons (Sharp et al., Nucleic Acids Res. 15 (1987): 1281-1295., Jansen et al., Nucleic Acids Res. 31 (2003): 2242-2251).

Therefore, a preferred embodiment of the present invention relates to an FGF2 and FGF7 mRNA, wherein the FGF2 mRNA has a codon adaption index (CAI) of at least 0.76, preferably at least 0.77, at least 0.8, at least 0.82, at least 0.83, at least 0.84, at least 0.85, at least 0.86, at least 0.87, at least 0.88, at least 0.89 and, wherein the FGF7 mRNA has a codon adaption index (CAI) of at least 0.71, at least 0.74, preferably at least 0.75, at least 0.76, at least 0.77, at least 0.78, at least 0.79, at least 0.8, at least 0.81, at least 0.82, at least 0.83, at least 0.84, at least 0.85. Even a more preferred CAI of the FGF2 mRNAs according to the present invention is at least 0.9, especially at least 0.91. Even a more preferred CAI of the FGF7 mRNAs according to the present invention is at least 0.86, especially at least 0.87.

For comparison: native GC to AU ratio of FGF2 is 51.7% and of FGF7 is 39.5%; native CAIs of FGF2 is 0.76 and of FGF7 is 0.74.

Preferred CG to AU ratios are higher than the native ones, e.g. for FGF2 at least 52%, preferably at least 55%, especially at least 60% or for FGF7 at least 40%, preferably at least 45%, especially at least 50%.

Even more preferred, the FGF2 mRNAs according to the present invention have a CAI of at least 0.76 AND a GC content of at least 51.7% and the FGF7 mRNAs a CAI of at least 0.74 AND a GC content of at least 39.48% (or an even more preferred higher CAI/GC content).

Preferred consensus sequences of the FGF mRNA according to the present invention for FGF7 are SEQ ID NOs:12, 13 and 14; most preferred SEQ ID NO:12.

SEQ ID NO:12 comprises optimized GC-rich sequences; SEQ ID NO:13 comprises all optimized sequences including AU-rich sequences; SEQ ID NO:14 is the consensus for all optimised as well as the native sequence.

Preferred consensus sequences of the FGF mRNA according to the present invention for FGF2 are SEQ ID NOs:30, 31 and 32; most preferred SEQ ID NO:30.

SEQ ID NO:30 comprises optimized GC-rich sequences; SEQ ID NO:31 comprises all optimized sequences including AU-rich sequences; SEQ ID NO:32 is the consensus for all optimised as well as the native sequence.

Accordingly, the coding region of the FGF mRNA encoding human FGF7 is preferably SEQ ID NO:12, especially SEQ ID NOs:2, 3, 5, 6, 7, 8, 9, 10, or 11; the coding region of the FGF mRNA encoding human FGF2 is preferably SEQ ID NO:30, especially SEQ ID NOs:19, 20, 21, 22, 23, 24, 25, 26, 27, 28, or 29.

As shown in the example section, transfection of fibroblasts with the optimized FGF mRNA variants provided with the present invention differs in terms of its overall kinetics from what is known in the field about other mRNAs. Upon delivery of mRNA into a given cell, which is typically achieved quickly when supported by appropriate transfection agents, the mRNA content within a given cell is constantly dropping. In this regard, IVT mRNA is usually behaving as intrinsic mRNA as it is degraded by the same cellular processes. The amount of protein produced upon delivery parallels the amount of the respective IVT mRNA within the cell with a certain delay, which basically reflects the time needed to activate the translation machinery. Typically, after an initial peak, the level of the protein produced in cells upon transfection with IVT mRNA is constantly falling.

Surprisingly, and in sharp contrast to the usual situation described above, delivery of FGF mRNA variants into cells is characterized by a dissociation between the mRNA and protein kinetics. While the IVT mRNA is being gradually degraded upon delivery, protein secretion as shown here begins to rise on the first day (i.e. expression is lowest for the first 24h). It reaches a plateau on the second day, which is sustained for several days before FGF production is waning.

As shown in the example section, this pattern was obtained with 3 different cell lines of two origins from three species (mouse: 3T3 (fibroblast), pig: keratinocytes (in epithelial sheets), human: BJ cells (fibroblasts)). This is consistent with an effect of IVT mRNA-induced FGF protein on the auto- or paracrine regulation of endogenous FGF production.

Interestingly, this pattern is not achieved upon addition of recombinant FGF protein at similar amounts as expressed at 24h post transfection to the cells/organs. There, no such autoregulation has been observed 24h post addition of recombinant FGF protein suggesting that mRNA-induced expression is able to activate different intra- and extracellular effects compared to mere protein substitution.

The optimized sequences according to the present invention therefore have i.a. a very surprising effect compared to the native sequences: with the optimized sequences of FGF2 and FGF7, a significant prolongation of the effect can be obtained. Whereas it may be expectable that a peak of activity can be achieved 24 h after transformation, this is significantly different with the optimized sequences according to the present invention. As also disclosed in the example section of the present invention, the activity of the native and the optimized sequences is comparable in the initial phase after transformation. However, this changes significantly in the further course: Whereas the native sequence does not produce significant activity after 120 h anymore, the optimized sequences as provided with the present invention produce a 10-fold and an almost 100-fold improved effect. Moreover, it has to be emphasized that this effect after 120 h is even higher than after 24 h. It follows that administration of the mRNAs according to the present invention to human patients changes treatment paradigms by both significantly reducing the application frequency as compared to other, currently described approaches, as well as significantly surpassing the efficacy of currently described approaches. Such a treatment means e.g.: weekly application instead of daily injection and achieving increased treatment success in patients using the envisaged mRNAs. This increased success is anticipated to result from the intrinsic differences achieved within the treated area by localized, in situ expression of FGFs by the mRNAs according to the present invention.

According to a preferred embodiment, the FGF2/FGF7 mRNA according to the present invention is administered subcutaneously, intradermally, transdermally, epidermally, or topically, especially epidermally.

Administration of the present FGF2/FGF7 mRNA can be performed according to optimised expression aims, depending on the amount of mRNA applied, the stability of the mRNA molecule and the status of disease. For example, the FGF2/FGF7 mRNA may be administered at least once, at least twice, at least twice within one month, preferably weekly. For example, if the FGF2/FGF7 mRNA may be administered at least twice, at least twice within one month, preferably weekly doses applied may vary.

The amount of mRNA delivered per dose may also be made dependent on the stability of the molecule, etc.. Preferably the FGF2/FGF7 mRNA according to the present invention is administered in an amount of 0.01µg to 100 mg per dose, preferably of 0.1µg to 10mg per dose, especially of 1µg to 1mg per dose.

Suitable formulations for mRNA therapeutics are well available in the art (see e.g. Sahin et al., 2014; WO 2014/153052 A2 (paragraphs 122 to 136), etc.).

The present invention therefore also relates to a pharmaceutical formulation comprising an FGF2/FGF7 mRNA according to the present invention. The present formulation comprises the mRNA in a pharmaceutically acceptable environment, e.g. with suitable components usually provided in mRNA therapeutics (excipients, carriers, buffers, auxiliary substances (e.g. stabilizers), etc.)

The mRNA formulations according to the present invention preferably contain a suitable carrier. Suitable carriers include polymer based carriers, such as cationic polymers including linear and branched PEI and viromers, lipid nanoparticles and liposomes, nanoliposomes, ceramide-containing nanoliposomes, prote-oliposomes, cationic amphiphilic lipids e.g: SAINT®-Lipids, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, dry powders, poly(D-arginine), nanodendrimers, starch-based delivery systems, micelles, emulsions, sol-gels, niosomes, plasmids, viruses, calcium phosphate nucleotides, aptamers, peptides, peptide conjugates, small-molecule targeted conjugates, polylactic-co-glycolic acid (PLGA) polymers and other vectorial tags. Also contemplated is the use of bionanocapsules and other viral capsid protein assemblies as a suitable carrier. (Hum. Gene Ther. 2008 Sep;19(9):887-95).

Preferred carriers are cationic polymers including linear and branched PEI and viromers, lipid nanoparticles and liposomes, transfersomes, and nanoparticulates including calcium phosphate nanoparticulates (i.e. naked RNA precipitated with CaCl₂ and then administered).

A preferred embodiment of the present invention relates to the use of non-complexed mRNA, i.e. non-complexed mRNA in a suitable aqueous buffer solution, preferably a physiological glucose buffered aqueous solution (physiological). For example, a 1xHEPES buffered solution; a 1xPhosphate buffered solution, Na-Citrate buffered solution; Na-Acetate buffered solution; Ringer's Lactate solution; preferred with Glucose (e.g.: 5% Glucose); physiologic solutions can be preferably applied.

Preferably the present invention applies liposomes, especially liposomes which are based on DOTAP, DOTMA, Dotap-DOPE, DOTAP-DSPE, Dotap-DSPE-PEG, Dotap-DOPE-PEG, Dotap-DSPE-PEG-Na-Cholate, Dotap-DOPE-PEG-Na-Cholate, DOTAP with cationic amphiphilic macromolecules (CAM) as complexes, and combinations thereof.

According to another aspect, the present invention relates to a kit for administering the FGF2/FGF7 mRNA according to the present invention to a patient comprising
- the FGF mRNA as defined herein, and
- a skin delivery device.

Preferably, the skin delivery device is
- an intradermal delivery device, preferably selected from the group consisting of needle based injection systems,
- a transdermal delivery device, preferably selected from the group consisting of transdermal patches, hollow and solid microneedle systems, microstructured transdermal systems, electrophoresis systems, and iontophoresis systems, or
- an epidermal delivery device, preferably selected from the group consisting of needle free injection systems, laser based systems, especially Erbium YAG laser systems, and gene gun systems.

These administration devices are in principle available in the art; adaption to the administration of the mRNA according to the present invention is easily possible for a person skilled in the art.

The present invention also relates to a method for treating local skin hypotrophy conditions, preferably atrophic skin conditions, especially atrophic scars and glucocorticoid (GC)-induced skin atrophy, wherein the mRNA according to the present invention is administered in an effective amount to a patient in need thereof.

According to a further aspect, the present invention also relates to the use of the mRNAs according to the present invention for the cosmetic treatment of ageing skin, e.g. UV-damaged skin, hair loss, etc.. Also for these aspects, the molecules, formulations (adapted to cosmetic purposes), and methods disclosed with the present invention are suitable. The present invention therefore also relates to a cosmetic skin care method wherein an FGF mRNA according to the present invention is contacted with the human skin, especially an ageing skin. The present invention also contemplates cosmetic formulations comprising an FGF mRNA according to the present invention and a cosmetic carrier. Cosmetic carriers are well available in the art, often overlapping with the ingredients for a pharmaceutical formulation as disclosed above. Preferably, the cosmetic formulation according to the present invention is provided as an ointment, a gel, especially a hydrogel, an emulsion and/or contains liposomes, cationic polymers, nano- or microparticles.

### Sequences:

SEQ ID NO:1 FGF7 native 
augcacaaau ggauacugac auggauccug ccaacuuugc ucuacagauc
augcuuucac auuaucuguc uaguggguac uauaucuuua gcuugcaaug acau-
gacucc agagcaaaug gcuacaaaug ugaacuguuc cagcccugag cgacacacaa
gaaguuauga uuacauggaa ggaggggaua uaagagugag aagacucuuc
ugucgaacac agugguaccu gaggaucgau aaaagaggca aaguaaaagg gacccaa-
gag augaagaaua auuacaauau cauggaaauc aggacagugg caguuggaau ugug-
gcaauc aaaggggugg aaagugaauu cuaucuugca augaacaagg aaggaaaacu
cuaugcaaag aaagaaugca augaagauug uaacuucaaa gaacuaauuc uggaaaac-
ca uuacaacaca uaugcaucag cuaaauggac acacaacgga ggggaaaugu
uuguugccuu aaaucaaaag gggauuccug uaagaggaaa aaaaacgaag aaa-
gaacaaa aaacagccca cuuucuuccu auggcaauaa cuuaa
SEQ ID NO:2 FGF7 HUMAN MOD 
augcacaaau ggauucucac auggauucug ccuacguugc ucuacagaag
cugcuuucac aucaucuguc uugugggcac uaucucacug gcuugcaaug acau-
gacacc ggaacagaug gcaaccaaug ugaacuguuc uuccccagaa aggcauacca
gaagcuacga cuacauggaa ggaggggaua ucaggguucg cagauuguuc ugucgua-
 
cuc agugguaucu ucgcaucgac aaacggggua aggugaaggg aacacaggag au-
gaagaaca acuacaacau cauggagauu cggacaguug cagucgggau ugucgccaua
aagggugugg aauccgaguu cuaucuggcc augaacaaag aaggcaaacu
guaugccaag aaagagugca augaggauug caauuucaaa gagcugauuc uggagaac-
ca cuacaacacc uaugcuagug cgaaauggac ccauaaugga ggggaaaugu uugug-
gcacu caaucagaag ggcauacccg uacgaggcaa gaaaacgaag aaggagcaaa
agaccgcuca uuuucugccc auggccauca cuuga
SEQ ID NO:3 FGF7 
augcacaagu ggauccugac cuggauccug ccuacacugc uguacagaag
cugcuuccac aucaucugcc ucgugggcac aaucagccug gccugcaacg
auaugacccc ugagcagaug gccaccaacg ugaacuguag cagccccgag aga-
cacaccc gguccuacga uuauauggaa ggcggcgaca ucagagugcg gcggcuguuc
uguagaaccc agugguaucu gcggaucgac aagcggggca aagugaaggg cacccaa-
gag augaagaaca acuacaacau cauggaaauc cggaccgugg ccgugggcau cgug-
gcuauu aagggcgucg agagcgaguu cuaccuggcc augaacaaag agggcaagcu
guacgccaag aaagagugca acgaggacug caacuucaaa gagcugaucc ucgagaac-
ca cuacaauacc uacgccagcg ccaaguggac ccacaauggc ggcgaaaugu ucgug-
gcccu gaaccagaaa ggcauccccg ugcgcggcaa aaagaccaaa aaagagcaga
aaacggccca cuuccugccu auggccauca ccuaa
SEQ ID NO:4 FGF7 
augcacaagu ggauccucac auggauccug ccuacgcugc ucuaccgcag
cugcuuccac aucaucuguc uggugggcac uaucucacug gcuugcaacg acau-
gacccc ggagcagaug gcaaccaacg ugaacuguag cuccccagag aggcacaccc
ggagcuacga cuacauggag ggaggggaca ucaggguccg cagacuguuc ugucgua-
cuc agugguaccu gcgcaucgac aagcggggua aggugaaggg aacccaggag au-
gaagaaca acuacaacau cauggagauc cggacagugg cggucgggau cgucgccauc
aagggugugg aguccgaguu cuaccuggcc augaacaagg agggcaagcu
guacgccaag aaggagugca acgaggacug caacuucaag gagcugaucc uggagaac-
ca cuacaacacc uacgcuagug cgaaguggac ccacaacgga ggggagaugu ucgug-
gcacu caaccagaag ggcauccccg uccgaggcaa gaagacgaag aaggagcaga
agaccgcgca cuuccugccc auggccauca ccuga
SEQ ID NO:5 FGF7 
augcauaagu ggauucuuac auggauucuc ccaacacuuc uuuacagauc
augcuuucau auuaucuguu uggugggaac gauuucucuu gcuuguaaug
auaugacucc agagcaaaug gcuacuaaug uuaacuguuc cucaccugag cgucauac-
ua gaucuuauga uuacauggag ggaggugaua uaagaguuag gagacuuuuc
ugucgaacac agugguaucu gagaaucgau aagagaggua aagucaaagg aacccag-
 
gag augaagaaua acuacaauau cauggagauc aggacagugg caguuggaau
aguugcaauc aaaggagucg aaagugaguu cuaucuugcu augaacaagg
aaggaaaacu guacgcaaag aaagaaugua augaggauug caacuucaag
gagcuuaucc uggaaaacca uuacaacacc uaugcaagug caaaauggac ucacaac-
gga ggagaaaugu uuguugcauu gaaucagaaa gggauaccug ugagaggaaa
gaaaaccaag aaagaacaga aaacugcuca cuuucuuccu auggcuauua ccuga
SEQ ID NO:6 FGF7 
augcacaagu ggauccugac cuggauccug cccacccugc uguacagaag
cugcuuccac aucaucugcc uggugggcac caucagccug gccugcaacg acau-
gacccc cgagcagaug gccaccaacg ugaacugcag cagccccgag agacacacca
gaagcuacga cuacauggag ggcggcgaca ucagagugag aagacuguuc ug-
cagaaccc agugguaccu gagaaucgac aagagaggca aggugaaggg cacccaggag
augaagaaca acuacaacau cauggagauc agaaccgugg ccgugggcau cgug-
gccauc aagggcgugg agagcgaguu cuaccuggcc augaacaagg agggcaagcu
guacgccaag aaggagugca acgaggacug caacuucaag gagcugaucc uggagaac-
ca cuacaacacc uacgccagcg ccaaguggac ccacaacggc ggcgagaugu ucgug-
gcccu gaaccagaag ggcauccccg ugagaggcaa gaagaccaag aaggagcaga
agaccgccca cuuccugccc auggccauca ccuga
SEQ ID NO:7 FGF7 
augcacaagu ggauccugac cuggauccug cccacccugc uguaccgcag
cugcuuccac aucaucugcc uggugggcac caucagccug gccugcaacg acau-
gacccc cgagcagaug gccaccaacg ugaacugcag cagccccgag cgccacaccc
gcagcuacga cuacauggag ggcggcgaca uccgcgugcg ccgccuguuc ugccg-
caccc agugguaccu gcgcaucgac aagcgcggca aggugaaggg cacccaggag au-
gaagaaca acuacaacau cauggagauc cgcaccgugg ccgugggcau cguggccauc
aagggcgugg agagcgaguu cuaccuggcc augaacaagg agggcaagcu
guacgccaag aaggagugca acgaggacug caacuucaag gagcugaucc uggagaac-
ca cuacaacacc uacgccagcg ccaaguggac ccacaacggc ggcgagaugu ucgug-
gcccu gaaccagaag ggcauccccg ugcgcggcaa gaagaccaag aaggagcaga
agaccgccca cuuccugccc auggccauca ccuaa
SEQ ID NO:8 FGF7 
augcauaagu ggauucuuac auggauucuc ccaacacugc uguacagguc
augcuuucac auuaucuguc uggugggaac gauuucucuu gcuugcaaug acau-
gacucc agagcaaaug gcuacuaaug ugaacuguuc cucaccugag cgucauacua
gaucuuauga cuacauggag ggaggugaua uaagagugag gagacuuuuc
ugucgaacac agugguaucu gcggaucgau aagagaggua aagucaaagg cacccag-
gag augaagaaua acuacaauau cauggagauc aggacagugg caguuggaau
 
aguugcaauc aaaggggucg aaagcgaguu cuaucuugcu augaacaagg
aaggcaaacu guacgccaag aaagaaugca augaggauug caacuucaag
gagcuuaucc uggaaaacca uuacaacacc uaugcaagug caaaauggac ucacaac-
gga ggggaaaugu uuguugcauu gaaucagaaa gggauaccug ugagaggcaa
gaaaaccaag aaagaacaga aaacugccca cuuucuuccu auggcuauua ccuga
SEQ ID NO:9 FGF7 
augcauaagu ggauauugac guggauuuua ccuacucucc uauauagguc
cugcuuccau auaauuuguu uggugggcac cauuucucuu gccugcaaug
auaugacacc cgagcagaug gcaaccaacg uaaacuguuc cucacccgag cgacauac-
ga gaagcuacga cuacauggag ggaggugaua uuagggucag acgccuguuu ugucg-
gacac agugguaucu uagaauugac aaacguggua aggucaaggg gacccaggaa au-
gaaaaaua acuauaauau cauggaaauc cgcaccgugg caguggggau cguggcgauc
aagggagugg aaagcgaauu cuaucuggcu augaacaaag agggaaagcu guacgcu-
aaa aaagaaugca augaggacug caacuuuaaa gaacugaucc ucgagaauca
cuacaauacc uacgccagug ccaaguggac acacaacggg ggcgagaugu ucguug-
cacu gaaccagaag ggcaucccag uucggggcaa gaaaacaaaa aaggagcaaa
agacugcuca cuuucucccg auggccauca cuuga
SEQ ID NO:10 FGF7 
augcauaaau ggauccuuac guggauacug ccgacacucc uuuauagguc
uuguuuucac auaauuugcc ucguuggaac uauaucucuu gccugcaacg acau-
gacccc agaacaaaug gcuacaaacg ugaauuguuc cagucccgaa agacacacgc
gaaguuauga cuacauggaa ggcggcgaua uaagaguuag gagacuuuuu
ugucgaacgc aaugguaucu gaggauugac aagcgcggga agguaaaagg gacccag-
gag augaagaaca acuauaacau aauggagauu aggacagugg cugugggcau
cguagcgauc aaagguguag aaucagaguu uuaccuggcc augaacaaag aaggu-
aaacu uuaugcuaaa aaagaaugca acgaagauug uaacuucaaa gaauugaucc
uugaaaauca cuauaacaca uaugcauccg cgaaguggac acauaacggg ggagaaau-
gu ucgucgcguu gaaucaaaaa gguauuccgg uucggggaaa aaaaaccaag
aaggagcaga agacggcuca cuucuugcca auggccauaa cuuaa
SEQ ID NO:11 FGF7 
augcacaagu ggauccuuac guggauacuc ccaacacuuu uguaucgaag
uuguuuucac auuauuugcc uggucggcac gauuucauug gccugcaacg
auaugacacc ggaacagaug gcuacaaacg uaaacuguag uucacccgag cgg-
cacacuc gaucuuacga uuacauggaa gguggagaca ucaggguuag aagacucuuu
ugcaggacgc aaugguaccu ccgcauagau aagagaggaa aggugaaagg aacacag-
gaa augaaaaaua acuacaacau aauggaaauu cggacugucg cugugggaau
cguugccauc aaaggagugg aaucagaauu cuaccuggcu augaauaagg agggaaa-
 
gcu cuaugcgaaa aaggagugca acgaggacug uaauuucaaa gaacuuaucc
uugaaaacca uuacaacacc uaugcgagug ccaaguggac ucauaacggu ggugagau-
gu ucguagcucu gaaucagaag ggcauuccgg uccggggaaa gaagacuaag aaa-
gagcaga aaacggcaca cuuucuuccu auggcgauua cauaa
SEQ ID NO:12 FGF7 consensus (optimized GC-rich sequences) 
augcacaaru ggauycusac muggauycug ccyacvyugc usuacmgmag
cugcuuycac aucaucugyc ubgugggcac haucwsmcug gcyugcaayg ayaugac-
mcc bgarcagaug gcmaccaayg ugaacugyws ywscccmgar mgvcayaccm gvws-
cuacga yuayauggar ggmggsgaya ucmgvgubmg vmgvyuguuc ugymghacyc
agugguaycu kmgvaucgac aarmgvggya argugaaggg macmcargag augaa-
gaaca acuacaacau cauggarauy mgvacmgukg cvgusggsau ygusgcyauh
aagggygusg arwscgaguu cuaycuggcc augaacaarg arggcaarcu
guaygccaag aargagugca aygaggayug caayuucaar gagcugauyc usgagaac-
ca cuacaayacc uaygcyagyg csaaruggac ccayaayggm ggsgaraugu uygug-
gcmcu saaycagaar ggcaumcccg uvmgmggcaa raaracsaar aargagcara
aracsgcbca yuuycugccy auggccauca cyura
SEQ ID NO:13 FGF7 consensus (all optimized sequences) 
augcayaaru ggauhyubac vuggauhyuv ccnacnyuby unuaymgvws hug-
yuuycay auhauyugyy ubgubggmac nauhwshyuk gcyugyaayg ayaugachcc
ngarcaraug gchachaayg udaayugyws ywshcchgar mgncayacbm
gvwsyuayga yuayauggar gghggngaya uhmgvgubmg vmgvyubuuy
ugymgnacnc arugguaycu bmgvauhgay aarmgnggna arguvaargg vacmcar-
gar augaaraaya acuayaayau mauggarauy mgvachgubg cngubggvau
hgungcnauh aarggnguvg arwshgaruu yuaycukgcy augaayaarg argghaar-
cu buaygcnaar aargarugya aygargayug yaayuuyaar garyukauyc ub-
garaayca yuayaayacm uaygcnwsyg cvaaruggac hcayaayggn ggngaraugu
uygungcnyu saaycaraar ggbauhccng unmgvggmaa raaracnaar aargar-
cara aracbgcnca yuuyyubccn auggcbauha chura
SEQ ID NO:14 FGF7 consensus (all optimized and native sequences) 
augcayaaru ggauhyubac vuggauhyuv ccnacnyuby unuaymgvws hug-
yuuycay auhauyugyy ungubgghac nauhwshyud gcyugyaayg ayaugachcc
ngarcaraug gchachaayg udaayugyws ywshcchgar mgncayacnm
gvwsyuayga yuayauggar gghggngaya uhmgvgubmg vmgvyubuuy
ugymgnacnc arugguaycu bmgvauhgay aarmgnggna arguvaargg vacmcar-
gar augaaraaya ayuayaayau mauggarauy mgvachgubg cngubggvau
hgungcnauh aarggnguvg arwshgaruu yuaycukgch augaayaarg argghaar-
cu buaygcnaar aargarugya aygargayug yaayuuyaar garyudauyc ub-
 
garaayca yuayaayacm uaygcnwshg cnaaruggac hcayaayggn ggngaraugu
uygungcnyu vaaycaraar ggbauhccng unmgvggmaa raaracnaar aargar-
cara aracngcnca yuuyyubccn auggcnauha chura
SEQ ID NO:15 FGF protein 
MHKWILTWIL PTLLYRSCFH IICLVGTISL ACNDMTPEQM ATNVNCSSPE RHTRSYDYME
GGDIRVRRLF CRTQWYLRID KRGKVKGTQE MKNNYNIMEI RTVAVGIVAI KGVESEFYLA
MNKEGKLYAK KECNEDCNFK ELILENHYNT YASAKWTHNG GEMFVALNQK GIPVRGKKTK
KEQKTAHFLP MAIT
SEQ ID NO:16 Model Seq including the 5'UTR (aGlobin, human+ Koszak) 
gggagacaua aacccuggcg cgcucgcggc ccggcacucu ucuggucccc acagacucag
agagaaccca cc
SEQ ID NO:17 Model Seq including the 3'UTR (aGlobin, human+ Poly A site and first A (of 120)) 
gcuggagccu cgguggccau gcuucuugcc ccuugggccu ccccccagcc ccuccucccc
uuccugcacc cguacccccg uggucuuuga auaaagucug agugggcggc a
SEQ ID NO:18 FGF2 native 
auggcagccg ggagcaucac cacgcugccc gccuugcccg aggauggcgg
cagcggcgcc uucccgcccg gccacuucaa ggaccccaag cggcuguacu
gcaaaaacgg gggcuucuuc cugcgcaucc accccgacgg ccgaguugac
gggguccggg agaagagcga cccucacauc aagcuacaac uucaagcaga agagagag-
ga guugugucua ucaaaggagu gugugcuaac cguuaccugg cuaugaagga agaug-
gaaga uuacuggcuu cuaaaugugu uacggaugag uguuucuuuu uugaacgauu
ggaaucuaau aacuacaaua cuuaccgguc aaggaaauac accaguuggu augug-
gcacu gaaacgaacu gggcaguaua aacuuggauc caaaacagga ccugggcaga aa-
gcuauacu uuuucuucca augucugcua agagcuga
SEQ ID NO:19 FGF2 human mod 
auggcugcag gcaguaucac cacucuccca gcauugccug aagauggagg
uucaggcgcc uuuccuccag gccacuuuaa agaccccaag agacuguacu gcaa-
gaaugg uggguucuuc cugcgcauuc aucccgaugg acguguagac ggagucaggg
aaaagucaga uccgcacaua aagcuccagc uccaagcuga ggaaagaggg guugug-
ucca ucaaaggggu gugugccaau cgcuaucugg cgaugaaaga ggacggcaga
cuucuggcua gcaagugugu gacagacgag ugcuucuucu uugagcgguu
ggaguccaac aacuacaaca ccuaucgaag caggaaguac acgucuuggu augucg-
cacu gaaacggacu gggcaguaca agcuuggcag caagacagga ccuggucaga aa-
 gccauucu guuucugccc augucugcca aaaguuga
SEQ ID NO:20 FGF2 
auggccgcug gcucuauuac aacacugccc gcucugccug aggauggcgg aucuggug-
cu uuuccaccug gccacuucaa ggaccccaag cggcuguacu gcaagaacgg cggau-
ucuuc cugcggauuc accccgacgg aagaguggac ggcgugcggg aaaaaagcga
cccucacauc aagcuccagc ugcaggccga agagagaggc gucgucagua
ucaaaggcgu gugcgccaac agauaccugg ccaugaagga agauggccgg
cugcuggccu cuaagugcgu gaccgaugag ugcuucuucu ucgaacggcu ggaaa-
gcaac aacuacaaca ccuacagaag ccggaaguac accucuuggu acguggcccu
gaagcggacc ggccaguaua agcugggcuc uaagacaggc ccaggccaga ag-
gccauccu guuucugccu augagcgcca agagcuga
SEQ ID NO:21 FGF2 
auggcugcag gcagcaucac cacccuccca gcacugccug aggacggagg
uuccggcgcc uucccuccag gccacuucaa ggaccccaag cgccuguacu gcaa-
gaacgg uggguucuuc cugcgcaucc accccgaugg ccgugucgac ggcgucaggg
agaaguccga cccgcacaua aagcuccagc uccaggcuga ggagagaggg gucgug-
ucca ucaagggggu gugcgccaau cgcuaucugg cgaugaagga ggacggcagg
cuccuggcua gcaagugugu gaccgacgag ugcuucuucu uugagcggcu
ggaguccaac aacuacaaca ccuaccgaag ccgcaaguac acgagcuggu acgucg-
cacu gaagcggacu gggcaguaca agcugggcag caagacagga ccuggucaga ag-
gccauccu guuccugccc auguccgcca agagcuga
SEQ ID NO:22 FGF2 
auggcagcag guaguauuac cacucuuccu gcuuugccug aagauggugg uucaggug-
cu uuuccuccag gucauuucaa agauccuaag agauuguauu guaagaacgg aggau-
ucuuu cugagaauac acccagaugg cagaguugau gguguccgug aaaagucuga
uccucacauc aagcugcagc uucaagccga agagagggga guugugucua
ucaaaggugu gugugcuaau agauaccugg cuaugaaaga agaugguaga
cuucuggcau caaagugugu gacggaugaa ugcuucuuuu ucgagcguuu
ggaauccaac aauuacaaca cauaccguag cagaaaguac acaaguuggu auguug-
cacu gaaacgaaca ggucaguaua aacuggguuc uaaaacagga ccaggacaga ag-
gcgauuuu guuucuuccg augucugcua agucuuga
SEQ ID NO:23 FGF2 
auggccgccg gcagcaucac cacccugccc gcccugcccg aggacggcgg
cagcggcgcc uucccccccg gccacuucaa ggaccccaag cgccuguacu gcaa-
gaacgg cggcuucuuc cugcgcaucc accccgacgg ccgcguggac ggcgugcgcg
agaagagcga cccccacauc aagcugcagc ugcaggccga ggagcgcggc guggugag-
 
ca ucaagggcgu gugcgccaac cgcuaccugg ccaugaagga ggacggccgc
cugcuggcca gcaagugcgu gaccgacgag ugcuucuucu ucgagcgccu ggagag-
caac aacuacaaca ccuaccgcag ccgcaaguac accagcuggu acguggcccu gaa-
gcgcacc ggccaguaca agcugggcag caagaccggc cccggccaga aggccauccu
guuccugccc augagcgcca agagcuaa
SEQ ID NO:24 FGF2 
auggccgcug gcagcaucac aacauugccu gcucugccug aggauggcgg cucuggug-
cu uuuccaccug gccacuucaa ggaccccaag cggcuguacu gcaagaacgg cggau-
ucuuc cugcggauuc accccgacgg aagaguggac ggcgugcggg aaaaaagcga
cccucacauc aagcuccagc ugcaggccga agagagaggc gucgucagua
ucaaaggcgu gugcgccaac agauaccugg ccaugaagga agauggccgg
cugcuggccu cuaagugcgu gaccgaugag ugcuucuucu ucgaacggcu ggaaa-
gcaac aacuacaaca ccuacagaag ccggaaguac accucuuggu acguggcccu
gaagcggacc ggccaguaua agcugggcuc uaagacaggc ccaggccaga ag-
gccauccu guuucugccu augagcgcca agagcuga
SEQ ID NO:25 FGF2 
auggcagcag gcagcaucac uaccuugccc gcccuuccgg aagauggggg aa-
gcggggcc uuccccccag ggcacuuuaa ggauccgaag cgacuguauu guaaaacgg
gggcuucuuu cuucggaucc auccagaugg ccgaguagac ggcguccgag aaaa-
gaguga cccccauauc aaacuucagc uccaggccga ggaaaggggu guggugagua
uaaagggggu gugcgcgaau cgauaccuug cuaugaagga ggacggucgc
cuucucgcca gcaaaugcgu gacugacgag ugcuucuuuu ucgagcgauu
ggaauccaac aauuacaaca cauaccggag uagaaaauau accuccuggu au-
guagcgcu gaaaaggacc gggcaguaua agcucgggag uaaaaccggu ccgggccaaa
aagcaauacu guuucuuccc augagcgcca aauccuga
SEQ ID NO:26 FGF2 
auggccgcgg gcucaauaac cacgcuuccu gcccugcccg aggacggggg aucaggug-
ca uucccuccag gccacuuuaa agaucccaaa cgacuguacu gcaaaacgg
cggcuuuuuc uugcgaaucc aucccgacgg gagaguugau ggugucagag aaaaaa-
guga cccgcacaua aagcuccaac ugcaagcgga agaaaggggc guugucucca uu-
aaaggagu gugcgcgaau agguaccugg cuaugaagga ggacggacga uugcucgccu
caaagugcgu aaccgaugag ugcuuuuuuu ucgagcggcu cgaaucaaac aau-
uacaaca cauaccgaag ccgcaaguac acgucuuggu acgucgcccu gaagaggacg
ggacaguaca aacucggguc aaaaaccggc cccggacaaa aggcuauccu
cuuucucccu auguccgcaa aaucuuga
SEQ ID NO:27 FGF2 
auggcagcug gcucuauuac uacgcugccg gcucucccug aggacggagg
cuccggugcc uuccccccag ggcacuuuaa agauccaaaa aggcuuuauu
guaaaaacgg cggguuuuuu cuccggaucc accccgacgg ccgcguagau ggagug-
aggg aaaagagcga cccucauaua aaacugcagc ugcaggcuga ggagcgggga
gucguuucga ucaaaggggu cugcgcaaac cgcuaccuug caaugaagga agacggaa-
ga cuccuagcga guaaaugugu gacagaugaa ugcuucuucu uugagagacu
ggaguccaau aauuauaaca ccuacagaag ccgaaaguau acuaguuggu acgug-
gccuu gaagcguacc ggucaauaca agcugggcuc uaagacaggu cccgggcaga ag-
gccauuuu auucuugccu augucagcca agucauga
SEQ ID NO:28 FGF2 
auggcagccg guucgauuac uacccuaccu gcccucccgg aagauggugg aaguggcg-
ca uuuccuccag gacauuuuaa ggauccaaaa cgccuguacu gcaagaaugg uggau-
ucuuu uuacgcauuc accccgaugg gcgagucgac gggguccgug aaaaguccga
cccccacauc aaacuccagu ugcaagcuga ggagagaggc gugguuucaa
ucaagggcgu augcgcuaau agauaucuug ccaugaagga ggacgggcgg
cuccuggccu caaaaugugu gacugacgag uguuuuuucu ucgagcggcu
ggaauccaac aacuacaaca cauacaggag uagaaaauac accucuuggu augug-
gcacu uaaaaggacg ggacaguaua aguugggguc uaagacaggc ccuggccaga aa-
gcgauacu guuccugccc augagcgcua agagcuga
SEQ ID NO:29 FGF2 
auggccgcag gcagcauuac cacucuuccu gccuugccug aggacggugg uucaggug-
cu uuuccuccag gucauuucaa agacccuaag cgacucuauu gcaagaacgg ag-
gcuucuuu cugaggauac acccagacgg cagaguugac gguguccgug aaaagucuga
uccucacauc aagcugcagc uucaagccga agagagggga guugugucua
ucaaaggggu gugugcuaau cgguaccugg cuaugaaaga agaugguaga
cuccuggcau caaagugugu gacggaugag ugcuucuuuu ucgagcguuu
ggaguccaac aauuacaaca ccuaccguag cagaaaguac accaguuggu augug-
gcacu gaaacgaaca ggucaguaua aacuggguag caaaacagga ccaggacaga ag-
gcgauuuu guuucuuccg augucugcua agucuuga
SEQ ID NO:30 FGF7 consensus (optimized GC-rich sequences) 
auggchgcng gcwshauhac hacnyubcch gchyukccbg argayggvgg hwshgg-
bgch uuycchcchg gscacuuyaa rgayccsaar mgvcuguayu gyaaraaygg
bggvuuyuuy yukcgvauyc ayccmgaygg vmghgungay gghgusmgvg
araarwshga yccbcayaum aarcubcarc uscargcbga rgarmgvggb gubgus-
wsya uhaarggvgu gugygcsaay mgvuaycukg cbaugaarga rgaygghmgv
yubcusgcyw shaarugygu rachgaygag ugcuuyuuyu uygarcgvyu sgarws-
maac aayuacaaca cmuaymgvag ymgvaaruay acswsyuggu ayguvgcvcu
 
gaarmgsacb ggvcaguaya arcubggsws haaracmggh ccngghcara argchau-
hcu suuycubccy augwsygcma arwsyura
SEQ ID NO:31 FGF7 consensus (all optimized sequences) 
auggchgcng gywsnauhac hacnyunccn gchyubccbg argayggngg hwshgg-
bgch uuycchcchg gncayuuyaa rgayccnaar mgvyubuayu gyaaraaygg
nggvuuyuuy yunmgvauhc ayccmgaygg vmghgungay ggngusmgng
araarwshga yccbcayaum aarcubcary ubcargcbga rgarmgvggn gubgub-
wsna uhaarggngu vugygcnaay mgvuaycukg cnaugaarga rgayggnmgv
yubcuvgcnw shaarugygu racngaygar ugyuuyuuyu uygarmgnyu sgarws-
maay aayuayaaca cmuaymgnag ymgvaaruay acnwsyuggu aygungcvyu
kaarmgnacn ggncaruaya aryubggbws haaracmggh ccnggncara argcnau-
hyu vuuyyubccb augwshgcha arwshura
SEQ ID NO:32 FGF2 consensus (all optimized and native sequences) 
auggchgcng gbwsnauhac hacnyunccn gchyubccbg argayggngg hwshgg-
bgch uuyccncchg gncayuuyaa rgayccnaar mgvyubuayu gyaaraaygg
nggvuuyuuy yunmgvauhc ayccmgaygg vmghgungay ggngusmgng
araarwshga yccbcayaum aarcuncary ubcargcnga rgarmgvggn gubgub-
wsna uhaarggngu vugygcnaay mgnuaycukg cnaugaarga rgayggnmgv yun-
cuvgcnw shaarugygu dacngaygar ugyuuyuuyu uygarmgnyu sgarwshaay
aayuayaaya chuaymgnws hmgvaaruay acnwsyuggu aygungcvyu
kaarmgnacn ggncaruaya aryubggnws haaracmggh ccnggncara argcnau-
hyu nuuyyubccn augwshgcha arwshura
SEQ ID NO:33 FGF2 protein
SEQ ID NO:34 RPL4_fwd primer sequence
   agcgtggctg tctcctctc
SEQ ID NO:35 RPL4_rev primer sequence
   gagccttgaa tacagcaggc
SEQ ID NO:36 ACTB_fwd primer sequence
   ggctgtattc ccctccatcg
SEQ ID NO:37 ACTB_rev primer sequence
   ccagttggta acaatgccat gt
SEQ ID NO:38 stabilization sequence
   yccancccwn ucycc

The consensus sequences SEQ ID Nos:12, 13, 14, 30, 31 and 32 are given with the following IUPAC nomenclature:

**Table 1: IUPAC Nomenclature:**

| **Symbol** | **Description** | **Bases represented** | | | | |
|---|---|---|---|---|---|---|
| **A** | **A**denine | A | | | | |
| **C** | **C**ytosine | | C | | | |
| **G** | **G**uanine | | | G | | 1 |
| **T** | **T**hymine | | | | T | |
| **U** | **U**racil | | | | U | |
| **W** | **W**eak | A | | | T/U | |
| **S** | **S**trong | | C | G | | |
| **M** | a**M**ino | A | C | | | 2 |
| **K** | **K**eto | | | G | T/U | |
| **R** | pu**R**ine | A | | G | | |
| **Y** | p**Y**rimidine | | C | | T/U | |
| **B** | not A (**B** comes after A) | | C | G | T/U | |
| **D** | not C (**D** comes after C) | A | | G | T/U | 3 |
| **H** | not G (**H** comes after G) | A | C | | T/U | |
| **V** | not T (**V** comes after T and U) | A | C | G | | |
| **N** | any **N**ucleotide (not a gap) | A | C | G | T/U | 4 |
| **Z** | **Z**ero | | | | | 0 |

**Table 2:**

| Sequence ID | CAI (Human /HEK) | GC Content % |
|---|---|---|
| 18 | 0,76 | 51,70 |
| 19 | 0,80 | 52,13 |
| 20 | 0,93 | 57,90 |
| 21 | 0,87 | 61,11 |
| 22 | 0,72 | 44,00 |
| 23 | 0,99 | 67,30 |
| 24 | 0,92 | 58,10 |
| 25 | 0,75 | 53,20 |
| 26 | 0,74 | 52,60 |
| 27 | 0,76 | 51,50 |
| 28 | 0,76 | 50,74 |
| 29 | 0,77 | 49,10 |

Table 2: Codon Adaptation Index and GC content of FGF2 variants (SEQ ID NO:1 is the standard for expression; SEQ ID NOs:2, 3 and 5: higher; SEQ ID NO:4: lower; it is therefore preferred to have a CAI ≥ 0,77 and a GC content ≥51,7%; compared to the native sequences SEQ ID NOs:1, 18 and 28).

**Table 3:**

| Sequence ID | CAI (Human /HEK) | GC Content % |
|---|---|---|
| 1 | 0,74 | 39,5 |
| 2 | 0,81 | 47,17 |
| 3 | 0,94 | 55,00 |
| 4 | 0,89 | 56,92 |
| 5 | 0,75 | 39,70 |
| 6 | 1,00 | 57,60 |
| 7 | 0,99 | 61,20 |
| 8 | 0,79 | 43,20 |
| 9 | 0,79 | 46,70 |
| 10 | 0,71 | 42,90 |
| 11 | 0,74 | 44,60 |

Table 3: Codon Adaptation Index and GC content of FGF7 variants (SEQ ID NO:1 is the standard for expression; SEQ ID NOs:2, 3 and 5: higher; SEQ ID NO:4: lower; it is therefore preferred to have a CAI ≥ 0,74 and a GC content ≥39,5%; compared to the native sequences SEQ ID NOs:1, 18 and 28).

The invention is further explained by way of the following examples and the figures, yet without being limited thereto.

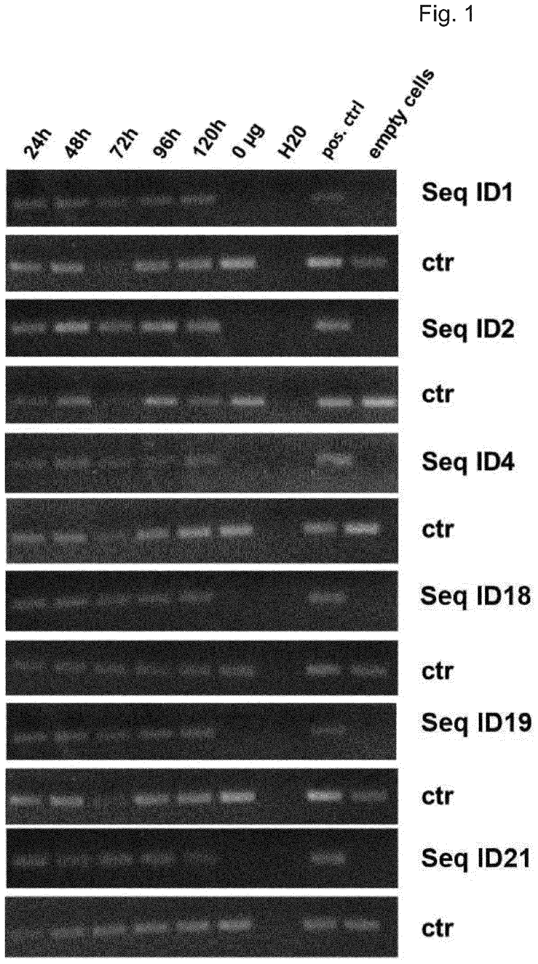

Figure 1 shows the RT-PCR based detection of IVT mRNA 24-120h post transfection of murine 3T3 cells (24-120h: samples taken 24-120h post transfection; 0µg: cells were treated with TransIT only and harvested 24h post transfection; H₂O: negative control containing no cDNA.; pos. control: cDNA from cellular RNA and IVT mRNA variants; empty cells: non-transfected 3T3 fibroblasts). SEQ ID NOs:1-4: analysis of respective human FGF7 IVT mRNA variants; SEQ ID NOs:18-21: analysis of respective human FGF2 IVT mRNA variants.

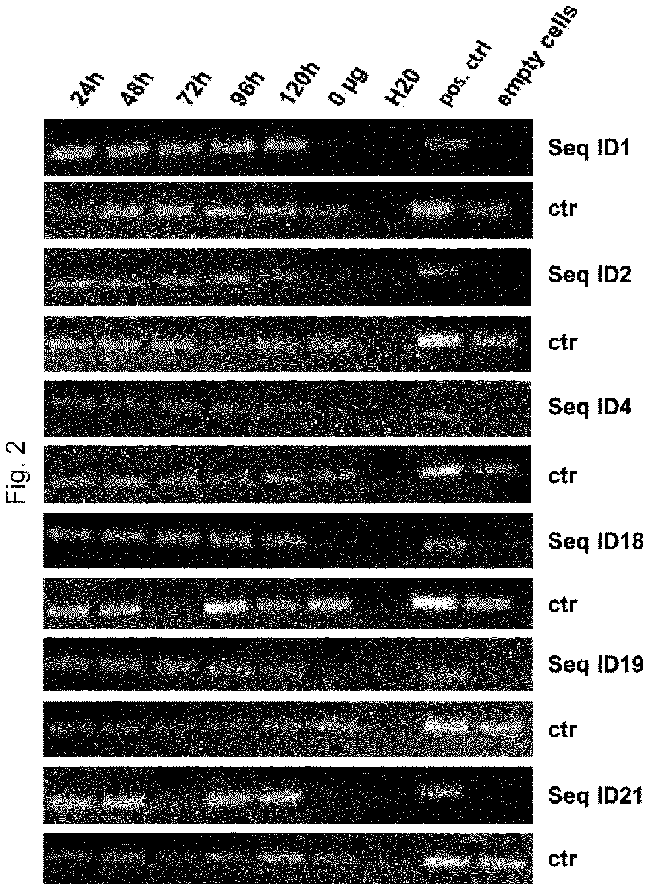

Figure 2 shows the RT-PCR based detection of IVT mRNA 24-120h post transfection of human BJ cells (24-120h: samples taken 24-120h post transfection; 0µg: cells were treated with TransIT only and harvested 24h post transfection; H₂O: negative control containing no cDNA.; pos. Control: cDNA from cellular RNA and IVT mRNA variants; empty cells: non-transfected fibroblasts). SEQ ID NOs:1-4: analysis of respective human FGF7 IVT mRNA variants; SEQ ID NO:18-21: analysis of respective human FGF2 IVT mRNA variants.

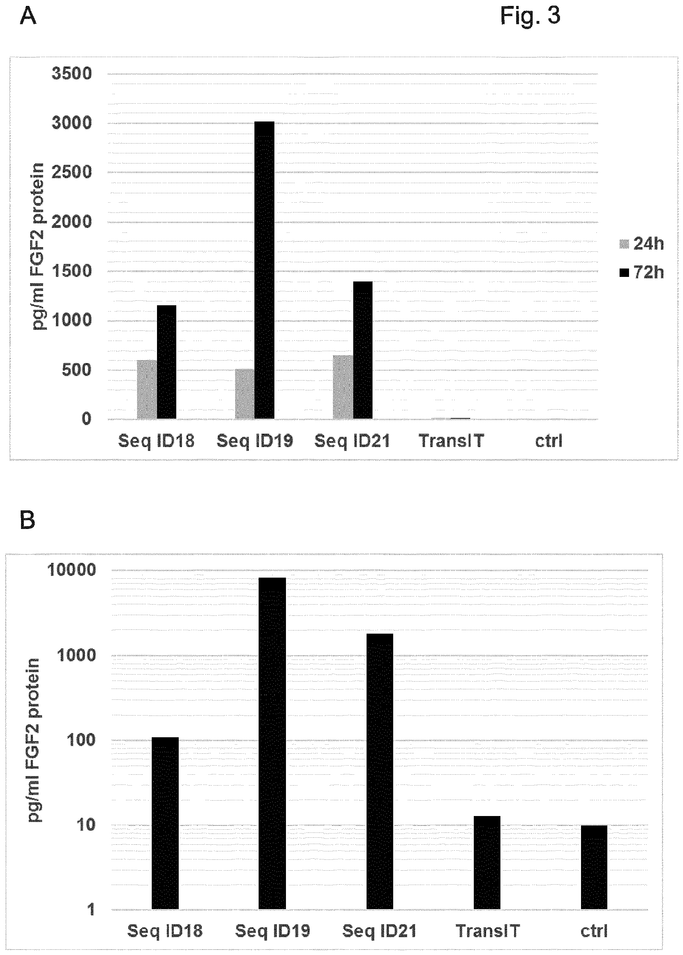

Figure 3 shows that IVT mRNA transfection of codon and GC content optimized FGF2 mRNA variants induces increased human FGF2 protein expression in murine 3T3 fibroblasts (SEQ ID NO: respective FGF2 mRNA sequence complexed with TransIT; TransIT: TransIT mRNA transfection reagent only; ctrl: buffer only; A: analysis at 24h and 72h post transfection; B: analysis at 120h post transfection).

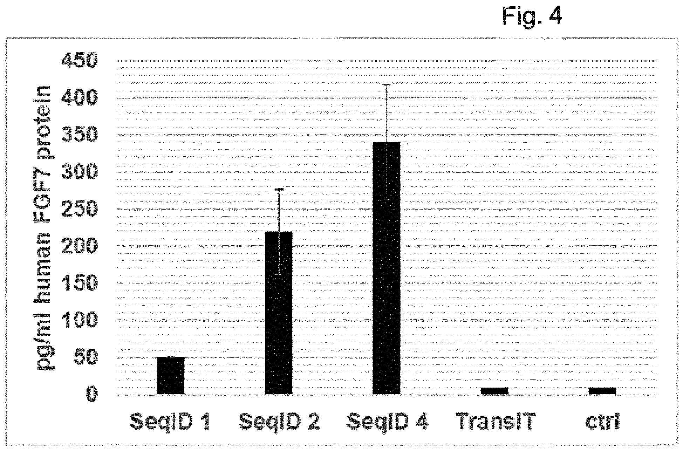

Figure 4 shows that IVT mRNA transfection of codon and GC content optimized FGF7 mRNA variants induces increased human FGF7 protein expression in murine 3T3 fibroblasts (SEQ ID NO: respective FGF7 mRNA sequence complexed with TransIT; TransIT: TransIT mRNA transfection reagent only; ctrl: buffer only; Analysis at 72h post transfection.

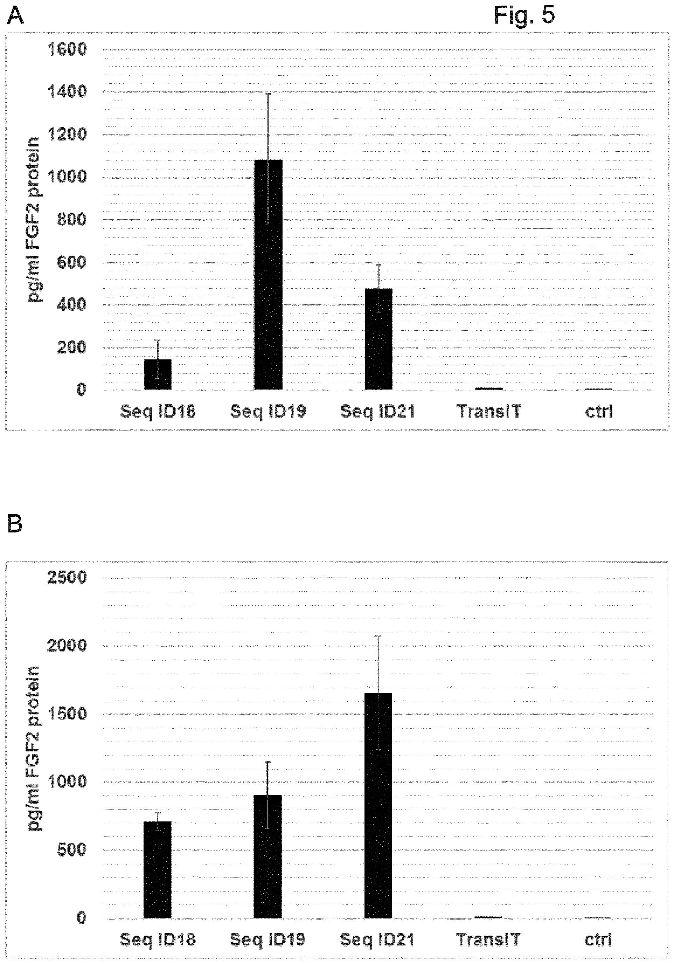

Figure 5 shows that IVT mRNA transfection of codon and GC content optimized FGF2 mRNA variants induces increased human FGF2 protein expression in human BJ fibroblasts (SEQ ID NO: respective FGF2 mRNA sequence complexed with TransIT; TransIT: TransIT mRNA transfection reagent only; ctrl: buffer only; Analysis at 24h post transfection (A) and 120h post transfection (B); error bars indicate SEM (samples analysed: n≤6).

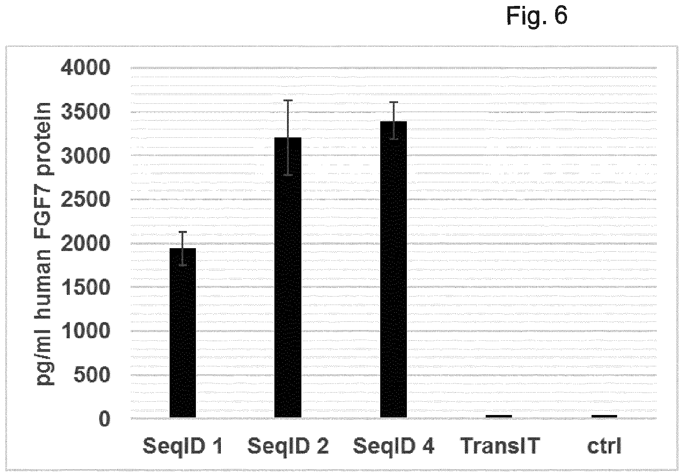

Figure 6 shows that IVT mRNA transfection of codon and GC content optimized FGF7 mRNA variants induces increased human FGF7 protein expression in human BJ fibroblasts (SEQ ID NO: respective FGF7 mRNA sequence complexed with TransIT; TransIT: TransIT mRNA transfection reagent only; ctrl: buffer only; Analysis at 48h post transfection.

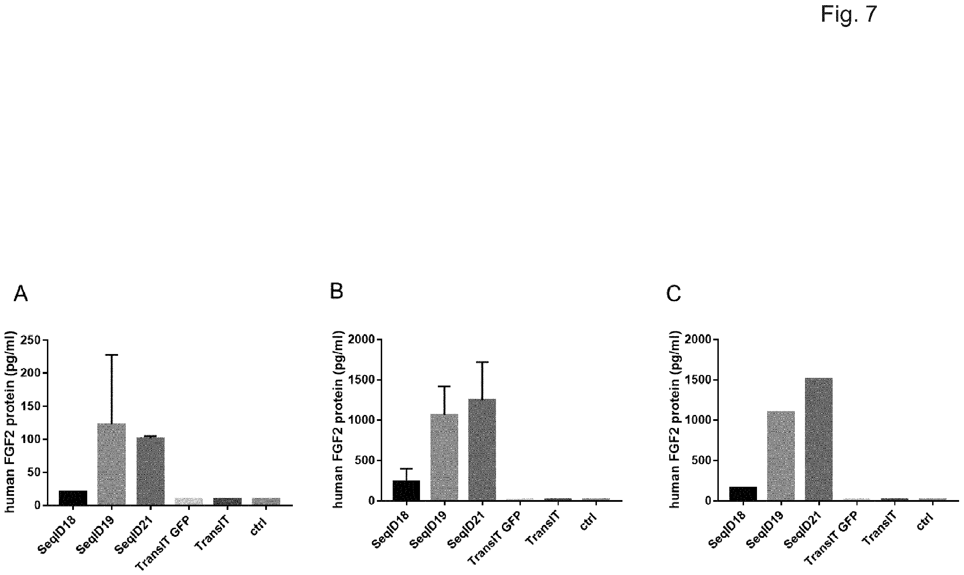

Figure 7 shows that IVT mRNA transfection of codon and GC content optimized mRNA variants induces increased human FGF2 protein expression in porcine skin epithelial sheets (SEQ ID NO: respective FGF2 mRNA sequence complexed with TransIT; TransIT: TransIT mRNA transfection reagent only; TransIT GFP: eGFP mRNA sequence complexed with TransIT; ctrl: buffer only; Analysis at 24h (A), 48h (B) and 72h (C) post transfection.

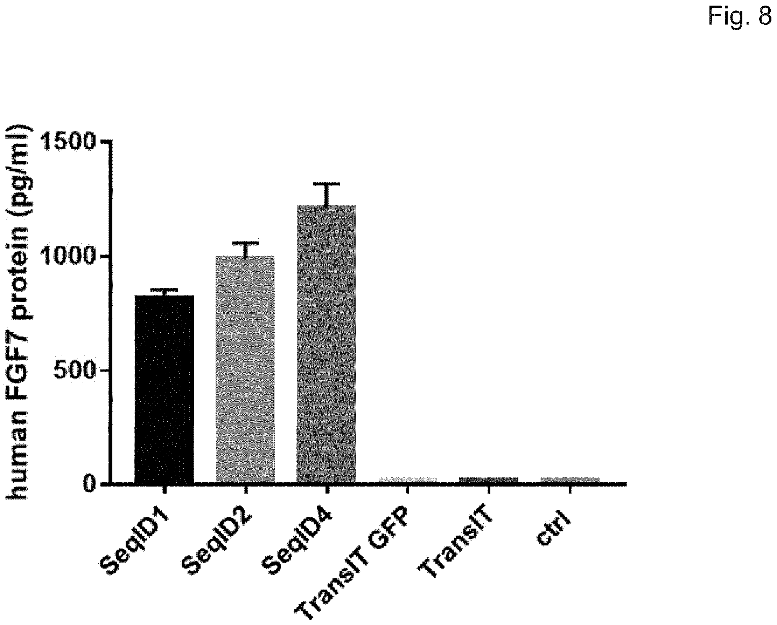

Figure 8 shows that IVT mRNA transfection of codon and GC content optimized mRNA variants induces increased human FGF7 protein expression in porcine skin epithelial sheets (SEQ ID NO: respective FGF7 mRNA sequence complexed with TransIT; TransIT: TransIT mRNA transfection reagent only; TransIT GFP: eGFP mRNA sequence complexed with TransIT; ctrl: buffer only; A: analysis at 24h post transfection.

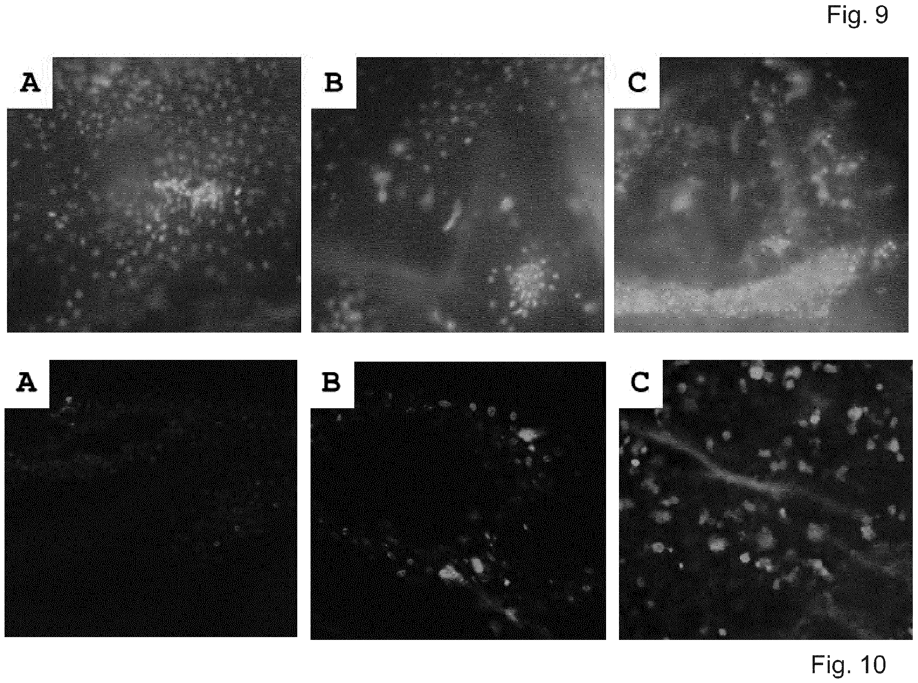

Figure 9 shows that EGFP mRNA transfection of porcine epithelial sheets using TransIT mRNA transfection reagent induces eGFP expression in porcine skin epithelial sheets (A: porcine skin transfected with liposomes only; B: porcine skin transfected with 0,5µg/ml eGFP IVTm RNA, formulated in TransIT; C: porcine skin transfected with 1µg/ml eGFP IVTm RNA, formulated in TransIT).

Figure 10 shows that eGFP mRNA transfection of porcine epithelial sheets using mRNA/Liposome complexes induces eGFP expression in porcine skin epithelial sheets (A: porcine skin transfected with liposomes only; B: porcine skin transfected with 2µg/ml eGFP IVTm RNA, formulated in liposomes; C: porcine skin transfected with 10µg/ml eGFP IVTm RNA, formulated in liposomes).

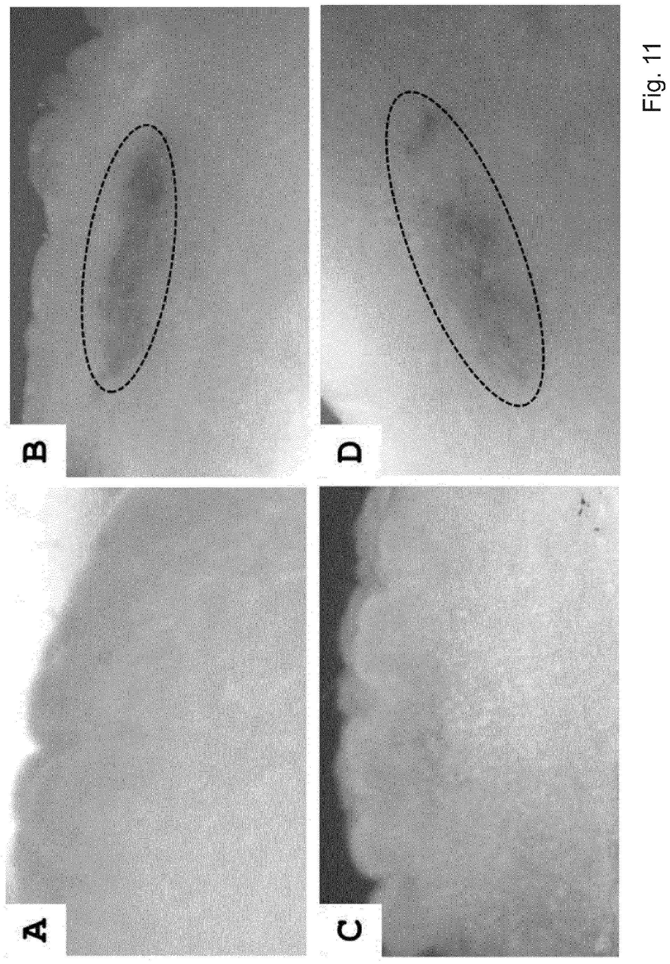

Figure 11 shows the detection of whole mount ß-Galactosidase (bGal) activity in porcine skin explants 24h after transfection with LacZ IVT mRNA (A: porcine skin transfected with DOTAP-liposomes only w/o Rnase inhibitor; B: porcine skin transfected with 5µg LacZ IVTm RNA, formulated in DOTAP-liposomes w/o Rnase inhibitor; C: porcine skin transfected with DOTAP-liposomes only + Rnase inhibitor; D: porcine skin transfected with 5µg LacZ IVTm RNA, formulated in DOTAP-liposomes w/o Rnase inhibitor; succesful transfection is highlighted in encircled areas in B and D, respectively).

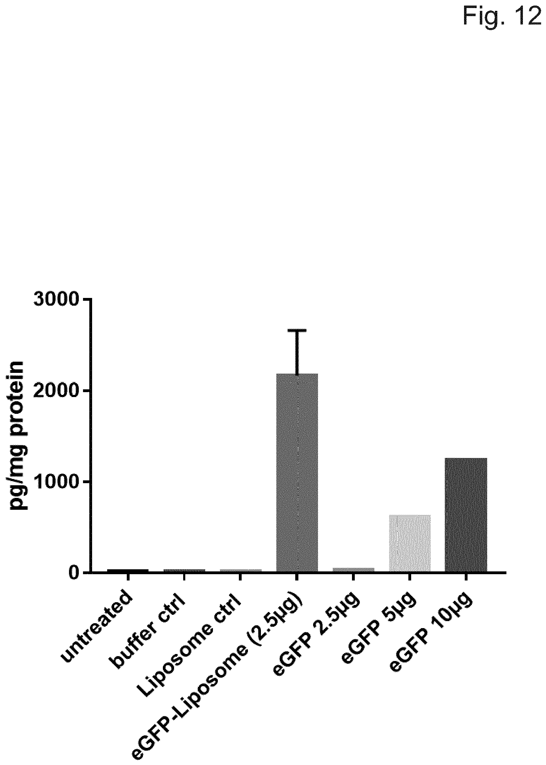

Figure 12 shows the detection of eGFP expression in porcine skin explants 24h after transfection with eGFP IVT mRNA (untreated: non-treated biopsy; LNP ctrl: porcine skin LNP control treated; eGFP-LNP: porcine skin transfected with mRNA-Lipid-Nano Particles (concentration shown: 2.5µg eGFP mRNA/dose); eGFP 2.5µg, eGFP 5µg and eGFP 10µg: porcine skin transfected with non-complexed eGFP IVT-mRNA (concentrations shown: 2,5+5+10µg mRNA/dose); buffer ctrl porcine skin treated with buffer only).

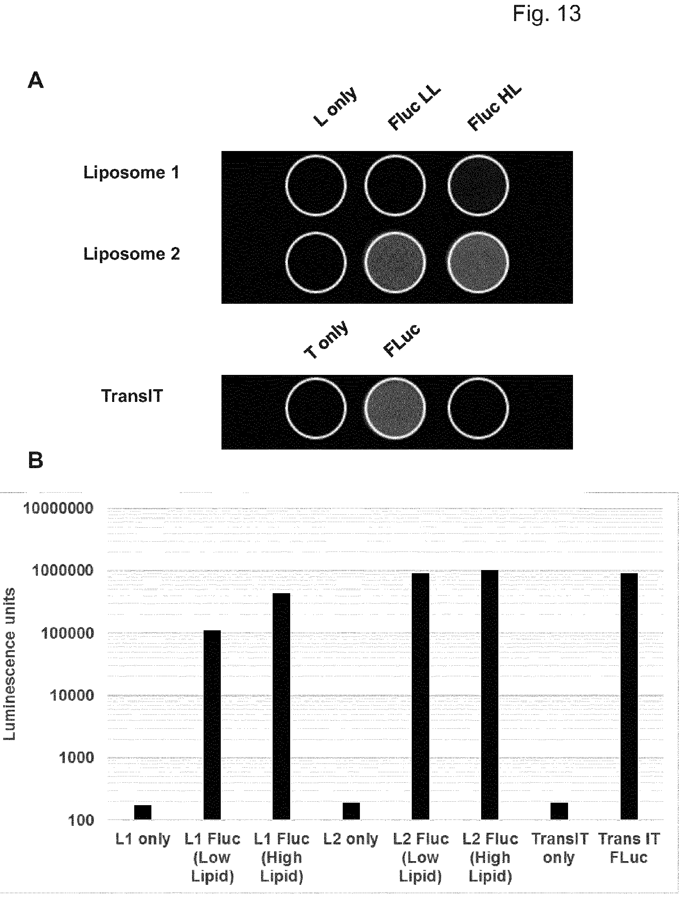

Figure 13 shows the detection of Firefly Luciferase (FLuc) expression in porcine skin epithelial sheets 24h after transfection with FLuc IVT mRNA (1µg mRNA/transfection) Porcine skin was transfected with 2 different liposomes only (L1 and L2 only) or Liposome mRNA complexes using a high Lipid to mRNA ratio (Fluc HL, high lipid) or a low lipid to mRNA ratio (Fluc LL, low lipid); Fluc mRNA complexed (TransIT Fluc) or non-complexed TransIT (TransIT only) was used as control A: Bioluminescense detection in a 96 well plate; B: detection of Luminescence units on an Tecan Infinite Luminescence reader.

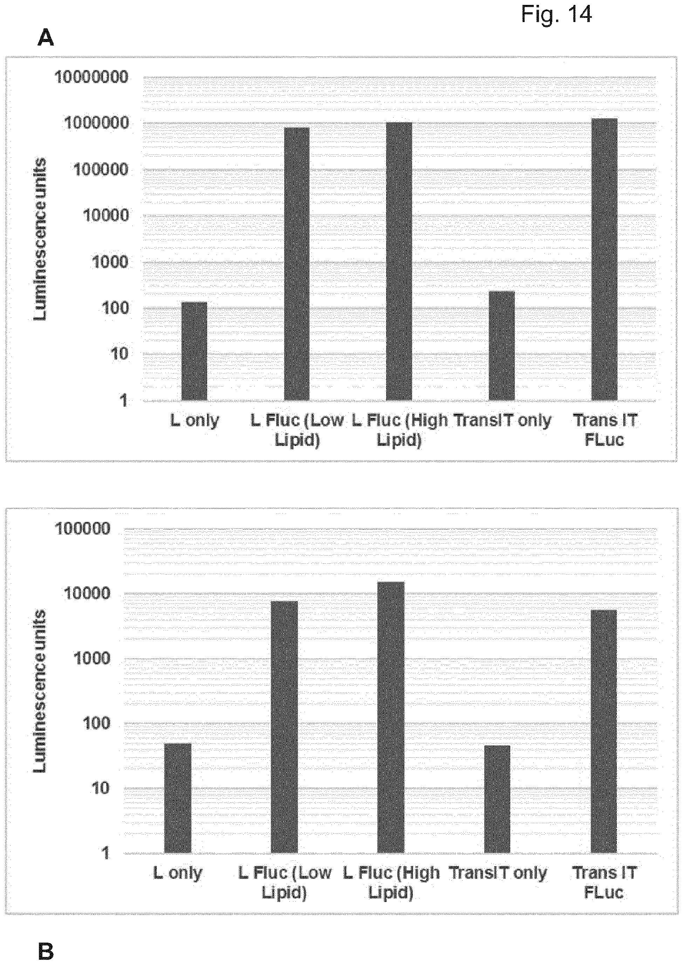

Figure 14 shows the detection of Firefly Luciferase (FLuc) expression in porcine skin explants 24h after transfection with FLuc IVT mRNA (1µg mRNA/transfection) Porcine skin was transfected with liposomes only (L only) or Liposome mRNA complexes using a high Lipid to mRNA ratio (L Fluc (High lipid)) or a low lipid to mRNA ratio (L Fluc (Low Lipid)); Fluc mRNA complexed (TransIT Fluc) or non-complexed TransIT (TransIT only) was used as control A: detection of Luminescence units from epithelial sheets 24h post transfection; B: detection of Luminescence units from dermal explants 24h post transfection.

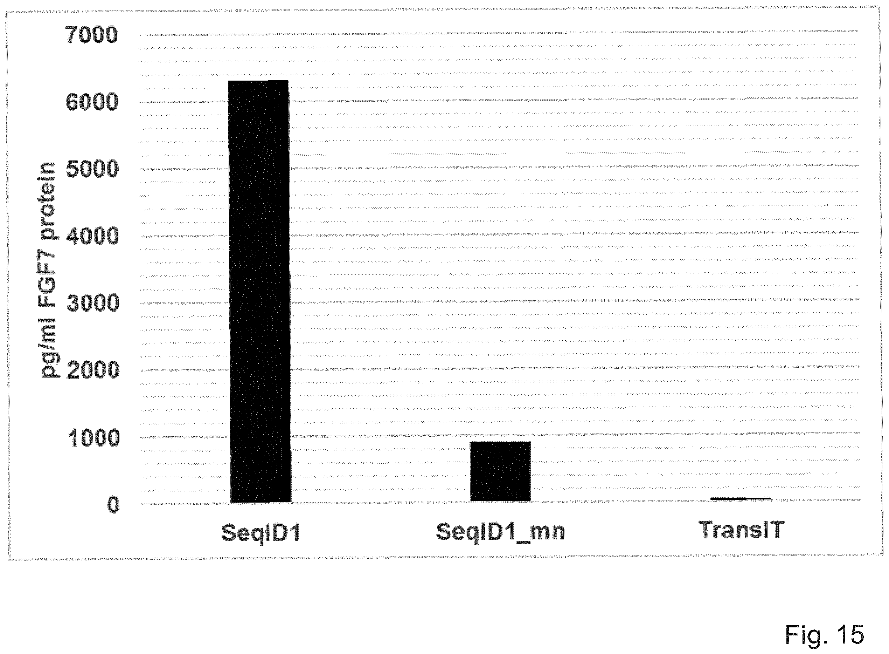

Figure 15 shows that IVT mRNA transfection of native FGF7 mRNA using non modified nucleotides induces increased human FGF7 protein expression in murine 3T3 fibroblasts as compared to a IVT mRNA containing modified nucleotides (exchange of C and 5meC as well as U and pseudoU nucleotides during in vitro transcription) (SEQ ID1: non modified FGF7 mRNA sequence complexed with TransIT; Seq ID1_mn: non modified FGF7 mRNA sequence containing modified nucleotides complexed with TransIT TransIT: TransIT mRNA transfection reagent only; Analysis at 24h post transfection.

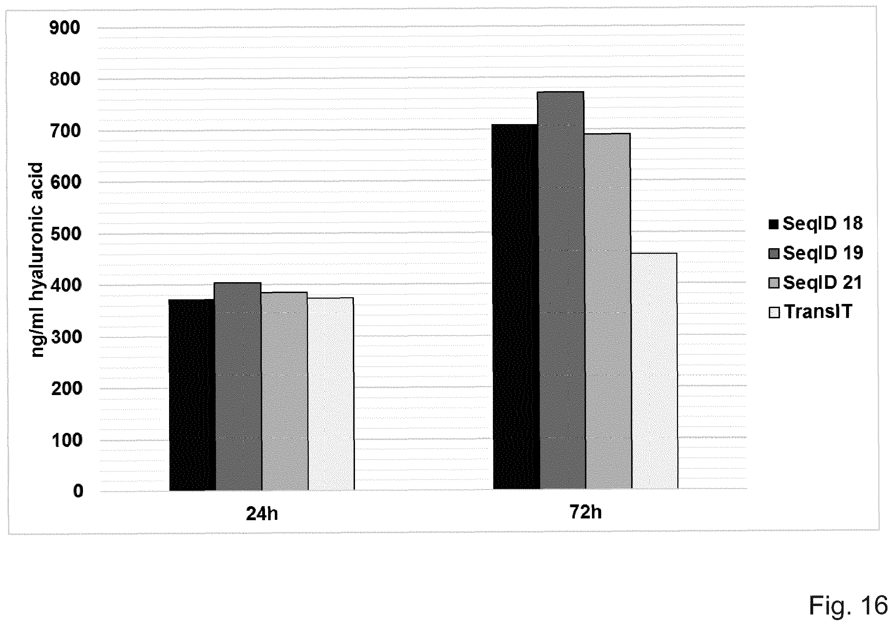

Figure 16 shows that IVT mRNA transfection of codon and GC content optimized FGF2 mRNA variants induces increased hyaluronic acid synthesis in murine 3T3 fibroblasts (SEQ ID NO: respective FGF2 mRNA sequence complexed with TransIT; TransIT: TransIT mRNA transfection reagent only;) Analysis has been performed at 24h and 72h post transfection.

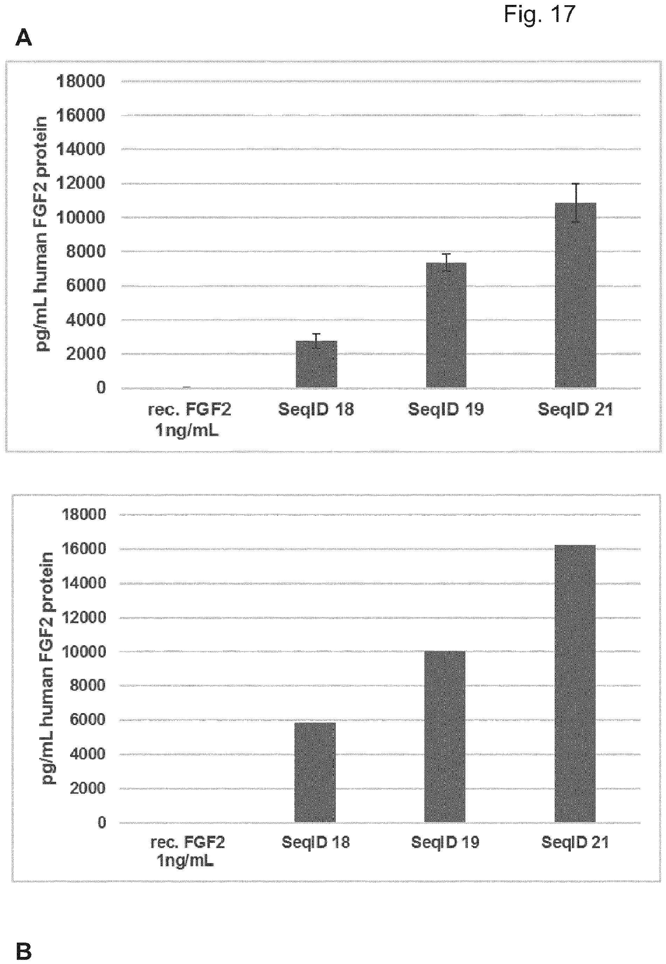

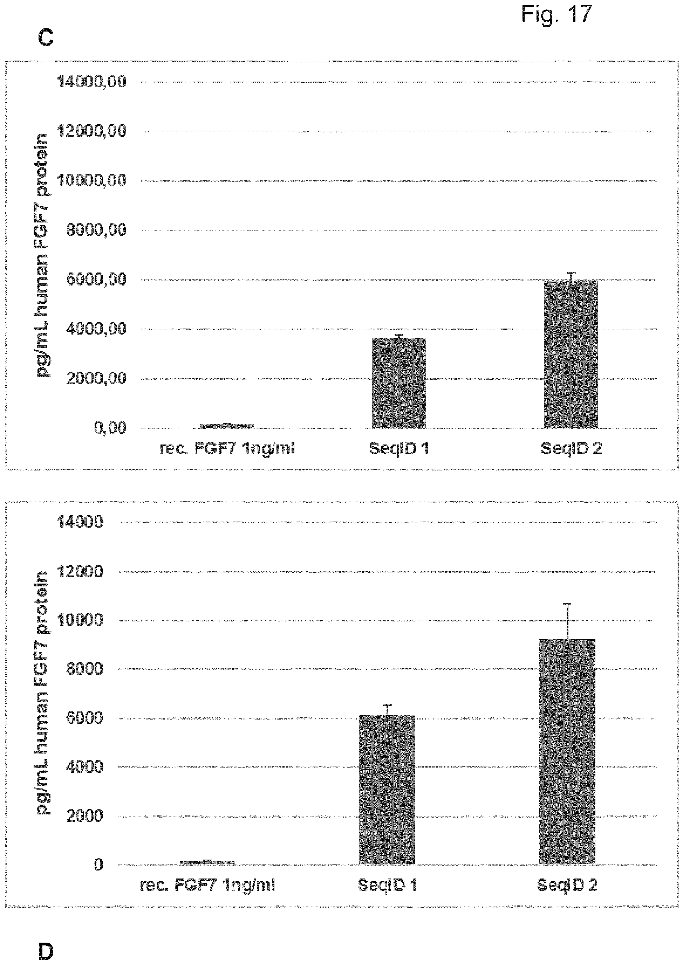

Figure 17 shows that in contrast to IVT mRNA induced protein, recombinant FGF protein is unstable in 3T3 cells and porcine primary fibroblasts 24h after transfection/addition. No remaining or newly synthesized FGF protein is detectable in all 4 experiments in the samples receiving rec. FGF proteins. SEQ ID NO: respective FGF2 and FGF7 mRNA sequence complexed with TransIT; rec. FGF2/7 1ng/ml: cells were exposed to recombinant FGF2 or FGF7 protein (1ng/ml); Analysis at 24h post transfection/protein addition; (A) FGF2 levels in 3T3 cells; (B) FGF2 levels in porcine primary fibroblasts; (C) FGF7 levels in 3T3 cells; (D) FGF7 levels in porcine primary fibroblasts.

### EXAMPLES:

### Material and methods:

### Transfection of murine 3T3 fibroblasts and human B.J. skin fibroblasts

For transfection, murine 3T3 fibroblasts and human B.J. skin fibroblasts were seeded at 4-6 x 10⁴ cells/well in 12-well plates. After 24 hours incubation in full EMEM or DMEM medium (Gibco, Thermo Fisher, USA), culture medium was replaced. Different formulations of IVT mRNA complexed with TransIT mRNA transfection reagent (Mirus Bio; complex formation according to manufacturer instructions) were prepared and added to the cells. 24 hours after transfection, medium was replaced with complete DMEM. Cell cultures were maintained under standard conditions for up to 5 days with daily medium changes until evaluation.

### Isolation and transfection of intact pig skin biopsies:

Full-thickness porcine skin flaps were isolated peri-mortally from pigs (samples were obtained in full compliance with national legislation (i.e. Tierversuchsgesetz 2012, TVG 2012 (BGBl. I Nr. 114/2012))) and disinfected using Octenisept® disinfectant (Schuelke + Mayr GmbH, Germany).

Transfection of intact pig skin was performed by direct, intradermal injection of the IVT mRNA solution (1-10µg mRNA/dose). LacZ IVT mRNA (completely modified using 5-methylcytidine, pseudouridine; Trilink Inc., USA) was formulated using either TransIT®-mRNA Transfection kit (Mirus Bio™) according to manufacturer instructions (with slight modification according to Kariko et al.; Mol. Ther. 2012. 20(5): 948-53) or DOTAP based liposomal formulations (Sigma Aldrich, USA). DOTAP based formulations were prepared using a lipid/RNA ratio of 5/1 (µg/µg). In addition, mRNA complexes were also supplemented with RNAse Inhibitor (5U/dose, RNasin, Promega, USA). Injection volume ranged from 20µL to 30µL.

Alternatively, transfection of intact pig skin was performed by direct, intradermal injection of eGFP IVT mRNA solution (0.5-25µg mRNA/dose). eGFP IVTmRNA (AMPTec, Germany) was formulated using either TransIT®-mRNA Transfection kit (Mirus Bio™) according to manufacturer instructions (with slight modification according to Kariko et al., 2012, or DOTAP based liposomal formulations (Sigma Aldrich, USA), or Lipid-Nano-particle formulations (Polymun, Austria) or SAINT based liposomal formulations (Synvolux, Netherlands). DOTAP based liposomal formulations were prepared using a lipid/RNA ratio of 5/1 (µg/µg). SAINT lipid based formulations were prepared using a lipid/RNA ratio of 2.5-4/1 (µg/µg). In addition, non-complexed mRNA in physiologic buffer was applied intradermally with an injection volume range from 20µL to 30µL.

After injection, punch biopsies of the injected areas (8mm diameter) were sampled, subcutaneous fat was removed and biopsies were transferred into standard complete culture medium in a petridish, epidermis facing the air-liquid interface (5mL; containing: Dulbecco's Modified Eagle Medium with GlutaMAX (DMEM), 10% FCS, 1X Penicillin-Streptomycin-Fungizone; obtained from Gibco, Life Technologies). Subsequent culture was performed at 37°C/5% CO₂ for 24 hours, at which point biopsies were normally harvested.

### Isolation and transfection of porcine epithelial sheets and dermal explants

Full-thickness porcine skin flaps were isolated peri-mortally from pigs (samples were obtained in full compliance with national legislation (i.e. Tierversuchsgesetz 2012, TVG 2012)) and disinfected using Octenisept® disinfectant (Schuelke + Mayr GmbH, Germany). Punch biopsies (6 or 8 mm diameter) were harvested from full-thickness skin flaps, subcutaneous fat removed, and biopsies were cut in two parts. Immediately afterwards, cut biopsies were transferred, epidermis facing the air-liquid interface, to 9 cm (diameter) petri-dishes containing 5 mL Dispase II digestion solution (ca. 2.5 Units/mL; Dispase II; Sigma Aldrich, USA). Subsequent digestion was performed at 4°C overnight. Dispase II digestion solution was prepared by diluting Dispase II stock solution (10 mg/mL in 50mM HEPES/150 mM NaCl; pH-7.4) 1:2 with 1x DMEM (Gibco) and adding 1x Penicillin/Streptomycin. Epidermal sheets were then separated from the underlying dermis (i.e. dermal explants) using forceps and transferred into DMEM for a short (5 min.) washing step. Subsequently sheets and dermal explants were put into complete DMEM culture medium and incubated at 37°C/5% CO₂ (6 to 8 hours) until transfection was performed in 24-well culture plates. Transfection of porcine epidermal sheets and dermal explants was performed using eGFP IVT mRNA (AmpTec, Germany), Firefly Fuciferase (FLuc) IVT mRNA or IVT mRNA constructs for FGF (e.g.: SEQ ID NOs:1-4 and 18-21). mRNA was formulated using either TransIT®-mRNA Transfection kit (Mirus Bio™) according to manufacturer instructions or liposomal formulations (Polymun, Austria) or SAINT based liposomal formulations (Synvolux, Netherlands). SAINT lipid based formulations were prepared using a lipid/RNA ratio of 2.5 (µg/µg) (Low Lipid formulation) or 4/1 (µg/µg) (High Lipid formulation). Liposomal formulations were prepared using a lipid/RNA ratio of 5/1 (µg/µg). All lipoplex solutions for transfection contained 0.1µg to 10µg mRNA/mL DMEM medium and epidermal sheets and dermal explants were cultured one to three days.

For analysis, tissue culture supernatants were collected for ELISA analysis. Sheets were harvested for RNA and protein extraction and subsequent analysis by qPCR and ELISA, respectively. eGFP transfected epidermal sheets were also analysed for eGFP expression by direct fluorescence microscopy and immuno-histochemistry detecting eGFP in situ. FLuc transfected epidermal sheets and dermal explants were analysed for Luciferase activity on a Tecan Infinite multimode reader.

### RT-PCR analysis of cells transfected using IVT mRNA preparations

Human B.J. cells and murine 3T3 fibroblasts were transfected using 1µg FGF2 or FGF7 IVT mRNAs complexed with TransIT mRNA transfection reagent. Total cellular RNAs were isolated from murine and human fibroblasts or porcine epithelial sheets at different time points post transfections using Tri-Reagent (Thermo Fisher, USA, according to manufacturer instructions) and mRNAs were reverse transcribed into cDNA by conventional RT-PCR (Pro-toscript First Strand cDNA synthesis kit, New England Biolabs, according to manufacturer instructions). cDNA samples were then subjected to conventional PCR and qPCR. Primers were obtained from Invitrogen.

PCR analysis detecting FGF variants was performed from cDNA obtained from cells and/or sheets transfected with different FGF2 and FGF7 variants using Platinum Taq Polymerase (Invitrogen, USA) and FGF variant specific primers (Invitrogen, USA). Primers for native FGF2 mRNA have been obtained from Biorad (USA). Human RPL4 and murine ACTB (Eurofins Genomics) were used as positive controls. PCR products were analysed using conventional agarose gel electrophoresis. qPCR was performed using the Luna® Universal qPCR Master Mix (New England Biolabs, USA, according to manufacturer's protocol) on a CFX Real-Time PCR Detection System (Biorad). Level and stability of IVT mRNAs was determined relative to internal standards to normalize for variances in the quality of RNA and the amount of input cDNA (e.g.: murine ACTB or human RPL4, respectively)

**Table 4: PCR primers**

| **Primer (Producer, SEQ ID NO:)** | **Sequence** |
|---|---|
| huRPL4_fw (EG, 34) | 5'-AGC GTG GCT GTC TCC TCT C-3' |
| huRPL4_rev (EG, 35) | 5'-GAG CCT TGA ATA CAG CAG GC-3' |
| muACTB_fw (EG, 36) | 5'-GGC TGT ATT CCC CTC CAT CG-3' |
| muACTB_rev (EG, 37) | 5'-CCA GTT GGT AAC AAT GCC ATG T-3' |
| F2_hum_3 fw (IVG, 38) | 5'-CGT GTA GAC GGA GTC AGG GA-3' |
| F2_hum_3 rev (IVG, 39) | 5'-GCA CAC ACC CCT TTG ATG GA-3' |
| F2_GC_3 fw (IVG, 40) | 5'-GCC TGT ACT GCA AGA ACG GT-3' |
| F2_GC_3 rev (IVG, 41) | 5'-CTG GAG CTT TAT GTG CGG GT-3' |
| F7_hum_1 fw (IVG, 42) | 5'-TTC GCA TCG ACA AAC GGG GT-3' |
| F7_hum_1 rev (IVG, 43) | 5'-GCG ACA ATC CCG ACT GCA AC-3' |
| F7_GC_3 fw (IVG, 44) | 5'-TGC ACA AGT GGA TCC TCA CA-3' |
| F7_GC_3 rev (IVG, 45) | 5'-CCA GTG AGA TAG TGC CCA CC-3' |
| F7_nat_2 fw (IVG, 46) | 5'-TGA ACT GTT CCA GCC CTG AG-3' |
| F7_nat_2 rev (IVG, 47) | 5'-TCA GGT ACC ACT GTG TTC GAC-3' |

| | |
|---|---|
| EG: Eurofins Genomics, IVG: Invitrogen | |

### Analysis of IVT mRNA induced human FGF2 and FGF7 protein

Murine 3T3 cell, human B.J. cells, porcine primary fibroblasts and porcine epithelial sheets were transfected using 0.1-1µg IVT mRNA for different FGF2 and FGF7 variants complexed with TransIT mRNA transfection reagent and cultured for up to 120h post transfection. Supernatants from transfected cells and epithelial sheets were obtained at several time points after transfection; cells were harvested at the same time points and protein was extracted. Protein was extracted using a cell extraction buffer (10mM HEPES, 10mM KCl, 0.1µM EDTA, 0.3% NP40 and Roche Protease Inhibitor, according to manufacturer's protocol). FGF determination in supernatants was performed using human FGF2 and FGF7 ELISA kits (Duo Set ELISA kits, R and D systems, Biotechne, USA, according to manufacturer's instructions), and measured on an Infinite 200 PRO multimode reader (Tecan AG, Switzerland).

### Analysis of IVT mRNA induced eGFP protein

Intact porcine skin explants and porcine epithelial sheets were transfected using 0.1-10µg eGFP IVT mRNA complexed with TransIT mRNA transfection reagent or different liposomal carriers or uncomplexed ("naked" in physiologic buffer) and cultured for 24h post transfection. Samples were harvested and protein extracted using cell extraction buffer (10mM HEPES, 10mM KCl, 0.1µM EDTA, 0.3% NP40 and Roche Protease Inhibitor, according to manufacturer's protocol). eGFP determination was performed using the GFP in vitro SimpleStep ELISA® kit (Abcam Plc., UK, according to manufacturer instructions), and measured on an Infinite 200 PRO multimode reader (Tecan AG, Switzerland).

### Detection of beta-galactosidase activity in porcine tissue

Whole-mount beta-galactosidase (bGal) staining of biopsies was performed in 24 well culture plates for 24 or 48 hours at 37°C. Positive staining controls were generated by injecting bGal enzyme (1U recombinant bGal protein/ injection) intradermally into pig skin. Before staining, biopsies were mildly fixed in a 4% formaldehyde solution with PBS for 1 hour at room temperature. After fixation, samples were washed in PBS (3x) and subsequently equilibrated in LacZ-washing buffer (2mM MgCl₂, 0.01% Na-deoxycholate and 0.02% NP-40 dissolved in PBS). After equilibration, samples were stored overnight (4°C). Samples were then incubated in staining solution at 37°C and the colour reaction monitored. The staining solution was freshly prepared (5mM K₄Fe(CN)₆ and 5mM K₃Fe(CN)₆ in LacZ buffer) and 1 mg/mL 5-Bromo-3-indolyl β-D-galactopyranoside, (Bluo-Gal) was added as colour substrate. If staining was performed for 48 hours the staining solution was substituted after 24 hours. Staining volume was generally 0.5 mL/well. Staining was stopped by washing in LacZ washing buffer and 3x PBS. Samples were subsequently either fixed overnight in buffered 4% formaldehyde solution and further processed for standard histology or were frozen in optimal cutting temperature compound (OCT) for subsequent histologic analyses.

### Analysis of IVT mRNA induced FLuc protein

Porcine epithelial sheets and dermal explants were transfected using 2µg/ml FLuc IVT mRNA complexed with TransIT mRNA transfection reagent or SAINT lipid based formulations and cultured for 24h post transfection. Samples were harvested and subjected to direct Luciferase activity measurement. Measurements were performed using Firefly Luc One-Step Glow Assay Kit (Thermo Scientific, USA, according to manufacturer's instructions) and analysed on an Infinite 200 PRO multimode reader (Tecan AG, Switzerland). In addition, samples were also assessed on a GelDoc-system directly detecting Luminescence.

### Analysis of IVT mRNA induced secretion of Hyaluronic acid

Murine 3T3 cells were seeded at 5 x 10⁴ cells/well in 12-well plates. After 24 hours incubation in full DMEM medium (Gib-co, Thermo Fisher, USA), culture medium was replaced. Different formulations of IVT mRNA encoding for FGF2 and FGF7 complexed with TransIT mRNA transfection reagent (Mirus Bio; complex formation according to manufacturer instructions) were prepared and added to the cells.

For analysis of Hyaluronic acid secretion, supernatant from transfected cells was obtained 24 to 120h hours after transfection. Supernatants were subjected to analysis of FGF induced Hyaluronan production using the Hyaluronan Quantikine ELISA Kit (R and D systems, Biotechne, USA, according to manufactuer's instructions). ELISA measurements were taken on an Infinite 200 PRO multimode reader (Tecan AG, Switzerland).

### Transfection of porcine primary skin fibroblasts

For transfection, porcine primary skin fibroblasts were seeded at 5 x 10³ cells/well in 96-well plates. After 24 hours incubation in full FGM-2 Fibroblast medium (LONZA, Switzerland), culture medium was replaced with complete DMEM. Different formulations of IVT mRNA complexed with TransIT mRNA transfection reagent (Mirus Bio; complex formation according to manufacturer instructions) were prepared and added to the cells. 24 hours after transfection, medium was replaced with complete DMEM. Cell cultures were maintained under standard conditions for up to 2 days with daily medium changes until evaluation.
**Example 1:** Detection of mRNA encoding different FGF2 and FGF7 variants by FGF-variant specific PCR from cDNA obtained from murine 3T3 fibroblast cells 24h-120h post transfection.
In order to assess whether FGF2 and FGF7 mRNA variants are stable in murine fibroblasts over prolonged time periods, different mRNAs encoding SEQ ID NOs:1-4 and 18-21 were used to transfect 3T3 cells. RNA was isolated at different time points (24-120h post transfection) and the presence of mRNA in transfected cells determined up to 120h post transfection by non-quantitative RT PCR.
Result: As shown in Figure 1, all FGF mRNA variants can be detected following transfection using 1µg mRNA/ml at all time points assessed, showing mRNA stability, irrespective of codon optimization or GC content in human cells for ≥120h.
Figure 1 shows 3T3 cells transfected without mRNA, or with 1µg mRNA complexed with TransIT mRNA transfection reagent. Total cellular RNAs were isolated at different time points after transfection (24h-120h) and mRNAs were reverse transcribed into cDNA by conventional RT-PCR. cDNA samples were then subjected to variant specific PCR using primers for SEQ ID NOs:1-4 and 18-21 for the detection of transfected FGF2 and FGF7 mRNAs and murine ACTB as a PCR control (shown as ctr). All FGF variants were stable over extended time periods in murine cells. It follows that there is differential expression and/or secretion of FGF as determined by the codon adaption index (CAI)and GC content.
**Example 2:** Detection of mRNA encoding different FGF2 and FGF7 variants by FGF-variant specific PCR from cDNA obtained from fibroblast cells 24h-120h post transfection.
In order to assess whether FGF mRNA variants are stable in human skin fibroblasts over prolonged time periods, different mRNAs encoding SEQ ID NOs:1-4 and 18-21 were used to transfect B.J. cells. RNA was isolated at different time points (24-120h post transfection) and the presence of mRNA in transfected cells was determined up to 120h post transfection by non-quantitative RT-PCR.
Result: As shown in Figure 2, all FGF mRNA variants can be detected following transfection using 1µg mRNA/ml at all time points assessed, showing mRNA stability, irrespective of codon optimization or GC content in human cells for ≥120h.
Figure 2 shows B.J. cells transfected without mRNA, or 1µg mRNA complexed with TransIT mRNA transfection reagent. Total cellular RNAs were isolated at different time points after transfection (24h-120h) and mRNAs were reverse transcribed into cDNA by conventional RT-PCR. cDNA samples were then subjected to variant specific PCR using primers for SEQ ID NOs:1-4 and 18-21 for detection of transfected FGF mRNAs and human RPL4 as PCR control (shown as ctr). All FGF variants were stable over extended time periods in human cells. mRNA was also detectable for extended time in porcine epithelial sheets. It follows that there is differential expression and or secretion of FGF2 and FGF7 according to CAI and GC content.
**Example 3:** Assessment of levels of human FGF2 protein by protein ELISA from cell culture supernatants of murine 3T3 fibroblast cells 24h, 72h and 120h post transfection with human FGF2 IVT mRNA variants.
In order to assess whether codon optimization for optimal translation efficiency in the human system (as determined by the CAI) and a concomitant increase of GC content in the coding sequence (CDS) of FGF2 mRNA variants led to expression changes in human FGF2 protein, 3T3 fibroblasts were transfected with different FGF2 mRNA variants. mRNAs encoding SEQ ID NOs:18, 19 and 21 were used to transfect 3T3 cells. Subsequently, the level of secretion of human FGF2 from transfected cells was determined for up to 120h post transfection.
Result: All 3 FGF2 mRNA variants are inducing high levels of human FGF2 protein using 1µg mRNA/ml at all time points assessed (Figure 3). Comparative analysis of IVT mRNA encoding for native human FGF2 and the two variants showed an unexpected combined effect of codon optimization (i.e. CAI levels ≥0,76) and an increase of GC content (GC content ≥51,7%) in the CDS of the mRNA. Figure 3 shows 3T3 cells (4*10⁴-5*10⁴/well) transfected using IVT mRNAs complexed with TransIT mRNA transfection reagent (1µg mRNA/ml). Supernatants were obtained and subjected to human FGF2 specific protein ELISA (R and D Systems). Values depicted are measured as pg/ml FGF2 protein.
Those sequences which were above the threshold (CAI≥0.76 and GC content ≥51,7%) induced significantly higher expression levels of FGF2 at early and late stages showing a surprisingly high benefit over native sequences in the amplitude and longevity of gene expression.
Surprisingly, transfection of fibroblasts with the optimized FGF mRNA variants, provided with the present invention, differs in terms of its overall kinetics from what is known in the field about other mRNAs:
Upon delivery of previously described IVT mRNA into a given cell, the internalized IVT mRNA content will continuously drop after an initial peak level, which is usually followed by a delayed drop in protein translation. Surprisingly, and in sharp contrast to the usual situation described above, delivery of the present invention, FGF mRNA variants, into cells is characterized by a dissociation between the mRNA and protein kinetics. Specifically, while the IVT mRNA is gradually degraded upon delivery, protein secretion as shown here (Figures 3-8) begins to rise on the first day (i.e. expression is lowest for the first 24h). It then reaches a plateau on the second day, which is sustained for several days before FGF production wanes. Importantly, this novel pattern was obtained with 3 different cell lines of two origins from three species. Moreover, it is consistent with an effect of IVT mRNA-induced FGF protein on the auto- or paracrine regulation of endogenous FGF production.
The optimized sequences according to the present invention therefore have i.a. a very surprising effect compared to the native sequences and to the recombinant protein:
With the optimized sequences of FGF2 and FGF7, a significant prolongation of the effect can be obtained. The observed activity of the native and the optimized sequences is comparable, but not reaching a plateau yet, in the initial 24h phase after transformation in 3T3 cells (Figure 3). However, this changes significantly in the further course of the experiment. The native sequence is able to increase expression as shown by analysis at 72h, but it does not induce significant activity after 120 h. In contrast, the optimized sequences, as provided with the present invention, induce increased expression in 3T3 cells after 72h and also, surprisingly, produce a 10-fold to almost 100-fold improved effect at 120h post transfection in this example (Figure 3). Moreover, it has to be emphasized that levels detectable after 120h are even higher than after 24h. Similar patterns have also been obtained in BJ cells (e.g.: Figure 5, with a significantly higher level of protein present at 120h as compared to 24h especially for SEQ ID NO:21) or in porcine epithelial sheets, where levels are increasing significantly at 48h and 72h as compared to the native sequence (see Figure 7). Interestingly, this pattern is not achieved upon addition of recombinant FGF protein at similar amounts as expressed at 24h post transfection to the cells/organs. Therefore, no such autoregulation has been observed 24h post addition of recombinant FGF protein suggesting that mRNA-induced expression is able to activate different intra- and extracellular effects compared to protein substitution.
**Example 4:** Assessment of levels of human FGF7 protein by protein ELISA from cell culture supernatants of murine 3T3 fibroblast cells up to 120h post transfection with human FGF7 IVT mRNA variants.
In order to assess whether codon optimization for optimal translation efficiency in the human system (as determined by the CAI) and concomitant increase of GC content in the CDS of FGF7 mRNA variants changes expression of human FGF7 protein, 3T3 fibroblasts were transfected with different FGF7 mRNA variants. mRNAs encoding SEQ ID NOs:1, 2 and 4 were used to transfect 3T3 cells. Subsequently, the level of secretion of human FGF7 from transfected cells was determined for up to 120h post transfection.
Result: All 3 FGF7 mRNA variants induced high levels of human FGF7 protein using 1µg mRNA/well at all time points assessed (Figure 4). Comparative analysis of IVT mRNA encoding for native human FGF4 and the two variants showed an unexpected combined effect of codon optimization (CAI≥0.74) and an increase of GC content (GC content ≥39,5%) in the CDS of the mRNA. Figure 4 shows 3T3 cells (4*10⁴-5*10⁴/well) transfected using IVT mRNAs complexed with TransIT mRNA transfection reagent (1µg mRNA/ml). Supernatants were obtained and subjected to human FGF7 specific protein ELISA (R and D Systems). Values depicted are measured as pg/ml FGF7 protein.
Those sequences which were above the threshold (CAI≥0.74 and GC content ≥39,5%) induced significantly higher expression levels of FGF7 at early and late stages showing a surprisingly high benefit native sequences in the amplitude and longevity of expression.
**Example 5:** Assessment of levels of human FGF2 protein by protein ELISA from cell culture supernatants of human B.J. fibroblast cells up to 120h post transfection with human FGF2 IVT mRNA variants.
In order to assess whether codon optimization for optimal translation efficiency in the human system (as determined by the CAI) and concomitant increase of GC content in the CDS of FGF2 mRNA variants changes expression of human FGF2 protein, B.J. fibroblasts were transfected with different FGF2 mRNA variants. mRNAs encoding SEQ ID NOs:18, 19 and 21 were used to transfect BJ cells. Subsequently, the level of secretion of human FGF2 from transfected cells was determined for up to 120h post transfection.
Result: All 3 FGF2 mRNA variants induced high levels of human FGF2 protein using 1µg mRNA/ml at all time points assessed (Figure 5). Comparative analysis of IVT mRNA encoding for native human FGF2 and the variants showed an unexpected combined effect of codon optimization (i.e. CAI levels ≥0,76) and an increase of GC content (GC content ≥51,7%) in the CDS of the mRNA. Figure. 5 shows BJ cells (4*10⁴-5*10⁴/well) transfected using IVT mRNAs complexed with TransIT mRNA transfection reagent (1µg mRNA/ml). Supernatants (≥n=2/condition) were obtained and subjected to human FGF2 specific protein ELISA (R and D systems). Values depicted are measured as pg/ml FGF2 protein.
Those sequences which were above the threshold of (CAI≥0.76 and GC content ≥51,7%) were inducing significantly higher expression levels of FGF2 at early and late stages showing a surprisingly high benefit over native sequences in the amplitude and longevity of gene expression.
**Example 6:** Assessment of levels of human FGF7 protein by protein ELISA from cell culture supernatants of human BJ fibroblast cells up to 120h post transfection with human FGF7 IVT mRNA variants.
In order to assess whether codon optimization for optimal translation efficiency in the human system (CAI) and concomitant increase of GC content in the CDS of FGF7 mRNA variants changes expression of human FGF7 protein in human skin fibroblasts, B.J. fibroblasts were transfected with different FGF7 mRNA variants. mRNAs encoding SEQ ID NOs:1, 2 and 4 were used to transfect B.J. cells. Subsequently, the level of secretion of human FGF7 from transfected cells was determined for up to 120h post transfection.
Result: All 3 FGF7 mRNA variants are inducing high levels of human FGF7 protein using 1µg mRNA/ml at all time points assessed (Figure 6). Comparative analysis of IVT mRNA encoding for native human FGF7 and the variants showed an unexpected combined effect of codon optimization (i.e. CAI levels ≥0,74) and an increase of GC content (GC content ≥39,5%) in the CDS of the mRNA. Figure 6 shows BJ cells (4*10⁴-5*10⁴/well) transfected using IVT mRNAs complexed with TransIT mRNA transfection reagent (1µg mRNA/ml). Supernatants were obtained and subjected to human FGF2 specific protein ELISA (R and D systems). Values depicted are measured as pg/ml FGF2 protein.
Those sequences which were above the threshold of (CAI≥0.74 and GC content ≥39,5%) were inducing significantly higher expression levels of FGF2 at early and late stages showing a surprisingly high benefit over native sequences in the amplitude and longevity of gene expression.
**Example 7:** Assessment of levels of human FGF2 protein by protein ELISA from cell culture supernatants of porcine epithelial sheets 24h to 72h post transfection with human FGF2 IVT mRNA variants.
In order to assess whether codon optimization for optimal translation efficiency in the human system (as determined by the CAI) and concomitant increase of GC content in the CDS of FGF2 mRNA variants changes expression of human FGF2 protein in porcine skin, epithelial sheets derived from porcine skin were transfected with different FGF2 mRNA variants. mRNAs encoding SEQ ID NOs:18, 19 and 21 were used to transfect epithelial sheets. TransIT alone as well as TransIT complexed to eGFP mRNA were used as controls. Subsequently, the level of secretion of human FGF2 from transfected tissues was determined for up to 72h post transfection.
Result: All 3 FGF2 mRNA variants induced high levels of human FGF2 protein using 1µg mRNA/ml at all time points assessed (Figure 7). Comparative analysis of IVT mRNA encoding for native human FGF2 and the variants showed an unexpected combined effect of codon optimization (i.e. CAI levels ≥0,76) and an increase of GC content (GC content ≥51,7%) in the CDS of the mRNA. Figure 7 shows porcine epithelial sheets transfected using IVT mRNAs complexed with TransIT mRNA transfection reagent (1µg mRNA/ml). Supernatants were obtained and subjected to human FGF2 specific protein ELISA (R and D systems). Values depicted are measured as pg/ml FGF2 protein.
Those sequences which were above the threshold of (CAI≥0.76 and GC content ≥51,7%) were inducing significantly higher expression levels of FGF2 at early and late stages showing a surprisingly high benefit over native sequences in the amplitude and longevity of expression in an intact porcine tissue.
**Example 8:** Assessment of levels of human FGF7 protein by protein ELISA from cell culture supernatants of porcine epithelial sheets 24h to 72h post transfection with human FGF7 IVT mRNA variants.
In order to assess whether codon optimization for optimal translation efficiency in the human system (as determined by the CAI) and concomitant increase of GC content in the CDS of FGF7 mRNA variants changes expression of human FGF7 protein in porcine skin, epithelial sheets derived from porcine skin were transfected with different FGF7 mRNA variants. mRNAs encoding SEQ ID NOs:1, 2 and 4 were used to transfect epithelial sheets. TransIT alone as well as TransIT complexed to eGFP mRNA were used as controls. Subsequently, the level of secretion of human FGF7 from transfected tissues was determined for up to 72h post transfection.
Result: All 3 FGF7 mRNA variants are inducing high levels of human FGF7 protein using 1µg mRNA/well at all time points assessed (Figure 8). Comparative analysis of IVT mRNA encoding for native human FGF7 and the variants showed an unexpected combined effect of codon optimization (i.e. CAI levels ≥0.74) and an increase of GC content (GC content ≥39,5%) in the CDS of the mRNA. Figure 8 shows porcine epithelial sheets transfected using IVT mRNAs complexed with TransIT mRNA transfection reagent (1µg mRNA/ml). Supernatants were obtained and subjected to human FGF7 specific protein ELISA (R and D systems). Values depicted are measured as pg/ml FGF7 protein.
Those sequences which were above the threshold of (CAI≥0.74 and GC content ≥39,5%) were inducing significantly higher expression levels of FGF7 at early and late stages showing a surprisingly high benefit over native sequences in the amplitude and longevity of expression in an intact porcine tissue.
**Example 9:** Detection of eGFP in porcine epithelial sheets 24h after transfection with eGFP IVT mRNA formulated using TransIT mRNA transfection reagent.
In this example eGFP expression was monitored over time as a positive control for transfection efficacy of experiments performed in example 7+8, respectively.
Porcine epithelial sheets were transfected with TransIT and TransIT complexed with eGFP mRNA as described above. In addition, a second formulation of TransIT and eGFP mRNA (0.5µg mRNA/ml) was included as a dosage control. Subsequently, eGFP protein expression in transfected tissue was monitored by direct fluorescence microscopy.
Result: similar levels of eGFP expression were detectable in both examples analysed showing comparability of transfection efficacies in both experiments (Figure 9). Importantly, a dose dependent effect of eGFP expression was detectable in this experiment.
Figure 9 shows eGFP mRNA formulated in TransIT used at different concentrations: 0.5 and 1µg mRNA/ml. Native organ samples were mounted 24h post transfection on Superfrost plus glass slides using Vectashield DAPI-Hard set embedding medium and subjected to direct fluorescence detection using a Zeiss AxioImage Z2 microscope with Apotome 2. Successful transfection was detected by eGFP positive cells in the epithelial sheets as compared to liposome only treated sheets.
**Example 10:** Detection of eGFP in porcine epithelial sheets 24h after transfection with eGFP IVT mRNA formulated using a liposomal transfection reagent.
In this example, eGFP expression induced by an alternative transfection formulation was monitored over time.
Porcine epithelial sheets were transfected with a liposome based formulation using two different eGFP mRNA concentrations (2µg/ml and 10µg/ml mRNA) as described above. Subsequently, eGFP protein expression in transfected tissue was monitored by direct fluorescence microscopy.
Result: eGFP expression was detectable in a dose dependent manner in this experiment (Figure 9 and Table 5). Overall transfection efficacy was comparable to results obtained in examples 7-9, respectively.

**Table 5: eGFP expression in porcine epithelial sheets 24h post transfection**

| Formulation of mRNA | Amount (mRNA/dose) | eGFP expression in epidermis (pg/mg total protein) |
|---|---|---|
| Cationic amphiphilic liposome | 5.4µg/ml | 775 |
| TransIT; lipoplex | 2µg/ml | 7677 |
| Cationic liposome | 2µg/ml | 8363 |
| Cationic liposome | 10µg/ml | 11180 |

Figure 10 shows eGFP mRNA formulated in liposomes used at two concentrations: 2 and 10µg mRNA/ml. 24h post transfection native organ samples were mounted on Superfrost plus glass slides using Vectashield DAPI-Hard set embedding medium and subjected to direct fluorescence detection using a Zeiss AxioImage Z2 microscope with Apotome 2. Successful transfection was detectable by concentration dependent increase in eGFP positive cells in the epithelial sheets as compared to liposome only treated sheets.
**Example 11:** Detection of whole mount β-Galactosidase (bGal) activity in porcine skin explants 24h after transfection with LacZ IVT mRNA formulated using a DOTAP based liposomal transfection reagent.
In order to assess whether mRNA variants are able to transfect mammalian skin in situ, punch biopsies obtained from porcine skin were transfected with different LacZ mRNA formulations. In this example LacZ expression induced by intradermal transfection was monitored by whole mount β-Galactosidase staining 24h post transfection.
Transfection of intact pig skin was done by direct, intradermal injection of the IVT mRNA solution (5µg mRNA/dose; +/-Rnase inhibitor). mRNA was formulated using DOTAP-liposomes. 24h post transfection organ samples were subjected to whole mount β-Galactosidase (bGal) staining. Successful transfection was detectable by BluoGal staining in situ. Subsequently, punch biopsies of the injected areas (8mm diameter) were taken, subcutaneous fat removed, and biopsies were cultured for 24h.
Result: LacZ expression was visualized by detection of bGal activity in transfected biopsies (Figure 11). bGal activity was comparable for different formulations of LacZ mRNA (+/- RNAse inhibitor) and expression was detectable as seen by blue staining in the upper dermal compartment of transfected biopsies.
**Example 12:** Detection of eGFP expression in porcine skin explants 24h after transfection with eGFP IVT mRNA formulated using various transfection reagents and non-complexed RNAs.
In order to assess whether mRNA variants are able to transfect mammalian skin in situ, punch biopsies obtained from porcine skin were transfected with different eGFP mRNA formulations. In this example eGFP expression induced by intradermal transfection was monitored by eGFP protein ELISA from tissue extracts obtained 24h post transfection. Along these lines, different formulations of eGFP mRNA were produced and injected (see Table 6 for details).

**Table 6: eGFP expression in porcine skin biopsies 24h post i.d. transfection**

| Formulation of mRNA | Amount (mRNA/dose) | eGFP expression in dermis |
|---|---|---|
| DOTAP based; liposomal | 1-10µg | + |
| SAINT lipid based; liposomal | 1-5µg | + |
| TransIT; lipoplex | 1-10µg | + |
| Lipid-Nano-particle | 1.2+2.4µg | + |
| Non-complexed | 1-25µg | + |

Formulations used included: eGFP mRNA complexed with TransIT mRNA transfection reagent, eGFP mRNA complexed with DOTAP based liposomal preparations, eGFP mRNA complexed with SAINT lipid based liposomal preparations, eGFP mRNA containing Lipid nano-particles and as non-complexed eGFP mRNA formulated in physiologic buffer.
Transfection of intact pig skin was performed by direct, intradermal injection of the IVT mRNA solutions (the eGFP IVT mRNA (1-25µg mRNA/dose)). mRNA was formulated using TransIT mRNA transfection reagent, DOTAP based-liposomes, SAINT lipid based-liposomes, lipid nano-particles or non-complexed mRNA in physiologic buffer. Subsequently, punch biopsies of the injected areas (8mm diameter) were taken, subcutaneous fat removed, biopsies were cultured for 24h and analyzed for eGFP expression. 24h post transfection organ samples were subjected to protein extraction and subsequent eGFP protein ELISA.
Result: eGFP expression was detectable by eGFP protein ELISA 24h post injection. (Figure 12, Table 5). eGFP Lipoplexes (LNPs and liposomal complexes) as well as TransIT (used as standard) showed similar concentration dependent eGFP induction. Optimal expression was detectable between 2.4µg and 5µg mRNA/dose. Non-complexed mRNA also showed successful transfection. However, the minimal concentration required in this experimental setting was 5µg mRNA/dose in order to induce detectable eGFP expression in porcine dermis showing less efficient transfection of mRNA in the absence of transfection reagents.
**Example 13:** Detection of Luciferase activity in porcine epithelial sheets 24h after transfection with FLuc IVT mRNA formulated using Trans IT and SAINT based liposomal transfection reagents.
In this example, FLuc expression induced by alternative transfection formulations was monitored over time.
Porcine epithelial sheets were transfected with Trans IT or a liposome based formulation using 2µg/ml mRNA as described above. Subsequently, Luciferase activity in transfected tissue was monitored by direct activity measurement.
Result: In this experiment, Luciferase activity was detectable in a lipid dose dependent manner (Figure 13 and Table 7). Overall transfection efficacy was comparable to results obtained in examples 7-10, respectively.

**Table 7: Luciferase activity in porcine epithelial sheets 24h post transfection**

| Formulation of mRNA | Amount (mRNA/dose) | Luciferase activity (activity units) |
|---|---|---|
| Trans IT | 0µg/ml | 190 |
| Saint based liposome 1 (low lipid formulation) | 0µg/ml | 174 |
| Saint based liposome 2 (low lipid formulation) | 0µg/ml | 190 |
| TransIT; lipoplex | 2µg/ml | 909047 |
| Saint based liposome 1 (high lipid formulation) | 2µg/ml | 433383 |
| Saint based liposome 1 (low lipid formulation) | 2µg/ml | 110871 |
| Saint based liposome 2 (high lipid formulation) | 2µg/ml | 1018908 |
| Saint based liposome 2 (low lipid formulation) | 2µg/ml | 910750 |

Figure 13 shows FLuc mRNA formulated in liposomes used at two lipid concentrations. 24h post transfection native organ samples were subjected to Firefly Luc One-Step Glow Assay Kit. Successful transfection was detectable by activity units (luminescence) and direct luminescence detection on a Gel-Doc system.
**Example 14:** Detection of Luciferase activity in porcine epithelial sheets and porcine dermal explants 24h after transfection with FLuc IVT mRNA formulated using Trans IT and SAINT based liposomal transfection reagents.
In this example, Firefly Luciferase expression induced by alternative transfection formulations was monitored over time.
Porcine epithelial sheets and dermal explants were transfected with Trans IT or a liposome based formulation using 2µg/ml mRNA as described above. Subsequently, Luciferase activity in transfected tissue was monitored by direct activity measurement.
Result: Luciferase activity was detectable in both, epithelial tissue as well as dermal tissue following transfection (Figure 14). Overall transfection efficacy was comparable to results obtained in examples 7-10, respectively.
Figure 14 shows FLuc mRNA formulated in liposomes used at two lipid concentrations. 24h post transfection native organ samples (i.e. epithelial sheets and dermal explants) were subjected to Firefly Luc One-Step Glow Assay Kit. Successful transfection was detectable by activity units (luminescence).
**Example 15:** Comparison of Seq ID NO:1 mRNAs to the mRNA variant containing 100% replacement of Pseudo-U for U and 5mC for C by assessment of levels of human FGF7 protein by protein ELISA from cell culture supernatants of murine 3T3 cells 24h to 120h post transfection.
In this example the effect of non-canonical/modified nucleotides (e.g.: pseudoU and m5C) on expression levels of FGF7 mRNA variants is assessed. Murine 3T3 cells were transfected with two forms of native (SEQ ID NO:1) IVT mRNA (mRNA: 1µg/ml): one containing 100% replacement of Pseudo-U for U and 5mC for C (indicated as Seq ID1_mn) and one w/o modified nucleotides (Seq ID1). Again, TransIT alone was used as control. Subsequently, the level of secretion of human FGF7 from transfected tissue is determined for up to 120h post transfection.
Result: FGF7 expression is visualized by detection of secreted FGF7 in cell supernatants. As shown in Figure 15, both mRNA variants induce high level expression of FGF7 protein at all time points tested.
Those sequences which were devoid of modified nucleotides are inducing significantly higher expression levels of FGF7 at early and late stages showing a surprisingly high benefit over modified nucleotides in wt, native sequences in the amplitude and longevity of expression in murine cells and an intact porcine tissue.
**Example 16:** Analysis of Hyaluronic acid levels following treatment of murine 3T3 fibroblast cells with optimized FGF2 mRNA variants up to 120h post transfection
In order to assess whether FGF2 mRNA variants were able to modify hyaluronic acid (=hyaluronan) secretion in murine 3T3 fibroblasts over extended time periods, fibroblasts were transfected with different FGF2 mRNA variants and hyaluronic acid secretion was assessed for up to 120h post transfection. mRNAs encoding SEQ ID NOs:18, 19 and 21 were used to transfect murine 3T3 cells. Again, TransIT alone was used as control. Subsequently, the level of secreted hyaluronan from the supernatant of transfected cells was determined for up to 120h post transfection using the Hyaluronan Quantikine ELISA Kit.
Result: All 3 FGF2 mRNA variants are inducing secretion of high levels of hyaluronan using 1µg mRNA/well at all time points assessed (Figure 16) showing bioactivity of the secreted FGF2 protein in this system and a high benefit in the amplitude of IVT mRNA induced hyaluronan synthesis in murine cells. Values depicted are measured as ng/ml hyaluronan.
**Example 17:** Analysis of Hyaluronic acid levels following treatment of murine 3T3 cells, porcine epithelial sheets and porcine fibroblast cells with optimized FGF mRNA variants up to 120h post transfection
In order to assess whether FGF mRNA variants are able to modify hyaluronan secretion in murine cells, porcine epithelial sheets and primary porcine fibroblasts over extended time periods, sheets and fibroblasts are transfected with different FGF mRNA variants and hyaluronic acid secretion is assessed for up to 72h post transfection.
mRNAs encoding SEQ ID NOs:1, 2, 3, 4, 5, 18, 19, 20, 21 and 22, are used to transfect sheets and fibroblasts cells (1µg/ml). Subsequently, the level of secreted hyaluronic acid (i.e. newly synthesized) from the supernatant of transfected cells is determined for up to 120h post transfection using the Hyaluronan Quantikine ELISA Kit.
Expected result: IVT mRNA induced Hyaluronan synthesis is visualized by detection of hyaluronan in supernatants. All FGF mRNA variants are inducing secretion of high levels of hyaluronic acid using 1µg mRNA/well at all time points assessed showing bioactivity of the secreted FGF protein in this system.
**Example 18:** Assessment of levels of human FGF7 protein by protein ELISA from cell culture supernatants of porcine epithelial sheets following transfection with human FGF7 IVT mRNA variants.
In this example, the effect of differential codon optimization for GC contents lower than the threshold (39,5%) and lower CAI levels (i.e. CAI<0.74) in the coding sequence (CDS) of FGF7 mRNA variants is assessed and compared to different IVT mRNA variants. Porcine epithelial sheets are transfected with native or optimized (SEQ ID NO:1 and SEQ ID NO:3), as well as variants displaying CAIs<0.74 and/or GC contents <39,5% (e.g.: SEQ ID NO:5) as described above. Again, TransIT alone as well as TransIT complexed to eGFP mRNA are used as controls. Subsequently, the level of secretion of human FGF7 from transfected tissue is determined for up to 72h post transfection.
Expected result: FGF7 expression is visualized by detection of secreted FGF7 in cell supernatants.
Those sequences which are above the threshold of (CAI≥0.74 and GC content ≥39,5%) are inducing significantly higher expression levels of FGF7 at early and late stages showing a surprisingly high benefit over wt, native sequences in the amplitude and longevity of expression in an intact porcine tissue whereas sequences which underwent optimization but are below the threshold of (CAI≥0.74 and GC content ≥39,5%) are less efficient in inducing FGF7 in porcine tissue.
**Example 19:** Assessment of levels of human FGF7 protein by protein ELISA from cell culture supernatants of murine 3T3 fibroblasts following transfection with human FGF7 IVT mRNA variants.
In this example, the effect of differential codon optimization for GC contents lower than the threshold (39,5%) and lower CAI levels (i.e. CAI<0.74) in the coding sequence (CDS) of FGF7 mRNA variants is assessed and compared to different IVT mRNA variants. 3T3 fibroblasts are transfected with native or optimized (SEQ ID NO:1 and SEQ ID NO:3), as well as variants displaying CAIs<0.74 and/or GC contents <39,5% (e.g.: SEQ ID NO:5) as described above. Again, TransIT alone is used as control. Subsequently, the level of secretion of human FGF7 from transfected cells is determined for up to 120h post transfection.
Expected result: FGF7 expression is visualized by detection of secreted FGF7 in cell supernatants.
Those sequences which are above the threshold of (CAI≥0.74 and GC content ≥39,5%) are inducing significantly higher expression levels of FGF7 at early and late stages showing a surprisingly high benefit over wt, native sequences in the amplitude and longevity of expression in murine cells whereas sequences which underwent optimization but were below the threshold of (CAI≥0.74 and GC content ≥39,5%) are less efficient in inducing FGF7 in cells.
**Example 20:** Assessment of levels of human FGF7 protein by protein ELISA from cell culture supernatants of human skin fibroblasts following transfection with human FGF7 IVT mRNA variants.
In this example, the effect of differential codon optimization for GC contents lower than the threshold (39,5%) and lower CAI levels (i.e. CAI<0.74) in the coding sequence (CDS) of FGF7 mRNA variants is assessed and compared to different IVT mRNA variants. Human skin fibroblasts are transfected with native or optimized (SEQ ID NO:1 and SEQ ID NO:3), as well as variants displaying CAIs<0.74 and/or GC contents <39,5% (e.g.: SEQ ID NO:5) as described above. Again, TransIT alone is used as control. Subsequently, the level of secretion of human FGF7 from transfected cells is determined for up to 120h post transfection.
Expected result: FGF7 expression is visualized by detection of secreted FGF7 in cell supernatants.
Those sequences which are above the threshold of (CAI≥0.74 and GC content ≥39,5%) are inducing significantly higher expression levels of FGF7 at early and late stages showing a surprisingly high benefit over wt, native sequences in the amplitude and longevity of expression in human cells.
Those sequences which are above the threshold of (CAI≥0.74 and GC content ≥39,5%) are inducing significantly higher expression levels of FGF7 at early and late stages showing a surprisingly high benefit over wt, native sequences in the amplitude and longevity of expression in human cells whereas sequences which underwent optimization but are below the threshold of (CAI≥0.74 and GC content ≥39,5%) are less efficient in inducing FGF7 in human cells.
**Example 21:** Assessment of levels of human FGF2 protein by protein ELISA from cell culture supernatants of porcine epithelial sheets following transfection with human FGF2 IVT mRNA variants.
In this example, the effect of differential codon optimization for GC contents lower than the threshold (51,7%) and lower CAI levels (i.e. CAI<0.76) in the coding sequence (CDS) of FGF2 mRNA variants is assessed and compared to different IVT mRNA variants. Porcine epithelial sheets are transfected with native or optimized (SEQ ID NO:18 and SEQ ID NO:20), as well as variants displaying CAIs<0,76 and/or GC contents <51,7% (e.g.: SEQ ID NO:22) as described above. Again, TransIT alone as well as TransIT complexed to eGFP mRNA are used as controls. Subsequently, the level of secretion of human FGF2 from transfected tissue is determined for up to 72h post transfection.
Expected result: FGF2 expression is visualized by detection of secreted FGF2 in cell supernatants.
Those sequences which are above the threshold of (CAI≥0.76 and GC content ≥51,7%) are inducing significantly higher expression levels of FGF2 at early and late stages showing a surprisingly high benefit over wt, native sequences in the amplitude and longevity of expression in an intact porcine tissue whereas sequences which underwent optimization but are below the threshold of (CAI≥0.76 and GC content ≥51,7%) are less efficient in inducing FGF2 in porcine tissue.
**Example 22:** Assessment of levels of human FGF2 protein by protein ELISA from cell culture supernatants of murine 3T3 fibroblasts following transfection with human FGF2 IVT mRNA variants.
In this example, the effect of differential codon optimization for GC contents lower than the threshold (51,7%) and lower CAI levels (i.e. CAI<0.76) in the coding sequence (CDS) of FGF2 mRNA variants is assessed and compared to different IVT mRNA variants. 3T3 fibroblasts were transfected with native or optimized (SEQ ID NO:18 and SEQ ID NO:20), as well as variants displaying CAIs<0.76 and/or GC contents <51,7% (e.g.: SEQ ID NO:22) as described above. Again, TransIT alone is used as control. Subsequently, the level of secretion of human FGF2 from transfected cells is determined for up to 120h post transfection.
Expected result: FGF2 expression is visualized by detection of secreted FGF2 in cell supernatants.
Those sequences which are above the threshold of (CAI≥0.76 and GC content ≥51,7%) are inducing significantly higher expression levels of FGF2 at early and late stages showing a surprisingly high benefit over wt, native sequences in the amplitude and longevity of expression in murine cells whereas sequences which underwent optimization but are below the threshold of (CAI≥0.76 and GC content ≥51,7%) are less efficient in inducing FGF2 in cells.
**Example 23:** Assessment of levels of human FGF2 protein by protein ELISA from cell culture supernatants of human skin fibroblasts following transfection with human FGF2 IVT mRNA variants.
In this example, the effect of differential codon optimization for GC contents lower than the threshold (51,7%) and lower CAI levels (i.e. CAI<0.76) in the coding sequence (CDS) of FGF2 mRNA variants is assessed and compared to different IVT mRNA variants. Human skin fibroblasts are transfected with native or optimized (SEQ ID NO:18 and SEQ ID NO:20), as well as variants displaying CAIs<0.76 and/or GC contents <51,7% (e.g.: SEQ ID NO:22) as described above. Again, TransIT alone is used as control. Subsequently, the level of secretion of human FGF2 from transfected cells is determined for up to 120h post transfection.
Expected result: FGF2 expression is visualized by detection of secreted FGF2 in cell supernatants.
Those sequences which are above the threshold of (CAI≥0.76 and GC content ≥51,7%) are inducing significantly higher expression levels of FGF2 at early and late stages showing a surprisingly high benefit over wt, native sequences in the amplitude and longevity of expression in human cells.
Those sequences which are above the threshold of (CAI≥0.76 and GC content ≥51,7%) are inducing significantly higher expression levels of FGF2 at early and late stages showing a surprisingly high benefit over wt, native sequences in the amplitude and longevity of expression in human cells whereas sequences which underwent optimization but are below the threshold of (CAI≥0,76 and GC content ≥51,7%) are less efficient in inducing FGF2 in human cells.
**Example 24:** Detection of mRNA encoding different FGF2 and FGF7 variants by FGF-variant specific quantitative Real Time-PCR from cDNA obtained from murine 3T3 fibroblast cells 24h-120h post transfection.
In order to assess whether FGF2 and FGF7 mRNA variants are stable in murine fibroblasts over prolonged time periods, different mRNAs encoding SEQ ID NOs:1-5 and 18-22 are used to transfect 3T3 cells. RNA is isolated at different time points (24-120h post transfection) and the presence of mRNA in transfected cells determined up to 120h post transfection by quantitative RT PCR.
Expected result: 3T3 cells transfected without mRNA, or with 1µg mRNA complexed with TransIT mRNA transfection reagent. Total cellular RNAs are isolated at different time points after transfection (24h-120h) and mRNAs are reverse transcribed into cDNA by conventional RT-PCR. cDNA samples are then subjected to variant specific quantitative real time PCR using primers for SEQ ID NOs:1-5 and 18-22 for the detection of transfected FGF2 and FGF7 mRNAs and murine ACTB as a PCR control. It follows that there is differential stability of IVT mRNAs in cells according to CAI and G+C content.
**Example 25:** Detection of mRNA encoding different FGF2 and FGF7 variants by FGF-variant specific quantitative Real Time-PCR from cDNA obtained from human BJ fibroblast cells 24h-120h post transfection.
In order to assess whether FGF2 and FGF7 mRNA variants are stable in human skin fibroblasts over prolonged time periods, different mRNAs encoding SEQ ID NOs:1-5 and 18-22 are used to transfect 3T3 cells. RNA is isolated at different time points (24-120h post transfection) and the presence of mRNA in transfected cells determined up to 120h post transfection by quantitative RT PCR.
Expected result: BJ cells transfected without mRNA, or with 1µg mRNA complexed with TransIT mRNA transfection reagent. Total cellular RNAs are isolated at different time points after transfection (24h-120h) and mRNAs are reverse transcribed into cDNA by conventional RT-PCR. cDNA samples are then subjected to variant specific quantitative real time PCR using primers for SEQ ID NOs:1-5 and 18-22 for the detection of transfected FGF2 and FGF7 mRNAs and human RPL4 as a PCR control. It follows that there is differential stability of IVT mRNAs in cells according to CAI and G+C content.
**Example 26:** Analysis of stability and activity of recombinant FGF proteins in comparison to IVT mRNA variant induced protein by ELISA.

In order to assess whether recombinant FGF2 or FGF7 protein are able to regulate FGF expression and subsequent activity in a similar manner as IVT mRNAs, murine 3T3 or porcine primary fibroblasts cells were either incubated in the presence of 1ng/ml recombinant FGF2 or FGF7 protein or were transfected with different FGF2 and FGF7 mRNA variants. mRNAs encoding SEQ ID NOs:1 and 2 and 18, 19 and 21 were used to transfect murine 3T3 cells and porcine primary fibroblast cells. Subsequently, the level of FGF2 and FGF7 protein was determined for 24h post transfection/addition of protein.

Result: FGF2 and FGF7 expression is visualized by detection of secreted FGF2 and FGF7 in cell supernatants by protein ELISA. As shown in Figure 17, both, recombinant FGF2 as well as recombinant FGF7 protein, are unstable over 24h in this experiment. In neither of the individual experiments performed, remaining FGF2 or FGF7 protein could be detected in the tissue culture supernatant of murine 3T3 - or porcine primary fibroblasts. In contrast IVT mRNA variants induced high FGF protein levels at the time of analysis in all experiments. In addition, this lack of detection also shows that no FGF2 or FGF7 protein was newly synthesized following recombinant FGF2/7 exposure. Importantly, this was also supported by the concomitant testing of detectability of porcine and murine FGFs by the human ELISA system: the ELISA systems employed were also able to detect murine and porcine FGFs. Thus, a lack of FGF signals in the ELISA shown is demonstrating that no feedback loop was initiated by the rec. proteins in these experiments in murine or porcine cells.

It follows that IVT mRNA variants as presented in this invention are surprisingly able to regulate FGF expression and activity/function in vertebrate cells differentially than recombinant FGF protein.

Accordingly, the present invention relates to the following preferred embodiments:
1. Fibroblast growth factor (FGF) messenger-RNA (mRNA), wherein the mRNA has a 5' CAP region, a 5' untranslated region (5'-UTR), a coding region encoding human FGF2 or FGF7, a 3' untranslated region (3'-UTR) and a poly-adenosine Tail (poly-A tail), for use in the treatment of local skin hypotrophy conditions,
   wherein the coding region of the FGF mRNA encodes for human fibroblast growth factor 2 (FGF2) or
   wherein the coding region of the FGF mRNA encodes for human fibroblast growth factor 7 (FGF7).
2. FGF mRNA for use according to embodiment 1, wherein the local skin hypotrophy condition is selected from the group consisting of cutis laxa, acrodermatitis chronica atrophicans, atrophodermia idiopathica et progressiva Pasini Pierini, scars resulting from perforating dermatoses, atrophy blanche, necrobiosis lipoidica, radiation dermatitis, striae cutis distensae, atrophic skin conditions, glucocorticoid (GC)-induced skin atrophy, atrophic scars, and skin ulcer.
3. FGF mRNA for use according to any one of embodiments 1 or 2, wherein the poly-A tail comprises at least 60 adenosine monophosphates, preferably at least 100 adenosine monophosphates, especially at least 120 adenosine monophosphates.
4. FGF mRNA for use according to any one of embodiments 1 to 3, wherein the 5'-UTR or 3'-UTR or the 5'-UTR and the 3'-UTR are different from the native FGF2 or FGF7 mRNA, preferably wherein the 5'-UTR or 3'-UTR or the 5'-UTR and the 3'-UTR contain at least one stabilisation sequence, preferably a stabilisation sequence with the general formula (C/U)CCANₓCCC(U/A)PyₓUC(C/U)CC (SEQ ID NO:38), wherein "x" is, independently in Nₓ and Pyₓ, an integer of 0 to 10, preferably of 0 to 5, especially 0, 1, 2, 4 and/or 5).
5. FGF mRNA for use according to any one of embodiments 1 to 4, wherein the 5'-UTR or 3'-UTR or the 5'-UTR and the 3'-UTR are different from the native FGF2 or FGF7 mRNA, and contain at least one destabilisation sequence element (DSE), preferably AU-rich elements (AREs) and/or U-rich elements (UREs), especially a single, tandem or multiple or overlapping copies of the nonamer UUAUUUA(U/A)(U/A).
6. FGF mRNA for use according to any one of embodiments 1 to 5, wherein the 5'-UTR or 3'-UTR or the 5'-UTR and the 3'-UTR are different from the native FGF2 or FGF7 mRNA, and wherein the 5'-UTR and/or 3'-UTR are the 5'-UTR and/or 3'-UTR of a different human mRNA than FGF2 or FGF7, preferably selected from alpha Globin, beta Globin, Albumin, Lipoxygenase, ALOX15, alpha(1) Collagen, Tyrosine Hydroxylase, ribosomal protein 32L, eukaryotic elongation factor 1a (EEF1A1), 5'-UTR element present in orthopoxvirus, and mixtures thereof, especially selected from alpha Globin, beta Globin, alpha(1) Collagen, and mixtures thereof.
7. FGF mRNA for use according to any one of embodiments 1 to 6, wherein in the FGF2 or FGF7 mRNA, at least 5%, preferably at least 10%, preferably at least 30%, especially at least 50% of all
   - cytidine residues are replaced by 5-methyl-cytidine residues, and/or
   - cytidine residues are replaced by 2-amino-2-deoxy-cytidine residues, and/or
   - cytidine residues are replaced by 2-fluoro-2-deoxy-cytidine residues, and/or
   - cytidine residues are replaced by 2-thio-cytidine residues, and/or
   - cytidine residues are replaced by 5-iodo-cytidine residues, and/or
   - uridine residues are replaced by pseudo-uridine residues, and/or
   - uridine residues are replaced by 1-methyl-pseudo-uridine residues, and/or
   - uridine residues are replaced by 2-thio-uridine residues, and/or
   - uridine residues are replaced by 5-methyl-uridine residues, and/or
   - adenosine residues are replaced by N6-methyl-adenosine residues.
8. FGF mRNA for use according to any one of embodiments 1 to 7, wherein in the FGF2 or FGF7 mRNA, at least 5%, preferably at least 10%, preferably at least 30%, especially at least 50% of all
   - cytidine residues are replaced by 5-methyl-cytidine residues, and/or
   - uridine residues are replaced by pseudo-uridine residues, and/or
   - uridine residues are replaced by 2-thio-uridine residues.
9. FGF mRNA for use according to any one of embodiments 1 to 8, wherein the FGF mRNA has a GC to AU ratio of at least 51.7%, preferably of at least 52%, more preferred 55%, even more preferred, at least 58%, especially at least 60% in case of the FGF2 mRNA and at least 39,5%, preferably of at least 43%, more preferred 45%, even more preferred, at least 50%, especially at least 55% in case of the FGF7 mRNA.
10. FGF mRNA for use according to any one of embodiments 1 to 9, wherein the FGF2 mRNA has a codon adaption index (CAI) of at least 0.77, preferably at least 0.8 and/or the FGF7 mRNA has a CAI of at least 0.75, preferably at least 0.77.
11. FGF mRNA for use according to any one of embodiments 1 to 10, wherein the coding region of the FGF mRNA encodes human FGF2 and is preferably SEQ ID NO:30, especially SEQ ID NO:31.
12. FGF mRNA for use according to any one of embodiments 1 to 10, wherein the coding region of the FGF mRNA encodes human FGF7 and is preferably SEQ ID NO:12, especially SEQ ID NOs:13.
13. FGF mRNA for use according to any one of embodiments 1 to 12, wherein the FGF mRNA is administered subcutaneously, intradermally, transdermally, epidermally, or topically, especially epidermally.
14. FGF mRNA for use according to any one of embodiments 1 to 13, wherein the FGF mRNA is administered at least twice within one month, preferably weekly.
15. FGF mRNA for use according to any one of embodiments 1 to 14, wherein the FGF mRNA is administered in an amount of 0.01µg to 100 mg per dose, preferably of 0.1µg to 10mg per dose, especially of 1µg to 1mg per dose.
16. Pharmaceutical formulation, preferably for use in the treatment of local skin hypotrophy conditions, preferably atrophic scars and glucocorticoid (GC)-induced skin atrophy, comprising the FGF mRNA as defined in any one of embodiments 1 to 15.
17. Pharmaceutical formulation, preferably for use according to embodiment 16, comprising a pharmaceutically acceptable carrier, preferably polymer based carriers, especially cationic polymers, including linear and branched PEI and viromers; lipid nanoparticles and liposomes, nanoliposomes, ceramide-containing nanoliposomes, proteoliposomes, cationic amphiphilic lipids e.g.: SAINT-Lipids, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, dry powders, poly(D-arginine), nanodendrimers, starch-based delivery systems, micelles, emulsions, sol-gels, niosomes, plasmids, viruses, calcium phosphate nucleotides, aptamers, peptides, peptide conjugates, vectorial tags, polylactic-co-glycolic acid (PLGA) polymers; preferably small-molecule targeted conjugates, or viral capsid proteins, preferably cationic polymers and liposomes, especially cationic polymers.
18. Pharmaceutical formulation for use according to embodiment 16 or 17, comprising cationic polymers including linear and branched PEI and viromers, lipid nanoparticles and liposomes, transfersomes, and nanoparticulates, preferably calcium phosphate nanoparticulates and cationic polymers, especially cationic polymers.
19. Pharmaceutical formulation for use according to embodiment 16, wherein the mRNA is non-complexed mRNA, and wherein preferably the non-complexed mRNA is contained in a suitable aqueous buffer solution, especially a physiological glucose buffered aqueous solution.
20. Pharmaceutical formulation for use according to any one of embodiments 16 to 19, wherein the formulation comprises a 1xHEPES buffered solution; a 1xPhosphate buffered solution, a Na-Citrate buffered solution; a Na-Acetate buffered solution; Ringer's lactate solution; preferably additionally comprising glucose, especially 5% Glucose.
21. Pharmaceutical formulation for use according to any one of embodiments 16 to 20, wherein the formulation comprises FGF2 and FGF7 mRNAs as defined in any one of embodiments 1 to 15.
22. Kit for administering the FGF mRNA for use according to any one of embodiments 1 to 15 to a patient comprising
   - the FGF2 and/or FGF7 mRNA as defined in any one of embodiments 1 to 15, and
   - a skin delivery device.
23. Kit according to embodiment 22, wherein the skin delivery device is an intradermal delivery device, preferably selected from the group consisting of needle based injection systems.
24. Kit according to embodiment 22, wherein the skin delivery device is a transdermal delivery device, preferably selected from the group consisting of transdermal patches, hollow and solid microneedle systems, microstructured transdermal systems, electrophoresis systems, and iontophoresis systems.
25. Kit according to embodiment 22, wherein the skin delivery device is an epidermal delivery device, preferably selected from the group consisting of needle free injection systems, laser based systems, especially Erbium YAG laser systems, and gene gun systems.
26. Method for treating local skin hypotrophy conditions, preferably atrophic scars and glucocorticoid (GC)-induced skin atrophy, wherein the FGF2 mRNA and/or FGF7 mRNA as defined in any one of embodiments 1 to 15 is administered in an effective amount to a patient in need thereof.
27. Cosmetic skin care method wherein a FGF messenger-RNA (mRNA) is contacted with the human skin, wherein the mRNA has a 5' CAP region, a 5' untranslated region (5'-UTR), a coding region encoding human FGF, a 3' untranslated region (3'-UTR) and a poly-adenosine Tail (poly-A tail), wherein the coding region of the FGF mRNA encodes for human fibroblast growth factor 2 (FGF2) or wherein the coding region of the FGF mRNA encodes for human fibroblast growth factor 7 (FGF7).
28. Cosmetic skin care method according to embodiment 27, wherein the skin is an ageing skin.
29. Cosmetic skin care method according to embodiment 27 or 28, wherein the FGF mRNA molecule is defined as in any one of embodiments 3 to 11.
30. Cosmetic formulation comprising an FGF mRNA, wherein the mRNA has a 5' CAP region, a 5' untranslated region (5'-UTR), a coding region encoding human FGF, a 3' untranslated region (3'-UTR) and a poly-adenosine Tail (poly-A tail), wherein the coding region of the FGF mRNA encodes for human fibroblast growth factor 2 (FGF2) or wherein the coding region of the FGF mRNA encodes for human fibroblast growth factor 7 (FGF7), and a cosmetic carrier.
31. Cosmetic formulation according to embodiment 30, wherein the FGF mRNA molecule is defined as in any one of embodiments 3 to 11.
32. Cosmetic formulation according to embodiment 30 or 31, wherein the cosmetic carrier is an ointment, a gel, especially a hydrogel, a liposome, nano/microparticles, or an emulsion.

## Claims

1. Fibroblast growth factor (FGF) messenger-RNA (mRNA), wherein the mRNA has a 5' CAP region, a 5' untranslated region (5'-UTR), a coding region encoding human FGF, a 3' untranslated region (3'-UTR) and a poly-adenosine Tail (poly-A tail), for use in the treatment of local skin hypotrophy conditions,
wherein the coding region of the FGF mRNA encodes for human fibroblast growth factor 2 (FGF2) or
wherein the coding region of the FGF mRNA encodes for human fibroblast growth factor 7 (FGF7).

2. FGF mRNA for use according to claims 1, wherein the local skin hypotrophy condition is selected from the group consisting of cutis laxa, acrodermatitis chronica atrophicans, atrophodermia idiopathica et progressiva Pasini Pierini, scars resulting from perforating dermatoses, atrophy blanche, necrobiosis lipoidica, radiation dermatitis, striae cutis distensae, atrophic skin conditions, glucocorticoid (GC)-induced skin atrophy, atrophic scars and skin ulcer.

3. FGF mRNA for use according to claim 1 or 2, wherein the poly-A tail comprises at least 60 adenosine monophosphates, preferably at least 100 adenosine monophosphates, especially at least 120 adenosine monophosphates.

4. FGF mRNA for use according to any one of claims 1 to 3, wherein the 5'-UTR or 3'-UTR or the 5'-UTR and the 3'-UTR are different from the native FGF2 or FGF7 mRNA, preferably wherein the 5'-UTR or 3'-UTR or the 5'-UTR and the 3'-UTR contain at least one stabilisation sequence, preferably a stabilisation sequence with the general formula (C/U)CCANₓCCC(U/A)PyₓUC(C/U)CC (SEQ ID NO:38), wherein "x" is, independently in Nₓ and Pyₓ, an integer of 0 to 10, preferably of 0 to 5, especially 0, 1, 2, 4 and/or 5.

5. FGF mRNA for use according to any one of claims 1 to 4, wherein the 5'-UTR or 3'-UTR or the 5'-UTR and the 3'-UTR are different from the native FGF2 or FGF7 mRNA, and wherein the 5'-UTR and/or 3'-UTR are the 5'-UTR and/or 3'-UTR of a different human mRNA than FGF, preferably selected from alpha Globin, beta Globin, Albumin, Lipoxygenase, ALOX15, alpha(1) Collagen, Tyrosine Hydroxylase, ribosomal protein 32L, eukaryotic elongation factor 1a (EEF1A1), 5'-UTR element present in orthopoxvirus, and mixtures thereof, especially selected from alpha Globin, beta Globin, alpha(1) Collagen, and mixtures thereof.

6. FGF mRNA for use according to any one of claims 1 to 5, wherein the FGF mRNA has a GC to AU ratio of at least 51.7%, preferably of at least 52%, more preferred 55%, even more preferred, at least 58%, especially at least 60% in case of the FGF2 mRNA and at least 39,5%, preferably of at least 43%, more preferred 45%, even more preferred, at least 50%, especially at least 55% in case of the FGF7 mRNA.

7. FGF mRNA for use according to any one of claims 1 to 6, wherein the FGF2 mRNA has a codon adaption index (CAI) of at least 0.77, preferably at least 0.8 and/or the FGF7 mRNA has a CAI of at least 0.75, preferably at least 0.77.

8. FGF mRNA for use according to any one of claims 1 to 7, wherein the FGF2-encoding mRNA is preferably SEQ ID NO:30, especially SEQ ID NOs:31; and/or
wherein the FGF7-encoding mRNA is preferably SEQ ID NO:12, especially SEQ ID NOs:13.

9. FGF mRNA for use according to any one of claims 1 to 8, wherein the FGF mRNA is administered subcutaneously, intradermally, transdermally, epidermally, or topically, especially epidermally.

10. Pharmaceutical formulation, especially for use in the treatment of local skin hypotrophy conditions, preferably atrophic scars and glucocorticoid (GC)-induced skin atrophy, comprising the FGF2 mRNA and/or FGF7 mRNA as defined in any one of claims 1 to 9.

11. Pharmaceutical formulation for use according to claim 10, comprising a pharmaceutically acceptable carrier, preferably polymer based carriers, especially cationic polymers, including linear and branched PEI and viromers; lipid nanoparticles and liposomes, nanoliposomes, ceramide-containing nanoliposomes, proteoliposomes, cationic amphiphilic lipids e.g.: SAINT-Lipids, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, dry powders, poly(D-arginine), nanodendrimers, starch-based delivery systems, micelles, emulsions, sol-gels, niosomes, plasmids, viruses, calcium phosphate nucleotides, aptamers, peptides, peptide conjugates, vectorial tags, preferably small-molecule targeted conjugates, or viral capsid proteins, poly-lactic-co-glycolic acid (PLGA) polymers; preferably cationic polymers and liposomes, especially cationic polymers.

12. Kit for administering the FGF mRNAs for use according to any one of claims 1 to 9 to a patient comprising
- the FGF2 and/or FGF7 mRNA as defined in any one of claims 1 to 9, and
- a skin delivery device.

13. Kit according to claim 12, wherein the skin delivery device is
- an intradermal delivery device, preferably selected from the group consisting of needle based injection systems, or
- a transdermal delivery device, preferably selected from the group consisting of transdermal patches, hollow and solid microneedle systems, microstructured transdermal systems, electrophoresis systems, and iontophoresis systems, or
- an epidermal delivery device, preferably selected from the group consisting of needle free injection systems, laser based systems, especially Erbium YAG laser systems, and gene gun systems.

14. Method for treating local skin hypotrophy conditions, preferably atrophic scars and glucocorticoid (GC)-induced skin atrophy, wherein the FGF2 mRNA and/or FGF7 mRNA as defined in any one of claims 1 to 9 is administered in an effective amount to a patient in need thereof.

15. Cosmetic skin care method, especially for the ageing skin, wherein a FGF messenger-RNA (mRNA) is contacted with the human skin, wherein the mRNA has a 5' CAP region, a 5' untranslated region (5'-UTR), a coding region encoding human FGF, a 3' untranslated region (3'-UTR) and a poly-adenosine Tail (poly-A tail), wherein the coding region of the FGF mRNA encodes for human fibroblast growth factor 2 (FGF2) or wherein the coding region of the FGF mRNA encodes for human fibroblast growth factor 7 (FGF7).


SEQUENCE LISTING

<110> Accanis Biotech F&E GmbH & Co KG

<120> Treatment of local skin hypotrophy conditions

<130> r71237

<160> 61

<170> BiSSAP 1.3

<210> 1
<211> 585
<212> RNA
<213> Homo sapiens

<220>
<221> CDS
<222> 1..585
<223> /transl_table=1

<400> 1
aug cac aaa ugg aua cug aca ugg auc cug cca acu uug cuc uac aga      48
Met His Lys Trp Ile Leu Thr Trp Ile Leu Pro Thr Leu Leu Tyr Arg
1               5                   10                  15

uca ugc uuu cac auu auc ugu cua gug ggu acu aua ucu uua gcu ugc      96
Ser Cys Phe His Ile Ile Cys Leu Val Gly Thr Ile Ser Leu Ala Cys
            20                  25                  30

aau gac aug acu cca gag caa aug gcu aca aau gug aac ugu ucc agc      144
Asn Asp Met Thr Pro Glu Gln Met Ala Thr Asn Val Asn Cys Ser Ser
        35                  40                  45

ccu gag cga cac aca aga agu uau gau uac aug gaa gga ggg gau aua      192
Pro Glu Arg His Thr Arg Ser Tyr Asp Tyr Met Glu Gly Gly Asp Ile
    50                  55                  60

aga gug aga aga cuc uuc ugu cga aca cag ugg uac cug agg auc gau      240
Arg Val Arg Arg Leu Phe Cys Arg Thr Gln Trp Tyr Leu Arg Ile Asp
65                  70                  75                  80

aaa aga ggc aaa gua aaa ggg acc caa gag aug aag aau aau uac aau      288
Lys Arg Gly Lys Val Lys Gly Thr Gln Glu Met Lys Asn Asn Tyr Asn
                85                  90                  95

auc aug gaa auc agg aca gug gca guu gga auu gug gca auc aaa ggg      336
Ile Met Glu Ile Arg Thr Val Ala Val Gly Ile Val Ala Ile Lys Gly
            100                 105                 110

gug gaa agu gaa uuc uau cuu gca aug aac aag gaa gga aaa cuc uau      384
Val Glu Ser Glu Phe Tyr Leu Ala Met Asn Lys Glu Gly Lys Leu Tyr
        115                 120                 125

gca aag aaa gaa ugc aau gaa gau ugu aac uuc aaa gaa cua auu cug      432
Ala Lys Lys Glu Cys Asn Glu Asp Cys Asn Phe Lys Glu Leu Ile Leu
    130                 135                 140

gaa aac cau uac aac aca uau gca uca gcu aaa ugg aca cac aac gga      480
Glu Asn His Tyr Asn Thr Tyr Ala Ser Ala Lys Trp Thr His Asn Gly
145                 150                 155                 160

ggg gaa aug uuu guu gcc uua aau caa aag ggg auu ccu gua aga gga     528
Gly Glu Met Phe Val Ala Leu Asn Gln Lys Gly Ile Pro Val Arg Gly
                165                 170                 175

aaa aaa acg aag aaa gaa caa aaa aca gcc cac uuu cuu ccu aug gca     576
Lys Lys Thr Lys Lys Glu Gln Lys Thr Ala His Phe Leu Pro Met Ala
            180                 185                 190

aua acu uaa                                                         585
Ile Thr


<210> 2
<211> 585
<212> RNA
<213> Homo sapiens

<220>
<221> CDS
<222> 1..585
<223> /transl_table=1

<400> 2
aug cac aaa ugg auu cuc aca ugg auu cug ccu acg uug cuc uac aga      48
Met His Lys Trp Ile Leu Thr Trp Ile Leu Pro Thr Leu Leu Tyr Arg
1               5                   10                  15

agc ugc uuu cac auc auc ugu cuu gug ggc acu auc uca cug gcu ugc      96
Ser Cys Phe His Ile Ile Cys Leu Val Gly Thr Ile Ser Leu Ala Cys
            20                  25                  30

aau gac aug aca ccg gaa cag aug gca acc aau gug aac ugu ucu ucc     144
Asn Asp Met Thr Pro Glu Gln Met Ala Thr Asn Val Asn Cys Ser Ser
        35                  40                  45

cca gaa agg cau acc aga agc uac gac uac aug gaa gga ggg gau auc     192
Pro Glu Arg His Thr Arg Ser Tyr Asp Tyr Met Glu Gly Gly Asp Ile
    50                  55                  60

agg guu cgc aga uug uuc ugu cgu acu cag ugg uau cuu cgc auc gac     240
Arg Val Arg Arg Leu Phe Cys Arg Thr Gln Trp Tyr Leu Arg Ile Asp
65                  70                  75                  80

aaa cgg ggu aag gug aag gga aca cag gag aug aag aac aac uac aac     288
Lys Arg Gly Lys Val Lys Gly Thr Gln Glu Met Lys Asn Asn Tyr Asn
                85                  90                  95

auc aug gag auu cgg aca guu gca guc ggg auu guc gcc aua aag ggu     336
Ile Met Glu Ile Arg Thr Val Ala Val Gly Ile Val Ala Ile Lys Gly
            100                 105                 110

gug gaa ucc gag uuc uau cug gcc aug aac aaa gaa ggc aaa cug uau     384
Val Glu Ser Glu Phe Tyr Leu Ala Met Asn Lys Glu Gly Lys Leu Tyr
        115                 120                 125

gcc aag aaa gag ugc aau gag gau ugc aau uuc aaa gag cug auu cug     432
Ala Lys Lys Glu Cys Asn Glu Asp Cys Asn Phe Lys Glu Leu Ile Leu
    130                 135                 140

gag aac cac uac aac acc uau gcu agu gcg aaa ugg acc cau aau gga     480
Glu Asn His Tyr Asn Thr Tyr Ala Ser Ala Lys Trp Thr His Asn Gly
145                 150                 155                 160

ggg gaa aug uuu gug gca cuc aau cag aag ggc aua ccc gua cga ggc     528
Gly Glu Met Phe Val Ala Leu Asn Gln Lys Gly Ile Pro Val Arg Gly
                165                 170                 175

aag aaa acg aag aag gag caa aag acc gcu cau uuu cug ccc aug gcc     576
Lys Lys Thr Lys Lys Glu Gln Lys Thr Ala His Phe Leu Pro Met Ala
            180                 185                 190

auc acu uga                                                         585
Ile Thr

<210> 3
<211> 585
<212> RNA
<213> Homo sapiens

<220>
<221> CDS
<222> 1..585
<223> /transl_table=1

<400> 3
aug cac aag ugg auc cug acc ugg auc cug ccu aca cug cug uac aga      48
Met His Lys Trp Ile Leu Thr Trp Ile Leu Pro Thr Leu Leu Tyr Arg
1               5                   10                  15

agc ugc uuc cac auc auc ugc cuc gug ggc aca auc agc cug gcc ugc      96
Ser Cys Phe His Ile Ile Cys Leu Val Gly Thr Ile Ser Leu Ala Cys
            20                  25                  30

aac gau aug acc ccu gag cag aug gcc acc aac gug aac ugu agc agc     144
Asn Asp Met Thr Pro Glu Gln Met Ala Thr Asn Val Asn Cys Ser Ser
        35                  40                  45

ccc gag aga cac acc cgg ucc uac gau uau aug gaa ggc ggc gac auc     192
Pro Glu Arg His Thr Arg Ser Tyr Asp Tyr Met Glu Gly Gly Asp Ile
    50                  55                  60

aga gug cgg cgg cug uuc ugu aga acc cag ugg uau cug cgg auc gac     240
Arg Val Arg Arg Leu Phe Cys Arg Thr Gln Trp Tyr Leu Arg Ile Asp
65                  70                  75                  80

aag cgg ggc aaa gug aag ggc acc caa gag aug aag aac aac uac aac     288
Lys Arg Gly Lys Val Lys Gly Thr Gln Glu Met Lys Asn Asn Tyr Asn
                85                  90                  95

auc aug gaa auc cgg acc gug gcc gug ggc auc gug gcu auu aag ggc     336
Ile Met Glu Ile Arg Thr Val Ala Val Gly Ile Val Ala Ile Lys Gly
            100                 105                 110

guc gag agc gag uuc uac cug gcc aug aac aaa gag ggc aag cug uac     384
Val Glu Ser Glu Phe Tyr Leu Ala Met Asn Lys Glu Gly Lys Leu Tyr
        115                 120                 125

gcc aag aaa gag ugc aac gag gac ugc aac uuc aaa gag cug auc cuc     432
Ala Lys Lys Glu Cys Asn Glu Asp Cys Asn Phe Lys Glu Leu Ile Leu
    130                 135                 140

gag aac cac uac aau acc uac gcc agc gcc aag ugg acc cac aau ggc     480
Glu Asn His Tyr Asn Thr Tyr Ala Ser Ala Lys Trp Thr His Asn Gly
145                 150                 155                 160

ggc gaa aug uuc gug gcc cug aac cag aaa ggc auc ccc gug cgc ggc     528
Gly Glu Met Phe Val Ala Leu Asn Gln Lys Gly Ile Pro Val Arg Gly
            165                 170                 175

aaa aag acc aaa aaa gag cag aaa acg gcc cac uuc cug ccu aug gcc     576
Lys Lys Thr Lys Lys Glu Gln Lys Thr Ala His Phe Leu Pro Met Ala
        180                 185                 190

auc acc uaa                                                         585
Ile Thr


<210> 4
<211> 585
<212> RNA
<213> Homo sapiens

<220>
<221> CDS
<222> 1..585
<223> /transl_table=1

<400> 4
aug cac aag ugg auc cuc aca ugg auc cug ccu acg cug cuc uac cgc      48
Met His Lys Trp Ile Leu Thr Trp Ile Leu Pro Thr Leu Leu Tyr Arg
1               5                   10                  15

agc ugc uuc cac auc auc ugu cug gug ggc acu auc uca cug gcu ugc      96
Ser Cys Phe His Ile Ile Cys Leu Val Gly Thr Ile Ser Leu Ala Cys
            20                  25                  30

aac gac aug acc ccg gag cag aug gca acc aac gug aac ugu agc ucc     144
Asn Asp Met Thr Pro Glu Gln Met Ala Thr Asn Val Asn Cys Ser Ser
        35                  40                  45

cca gag agg cac acc cgg agc uac gac uac aug gag gga ggg gac auc     192
Pro Glu Arg His Thr Arg Ser Tyr Asp Tyr Met Glu Gly Gly Asp Ile
    50                  55                  60

agg guc cgc aga cug uuc ugu cgu acu cag ugg uac cug cgc auc gac     240
Arg Val Arg Arg Leu Phe Cys Arg Thr Gln Trp Tyr Leu Arg Ile Asp
65                  70                  75                  80

aag cgg ggu aag gug aag gga acc cag gag aug aag aac aac uac aac     288
Lys Arg Gly Lys Val Lys Gly Thr Gln Glu Met Lys Asn Asn Tyr Asn
                85                  90                  95

auc aug gag auc cgg aca gug gcg guc ggg auc guc gcc auc aag ggu     336
Ile Met Glu Ile Arg Thr Val Ala Val Gly Ile Val Ala Ile Lys Gly
            100                 105                 110

gug gag ucc gag uuc uac cug gcc aug aac aag gag ggc aag cug uac     384
Val Glu Ser Glu Phe Tyr Leu Ala Met Asn Lys Glu Gly Lys Leu Tyr
        115                 120                 125

gcc aag aag gag ugc aac gag gac ugc aac uuc aag gag cug auc cug     432
Ala Lys Lys Glu Cys Asn Glu Asp Cys Asn Phe Lys Glu Leu Ile Leu
    130                 135                 140

gag aac cac uac aac acc uac gcu agu gcg aag ugg acc cac aac gga     480
Glu Asn His Tyr Asn Thr Tyr Ala Ser Ala Lys Trp Thr His Asn Gly
145                 150                 155                 160

ggg gag aug uuc gug gca cuc aac cag aag ggc auc ccc guc cga ggc 528
Gly Glu Met Phe Val Ala Leu Asn Gln Lys Gly Ile Pro Val Arg Gly
                165                 170                 175

aag aag acg aag aag gag cag aag acc gcg cac uuc cug ccc aug gcc 576
Lys Lys Thr Lys Lys Glu Gln Lys Thr Ala His Phe Leu Pro Met Ala
            180                 185                 190

auc acc uga 585
Ile Thr

<210> 5
<211> 585
<212> RNA
<213> Homo sapiens

<220>
<221> CDS
<222> 1..585
<223> /transl_table=1

<400> 5
aug cau aag ugg auu cuu aca ugg auu cuc cca aca cuu cuu uac aga 48
Met His Lys Trp Ile Leu Thr Trp Ile Leu Pro Thr Leu Leu Tyr Arg
1               5                   10                  15

uca ugc uuu cau auu auc ugu uug gug gga acg auu ucu cuu gcu ugu 96
Ser Cys Phe His Ile Ile Cys Leu Val Gly Thr Ile Ser Leu Ala Cys
            20                  25                  30

aau gau aug acu cca gag caa aug gcu acu aau guu aac ugu ucc uca 144
Asn Asp Met Thr Pro Glu Gln Met Ala Thr Asn Val Asn Cys Ser Ser
        35                  40                  45

ccu gag cgu cau acu aga ucu uau gau uac aug gag gga ggu gau aua 192
Pro Glu Arg His Thr Arg Ser Tyr Asp Tyr Met Glu Gly Gly Asp Ile
    50                  55                  60

aga guu agg aga cuu uuc ugu cga aca cag ugg uau cug aga auc gau 240
Arg Val Arg Arg Leu Phe Cys Arg Thr Gln Trp Tyr Leu Arg Ile Asp
65                  70                  75                  80

aag aga ggu aaa guc aaa gga acc cag gag aug aag aau aac uac aau 288
Lys Arg Gly Lys Val Lys Gly Thr Gln Glu Met Lys Asn Asn Tyr Asn
                85                  90                  95

auc aug gag auc agg aca gug gca guu gga aua guu gca auc aaa gga 336
Ile Met Glu Ile Arg Thr Val Ala Val Gly Ile Val Ala Ile Lys Gly
            100                 105                 110

guc gaa agu gag uuc uau cuu gcu aug aac aag gaa gga aaa cug uac 384
Val Glu Ser Glu Phe Tyr Leu Ala Met Asn Lys Glu Gly Lys Leu Tyr
        115                 120                 125

gca aag aaa gaa ugu aau gag gau ugc aac uuc aag gag cuu auc cug 432
Ala Lys Lys Glu Cys Asn Glu Asp Cys Asn Phe Lys Glu Leu Ile Leu
    130                 135                 140

gaa aac cau uac aac acc uau gca agu gca aaa ugg acu cac aac gga 480
Glu Asn His Tyr Asn Thr Tyr Ala Ser Ala Lys Trp Thr His Asn Gly
145                 150                 155                 160

gga gaa aug uuu guu gca uug aau cag aaa ggg aua ccu gug aga gga     528
Gly Glu Met Phe Val Ala Leu Asn Gln Lys Gly Ile Pro Val Arg Gly
                165                 170                 175

aag aaa acc aag aaa gaa cag aaa acu gcu cac uuu cuu ccu aug gcu     576
Lys Lys Thr Lys Lys Glu Gln Lys Thr Ala His Phe Leu Pro Met Ala
            180                 185                 190

auu acc uga                                                         585
Ile Thr


<210> 6
<211> 585
<212> RNA
<213> Homo sapiens

<220>
<221> CDS
<222> 1..585
<223> /transl_table=1

<400> 6
aug cac aag ugg auc cug acc ugg auc cug ccc acc cug cug uac aga      48
Met His Lys Trp Ile Leu Thr Trp Ile Leu Pro Thr Leu Leu Tyr Arg
1               5                   10                  15

agc ugc uuc cac auc auc ugc cug gug ggc acc auc agc cug gcc ugc      96
Ser Cys Phe His Ile Ile Cys Leu Val Gly Thr Ile Ser Leu Ala Cys
            20                  25                  30

aac gac aug acc ccc gag cag aug gcc acc aac gug aac ugc agc agc     144
Asn Asp Met Thr Pro Glu Gln Met Ala Thr Asn Val Asn Cys Ser Ser
        35                  40                  45

ccc gag aga cac acc aga agc uac gac uac aug gag ggc ggc gac auc     192
Pro Glu Arg His Thr Arg Ser Tyr Asp Tyr Met Glu Gly Gly Asp Ile
    50                  55                  60

aga gug aga aga cug uuc ugc aga acc cag ugg uac cug aga auc gac     240
Arg Val Arg Arg Leu Phe Cys Arg Thr Gln Trp Tyr Leu Arg Ile Asp
65                  70                  75                  80

aag aga ggc aag gug aag ggc acc cag gag aug aag aac aac uac aac     288
Lys Arg Gly Lys Val Lys Gly Thr Gln Glu Met Lys Asn Asn Tyr Asn
                85                  90                  95

auc aug gag auc aga acc gug gcc gug ggc auc gug gcc auc aag ggc     336
Ile Met Glu Ile Arg Thr Val Ala Val Gly Ile Val Ala Ile Lys Gly
            100                 105                 110

gug gag agc gag uuc uac cug gcc aug aac aag gag ggc aag cug uac     384
Val Glu Ser Glu Phe Tyr Leu Ala Met Asn Lys Glu Gly Lys Leu Tyr
        115                 120                 125

gcc aag aag gag ugc aac gag gac ugc aac uuc aag gag cug auc cug     432
Ala Lys Lys Glu Cys Asn Glu Asp Cys Asn Phe Lys Glu Leu Ile Leu
    130                 135                 140

gag aac cac uac aac acc uac gcc agc gcc aag ugg acc cac aac ggc     480
Glu Asn His Tyr Asn Thr Tyr Ala Ser Ala Lys Trp Thr His Asn Gly
145                 150                 155                 160

ggc gag aug uuc gug gcc cug aac cag aag ggc auc ccc gug aga ggc     528
Gly Glu Met Phe Val Ala Leu Asn Gln Lys Gly Ile Pro Val Arg Gly
                165                 170                 175

aag aag acc aag aag gag cag aag acc gcc cac uuc cug ccc aug gcc     576
Lys Lys Thr Lys Lys Glu Gln Lys Thr Ala His Phe Leu Pro Met Ala
            180                 185                 190

auc acc uga                                                         585
Ile Thr


<210> 7
<211> 585
<212> RNA
<213> Homo sapiens

<220>
<221> CDS
<222> 1..585
<223> /transl_table=1

<400> 7
aug cac aag ugg auc cug acc ugg auc cug ccc acc cug cug uac cgc      48
Met His Lys Trp Ile Leu Thr Trp Ile Leu Pro Thr Leu Leu Tyr Arg
1               5                   10                  15

agc ugc uuc cac auc auc ugc cug gug ggc acc auc agc cug gcc ugc      96
Ser Cys Phe His Ile Ile Cys Leu Val Gly Thr Ile Ser Leu Ala Cys
            20                  25                  30

aac gac aug acc ccc gag cag aug gcc acc aac gug aac ugc agc agc     144
Asn Asp Met Thr Pro Glu Gln Met Ala Thr Asn Val Asn Cys Ser Ser
        35                  40                  45

ccc gag cgc cac acc cgc agc uac gac uac aug gag ggc ggc gac auc     192
Pro Glu Arg His Thr Arg Ser Tyr Asp Tyr Met Glu Gly Gly Asp Ile
    50                  55                  60

cgc gug cgc cgc cug uuc ugc cgc acc cag ugg uac cug cgc auc gac     240
Arg Val Arg Arg Leu Phe Cys Arg Thr Gln Trp Tyr Leu Arg Ile Asp
65                  70                  75                  80

aag cgc ggc aag gug aag ggc acc cag gag aug aag aac aac uac aac     288
Lys Arg Gly Lys Val Lys Gly Thr Gln Glu Met Lys Asn Asn Tyr Asn
                85                  90                  95

auc aug gag auc cgc acc gug gcc gug ggc auc gug gcc auc aag ggc     336
Ile Met Glu Ile Arg Thr Val Ala Val Gly Ile Val Ala Ile Lys Gly
            100                 105                 110

gug gag agc gag uuc uac cug gcc aug aac aag gag ggc aag cug uac     384
Val Glu Ser Glu Phe Tyr Leu Ala Met Asn Lys Glu Gly Lys Leu Tyr
        115                 120                 125

gcc aag aag gag ugc aac gag gac ugc aac uuc aag gag cug auc cug     432
Ala Lys Lys Glu Cys Asn Glu Asp Cys Asn Phe Lys Glu Leu Ile Leu
    130                 135                 140

gag aac cac uac aac acc uac gcc agc gcc aag ugg acc cac aac ggc     480
Glu Asn His Tyr Asn Thr Tyr Ala Ser Ala Lys Trp Thr His Asn Gly
145                 150                 155                 160

ggc gag aug uuc gug gcc cug aac cag aag ggc auc ccc gug cgc ggc     528
Gly Glu Met Phe Val Ala Leu Asn Gln Lys Gly Ile Pro Val Arg Gly
                165                 170                 175

aag aag acc aag aag gag cag aag acc gcc cac uuc cug ccc aug gcc     576
Lys Lys Thr Lys Lys Glu Gln Lys Thr Ala His Phe Leu Pro Met Ala
            180                 185                 190

auc acc uaa                                                         585
Ile Thr


<210> 8
<211> 585
<212> RNA
<213> Homo sapiens

<220>
<221> CDS
<222> 1..585
<223> /transl_table=1

<400> 8
aug cau aag ugg auu cuu aca ugg auu cuc cca aca cug cug uac agg      48
Met His Lys Trp Ile Leu Thr Trp Ile Leu Pro Thr Leu Leu Tyr Arg
1               5                   10                  15

uca ugc uuu cac auu auc ugu cug gug gga acg auu ucu cuu gcu ugc      96
Ser Cys Phe His Ile Ile Cys Leu Val Gly Thr Ile Ser Leu Ala Cys
            20                  25                  30

aau gac aug acu cca gag caa aug gcu acu aau gug aac ugu ucc uca     144
Asn Asp Met Thr Pro Glu Gln Met Ala Thr Asn Val Asn Cys Ser Ser
        35                  40                  45

ccu gag cgu cau acu aga ucu uau gac uac aug gag gga ggu gau aua     192
Pro Glu Arg His Thr Arg Ser Tyr Asp Tyr Met Glu Gly Gly Asp Ile
    50                  55                  60

aga gug agg aga cuu uuc ugu cga aca cag ugg uau cug cgg auc gau     240
Arg Val Arg Arg Leu Phe Cys Arg Thr Gln Trp Tyr Leu Arg Ile Asp
65                  70                  75                  80

aag aga ggu aaa guc aaa ggc acc cag gag aug aag aau aac uac aau     288
Lys Arg Gly Lys Val Lys Gly Thr Gln Glu Met Lys Asn Asn Tyr Asn
                85                  90                  95

auc aug gag auc agg aca gug gca guu gga aua guu gca auc aaa ggg     336
Ile Met Glu Ile Arg Thr Val Ala Val Gly Ile Val Ala Ile Lys Gly
            100                 105                 110

guc gaa agc gag uuc uau cuu gcu aug aac aag gaa ggc aaa cug uac     384
Val Glu Ser Glu Phe Tyr Leu Ala Met Asn Lys Glu Gly Lys Leu Tyr
        115                 120                 125

gcc aag aaa gaa ugc aau gag gau ugc aac uuc aag gag cuu auc cug     432
Ala Lys Lys Glu Cys Asn Glu Asp Cys Asn Phe Lys Glu Leu Ile Leu
    130                 135                 140

gaa aac cau uac aac acc uau gca agu gca aaa ugg acu cac aac gga     480
Glu Asn His Tyr Asn Thr Tyr Ala Ser Ala Lys Trp Thr His Asn Gly
145                 150                 155                 160

ggg gaa aug uuu guu gca uug aau cag aaa ggg aua ccu gug aga ggc 528
Gly Glu Met Phe Val Ala Leu Asn Gln Lys Gly Ile Pro Val Arg Gly
                165                 170                 175

aag aaa acc aag aaa gaa cag aaa acu gcc cac uuu cuu ccu aug gcu 576
Lys Lys Thr Lys Lys Glu Gln Lys Thr Ala His Phe Leu Pro Met Ala
            180                 185                 190

auu acc uga 585
Ile Thr

<210> 9
<211> 585
<212> RNA
<213> Homo sapiens

<220>
<221> CDS
<222> 1..585
<223> /transl_table=1

<400> 9
aug cau aag ugg aua uug acg ugg auu uua ccu acu cuc cua uau agg 48
Met His Lys Trp Ile Leu Thr Trp Ile Leu Pro Thr Leu Leu Tyr Arg
1               5                   10                  15

ucc ugc uuc cau aua auu ugu uug gug ggc acc auu ucu cuu gcc ugc 96
Ser Cys Phe His Ile Ile Cys Leu Val Gly Thr Ile Ser Leu Ala Cys
            20                  25                  30

aau gau aug aca ccc gag cag aug gca acc aac gua aac ugu ucc uca 144
Asn Asp Met Thr Pro Glu Gln Met Ala Thr Asn Val Asn Cys Ser Ser
        35                  40                  45

ccc gag cga cau acg aga agc uac gac uac aug gag gga ggu gau auu 192
Pro Glu Arg His Thr Arg Ser Tyr Asp Tyr Met Glu Gly Gly Asp Ile
    50                  55                  60

agg guc aga cgc cug uuu ugu cgg aca cag ugg uau cuu aga auu gac 240
Arg Val Arg Arg Leu Phe Cys Arg Thr Gln Trp Tyr Leu Arg Ile Asp
65                  70                  75                  80

aaa cgu ggu aag guc aag ggg acc cag gaa aug aaa aau aac uau aau 288
Lys Arg Gly Lys Val Lys Gly Thr Gln Glu Met Lys Asn Asn Tyr Asn
                85                  90                  95

auc aug gaa auc cgc acc gug gca gug ggg auc gug gcg auc aag gga 336
Ile Met Glu Ile Arg Thr Val Ala Val Gly Ile Val Ala Ile Lys Gly
            100                 105                 110

gug gaa agc gaa uuc uau cug gcu aug aac aaa gag gga aag cug uac 384
Val Glu Ser Glu Phe Tyr Leu Ala Met Asn Lys Glu Gly Lys Leu Tyr
        115                 120                 125

gcu aaa aaa gaa ugc aau gag gac ugc aac uuu aaa gaa cug auc cuc 432
Ala Lys Lys Glu Cys Asn Glu Asp Cys Asn Phe Lys Glu Leu Ile Leu
    130                 135                 140

gag aau cac uac aau acc uac gcc agu gcc aag ugg aca cac aac ggg 480
Glu Asn His Tyr Asn Thr Tyr Ala Ser Ala Lys Trp Thr His Asn Gly
145                 150                 155                 160

ggc gag aug uuc guu gca cug aac cag aag ggc auc cca guu cgg ggc     528
Gly Glu Met Phe Val Ala Leu Asn Gln Lys Gly Ile Pro Val Arg Gly
                165                 170                 175

aag aaa aca aaa aag gag caa aag acu gcu cac uuu cuc ccg aug gcc     576
Lys Lys Thr Lys Lys Glu Gln Lys Thr Ala His Phe Leu Pro Met Ala
            180                 185                 190

auc acu uga                                                         585
Ile Thr

<210> 10
<211> 585
<212> RNA
<213> Homo sapiens

<220>
<221> CDS
<222> 1..585
<223> /transl_table=1

<400> 10
aug cau aaa ugg auc cuu acg ugg aua cug ccg aca cuc cuu uau agg      48
Met His Lys Trp Ile Leu Thr Trp Ile Leu Pro Thr Leu Leu Tyr Arg
1               5                   10                  15

ucu ugu uuu cac aua auu ugc cuc guu gga acu aua ucu cuu gcc ugc      96
Ser Cys Phe His Ile Ile Cys Leu Val Gly Thr Ile Ser Leu Ala Cys
            20                  25                  30

aac gac aug acc cca gaa caa aug gcu aca aac gug aau ugu ucc agu     144
Asn Asp Met Thr Pro Glu Gln Met Ala Thr Asn Val Asn Cys Ser Ser
        35                  40                  45

ccc gaa aga cac acg cga agu uau gac uac aug gaa ggc ggc gau aua     192
Pro Glu Arg His Thr Arg Ser Tyr Asp Tyr Met Glu Gly Gly Asp Ile
    50                  55                  60

aga guu agg aga cuu uuu ugu cga acg caa ugg uau cug agg auu gac     240
Arg Val Arg Arg Leu Phe Cys Arg Thr Gln Trp Tyr Leu Arg Ile Asp
65                  70                  75                  80

aag cgc ggg aag gua aaa ggg acc cag gag aug aag aac aac uau aac     288
Lys Arg Gly Lys Val Lys Gly Thr Gln Glu Met Lys Asn Asn Tyr Asn
                85                  90                  95

aua aug gag auu agg aca gug gcu gug ggc auc gua gcg auc aaa ggu     336
Ile Met Glu Ile Arg Thr Val Ala Val Gly Ile Val Ala Ile Lys Gly
            100                 105                 110

gua gaa uca gag uuu uac cug gcc aug aac aaa gaa ggu aaa cuu uau     384
Val Glu Ser Glu Phe Tyr Leu Ala Met Asn Lys Glu Gly Lys Leu Tyr
        115                 120                 125

gcu aaa aaa gaa ugc aac gaa gau ugu aac uuc aaa gaa uug auc cuu     432
Ala Lys Lys Glu Cys Asn Glu Asp Cys Asn Phe Lys Glu Leu Ile Leu
    130                 135                 140

gaa aau cac uau aac aca uau gca ucc gcg aag ugg aca cau aac ggg     480
Glu Asn His Tyr Asn Thr Tyr Ala Ser Ala Lys Trp Thr His Asn Gly
145                 150                 155                 160

gga gaa aug uuc guc gcg uug aau caa aaa ggu auu ccg guu cgg gga     528
Gly Glu Met Phe Val Ala Leu Asn Gln Lys Gly Ile Pro Val Arg Gly
                165                 170                 175

aaa aaa acc aag aag gag cag aag acg gcu cac uuc uug cca aug gcc     576
Lys Lys Thr Lys Lys Glu Gln Lys Thr Ala His Phe Leu Pro Met Ala
            180                 185                 190

aua acu uaa                                                         585
Ile Thr


<210> 11
<211> 585
<212> RNA
<213> Homo sapiens

<220>
<221> CDS
<222> 1..585
<223> /transl_table=1

<400> 11
aug cac aag ugg auc cuu acg ugg aua cuc cca aca cuu uug uau cga      48
Met His Lys Trp Ile Leu Thr Trp Ile Leu Pro Thr Leu Leu Tyr Arg
1               5                   10                  15

agu ugu uuu cac auu auu ugc cug guc ggc acg auu uca uug gcc ugc      96
Ser Cys Phe His Ile Ile Cys Leu Val Gly Thr Ile Ser Leu Ala Cys
            20                  25                  30

aac gau aug aca ccg gaa cag aug gcu aca aac gua aac ugu agu uca     144
Asn Asp Met Thr Pro Glu Gln Met Ala Thr Asn Val Asn Cys Ser Ser
        35                  40                  45

ccc gag cgg cac acu cga ucu uac gau uac aug gaa ggu gga gac auc     192
Pro Glu Arg His Thr Arg Ser Tyr Asp Tyr Met Glu Gly Gly Asp Ile
    50                  55                  60

agg guu aga aga cuc uuu ugc agg acg caa ugg uac cuc cgc aua gau     240
Arg Val Arg Arg Leu Phe Cys Arg Thr Gln Trp Tyr Leu Arg Ile Asp
65                  70                  75                  80

aag aga gga aag gug aaa gga aca cag gaa aug aaa aau aac uac aac     288
Lys Arg Gly Lys Val Lys Gly Thr Gln Glu Met Lys Asn Asn Tyr Asn
                85                  90                  95

aua aug gaa auu cgg acu guc gcu gug gga auc guu gcc auc aaa gga     336
Ile Met Glu Ile Arg Thr Val Ala Val Gly Ile Val Ala Ile Lys Gly
            100                 105                 110

gug gaa uca gaa uuc uac cug gcu aug aau aag gag gga aag cuc uau     384
Val Glu Ser Glu Phe Tyr Leu Ala Met Asn Lys Glu Gly Lys Leu Tyr
        115                 120                 125

gcg aaa aag gag ugc aac gag gac ugu aau uuc aaa gaa cuu auc cuu     432
Ala Lys Lys Glu Cys Asn Glu Asp Cys Asn Phe Lys Glu Leu Ile Leu
    130                 135                 140

gaa aac cau uac aac acc uau gcg agu gcc aag ugg acu cau aac ggu     480
Glu Asn His Tyr Asn Thr Tyr Ala Ser Ala Lys Trp Thr His Asn Gly
145                 150                 155                 160

ggu gag aug uuc gua gcu cug aau cag aag ggc auu ccg guc cgg gga     528
Gly Glu Met Phe Val Ala Leu Asn Gln Lys Gly Ile Pro Val Arg Gly
                165                 170                 175

aag aag acu aag aaa gag cag aaa acg gca cac uuu cuu ccu aug gcg     576
Lys Lys Thr Lys Lys Glu Gln Lys Thr Ala His Phe Leu Pro Met Ala
            180                 185                 190

auu aca uaa                                                         585
Ile Thr

<210> 12
<211> 585
<212> RNA
<213> Homo sapiens

<220>
<221> mRNA
<222> 1..585

<400> 12
augcacaaru ggauycusac muggauycug ccyacvyugc usuacmgmag cugcuuycac      60

aucaucugyc ubgugggcac haucwsmcug gcyugcaayg ayaugacmcc bgarcagaug     120

gcmaccaayg ugaacugyws ywscccmgar mgvcayaccm gvwscuacga yuayauggar     180

ggmggsgaya ucmgvgubmg vmgvyuguuc ugymghacyc agugguaycu kmgvaucgac     240

aarmgvggya argugaaggg macmcargag augaagaaca acuacaacau cauggarauy     300

mgvacmgtkg cvgtsggsat ygtsgcyath aagggygtsg arwscgagtt ctayctggcc     360

augaacaarg arggcaarcu guaygccaag aargagugca aygaggayug caayuucaar     420

gagcugauyc usgagaacca cuacaayacc uaygcyagyg csaaruggac ccayaayggm     480

ggsgaraugu uyguggcmcu saaycagaar ggcaumcccg uvmgmggcaa raaracsaar     540

aargagcara aracsgcbca yuuycugccy auggccauca cyura                     585

<210> 13
<211> 585
<212> RNA
<213> Homo sapiens

<220>
<221> unsure
<222>
join(33,36,42,81,111,153,186,216,219,246,249,312,324,327,336,387,456,480,483,
495,498,519,522,537,558,570)
<223> /note="any nucleotide"

<400> 13
augcayaaru ggauhyubac vuggauhyuv ccnacnyuby unuaymgvws hugyuuycay      60

auhauyugyy ubgubggmac nauhwshyuk gcyugyaayg ayaugachcc ngarcaraug     120

gchachaayg udaayugyws ywshcchgar mgncayacbm gvwsyuayga yuayauggar     180

gghggngaya uhmgvgubmg vmgvyubuuy ugymgnacnc arugguaycu bmgvauhgay     240

aarmgnggna arguvaargg vacmcargar augaaraaya acuayaayau mauggarauy     300

mgvachgtbg cngtbggvat hgtngcnath aarggngtvg arwshgartt ytayctkgcy     360

augaayaarg argghaarcu buaygcnaar aargarugya aygargayug yaayuuyaar     420

garyukauyc ubgaraayca yuayaayacm uaygcnwsyg cvaaruggac hcayaayggn     480

ggngaraugu uygungcnyu saaycaraar ggbauhccng unmgvggmaa raaracnaar     540

aargarcara aracbgcnca yuuyyubccn auggcbauha chura                     585


<210> 14
<211> 585
<212> RNA
<213> Homo sapiens

<220>
<221> unsure
<222>
join(33,36,42,72,81,111,153,159,186,216,219,246,249,312,324,327,336,387,456,4
62,480,483,495,498,519,522,537,555,558,570,576)
<223> /note="any nucleotide"

<400> 14
augcayaaru ggauhyubac vuggauhyuv ccnacnyuby unuaymgvws hugyuuycay      60

auhauyugyy ungubgghac nauhwshyud gcyugyaayg ayaugachcc ngarcaraug     120

gchachaayg udaayugyws ywshcchgar mgncayacnm gvwsyuayga yuayauggar     180

gghggngaya uhmgvgubmg vmgvyubuuy ugymgnacnc arugguaycu bmgvauhgay     240

aarmgnggna arguvaargg vacmcargar augaaraaya ayuayaayau mauggarauy     300

mgvachgtbg cngtbggvat hgtngcnath aarggngtvg arwshgartt ytayctkgch     360

augaayaarg argghaarcu buaygcnaar aargarugya aygargayug yaayuuyaar     420

garyudauyc ubgaraayca yuayaayacm uaygcnwshg cnaaruggac hcayaayggn     480

ggngaraugu uygungcnyu vaaycaraar ggbauhccng unmgvggmaa raaracnaar     540

aargarcara aracngcnca yuuyyubccn auggcnauha chura                     585


<210> 15
<211> 194
<212> PRT
<213> Homo sapiens

<400> 15
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser
        35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp

65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110
Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr

<210> 16
<211> 72
<212> RNA
<213> Homo sapiens

<220>
<221> misc_signal
<222> 1..5
<223> /function="Kozak"

<220>
<221> 5'UTR
<222> 1..72
<223> /function="including Koszak sequence"

<400> 16
gggagacaua aacccuggcg cgcucgcggc ccggcacucu ucuggucccc acagacucag      60

agagaaccca cc                                                           72

<210> 17
<211> 111
<212> RNA
<213> Homo sapiens

<220>
<221> 3'UTR
<222> 1..111
<223> /note="aGlobin 3'UTR including poly A site and first A of 120"

<220>
<221> polyA_signal
<222> 90..95

<220>
<221> polyA_site
<222> 111
<223> /note="first A of 120"

<400> 17
gcuggagccu cgguggccau gcuucuugcc ccuugggccu ccccccagcc ccuccucccc      60

uuccugcacc cguacccccg uggucuuuga auaaagucug agugggcggc a              111

<210> 18
<211> 468
<212> RNA
<213> Homo sapiens

<220>
<221> CDS
<222> 1..468
<223> /transl_table=1

<400> 18
aug gca gcc ggg agc auc acc acg cug ccc gcc uug ccc gag gau ggc      48
ggc agc ggc gcc uuc ccg ccc ggc cac uuc aag gac ccc aag cgg cug      96
uac ugc aaa aac ggg ggc uuc uuc cug cgc auc cac ccc gac ggc cga      144
guu gac ggg guc cgg gag aag agc gac ccu cac auc aag cua caa cuu      192
caa gca gaa gag aga gga guu gug ucu auc aaa gga gug ugu gcu aac      240
cgu uac cug gcu aug aag gaa gau gga aga uua cug gcu ucu aaa ugu      288
guu acg gau gag ugu uuc uuu uuu gaa cga uug gaa ucu aau aac uac      336
aau acu uac cgg uca agg aaa uac acc agu ugg uau gug gca cug aaa      384
cga acu ggg cag uau aaa cuu gga ucc aaa aca gga ccu ggg cag aaa      432
gcu aua cuu uuu cuu cca aug ucu gcu aag agc uga                      468

<210> 19
<211> 468
<212> RNA
<213> Homo sapiens

<220>
<221> CDS
<222> 1..468
<223> /transl_table=1

<400> 19
aug gcu gca ggc agu auc acc acu cuc cca gca uug ccu gaa gau gga     48
ggu uca ggc gcc uuu ccu cca ggc cac uuu aaa gac ccc aag aga cug     96
uac ugc aag aau ggu ggg uuc uuc cug cgc auu cau ccc gau gga cgu     144
gua gac gga guc agg gaa aag uca gau ccg cac aua aag cuc cag cuc     192
caa gcu gag gaa aga ggg guu gug ucc auc aaa ggg gug ugu gcc aau     240
cgc uau cug gcg aug aaa gag gac ggc aga cuu cug gcu agc aag ugu     288
gug aca gac gag ugc uuc uuc uuu gag cgg uug gag ucc aac aac uac     336
aac acc uau cga agc agg aag uac acg ucu ugg uau guc gca cug aaa     384
cgg acu ggg cag uac aag cuu ggc agc aag aca gga ccu ggu cag aaa     432
gcc auu cug uuu cug ccc aug ucu gcc aaa agu uga                     468

<210> 20
<211> 468
<212> RNA
<213> Homo sapiens

<220>
<221> CDS
<222> 1..468
<223> /transl_table=1

<400> 20
aug gcc gcu ggc ucu auu aca aca cug ccc gcu cug ccu gag gau ggc     48
gga ucu ggu gcu uuu cca ccu ggc cac uuc aag gac ccc aag cgg cug     96
uac ugc aag aac ggc gga uuc uuc cug cgg auu cac ccc gac gga aga     144
gug gac ggc gug cgg gaa aaa agc gac ccu cac auc aag cuc cag cug     192
cag gcc gaa gag aga ggc guc guc agu auc aaa ggc gug ugc gcc aac     240
aga uac cug gcc aug aag gaa gau ggc cgg cug cug gcc ucu aag ugc     288
gug acc gau gag ugc uuc uuc uuc gaa cgg cug gaa agc aac aac uac     336
aac acc uac aga agc cgg aag uac acc ucu ugg uac gug gcc cug aag     384
cgg acc ggc cag uau aag cug ggc ucu aag aca ggc cca ggc cag aag     432
gcc auc cug uuu cug ccu aug agc gcc aag agc uga                     468

<210> 21
<211> 468
<212> RNA
<213> Homo sapiens

<220>
<221> CDS
<222> 1..468
<223> /transl_table=1

<400> 21
aug gcu gca ggc agc auc acc acc cuc cca gca cug ccu gag gac gga      48
ggu ucc ggc gcc uuc ccu cca ggc cac uuc aag gac ccc aag cgc cug      96
uac ugc aag aac ggu ggg uuc uuc cug cgc auc cac ccc gau ggc cgu     144
guc gac ggc guc agg gag aag ucc gac ccg cac aua aag cuc cag cuc     192
cag gcu gag gag aga ggg guc gug ucc auc aag ggg gug ugc gcc aau     240
cgc uau cug gcg aug aag gag gac ggc agg cuc cug gcu agc aag ugu     288
gug acc gac gag ugc uuc uuc uuu gag cgg cug gag ucc aac aac uac     336
aac acc uac cga agc cgc aag uac acg agc ugg uac guc gca cug aag     384
cgg acu ggg cag uac aag cug ggc agc aag aca gga ccu ggu cag aag     432
gcc auc cug uuc cug ccc aug ucc gcc aag agc uga                     468

<210> 22
<211> 468
<212> RNA
<213> Homo sapiens

<220>
<221> CDS
<222> 1..468
<223> /transl_table=1

<400> 22
aug gca gca ggu agu auu acc acu cuu ccu gcu uug ccu gaa gau ggu      48
ggu uca ggu gcu uuu ccu cca ggu cau uuc aaa gau ccu aag aga uug      96
uau ugu aag aac gga gga uuc uuu cug aga aua cac cca gau ggc aga     144
guu gau ggu guc cgu gaa aag ucu gau ccu cac auc aag cug cag cuu     192
caa gcc gaa gag agg gga guu gug ucu auc aaa ggu gug ugu gcu aau     240
aga uac cug gcu aug aaa gaa gau ggu aga cuu cug gca uca aag ugu     288
gug acg gau gaa ugc uuc uuu uuc gag cgu uug gaa ucc aac aau uac     336
aac aca uac cgu agc aga aag uac aca agu ugg uau guu gca cug aaa     384
cga aca ggu cag uau aaa cug ggu ucu aaa aca gga cca gga cag aag     432
gcg auu uug uuu cuu ccg aug ucu gcu aag ucu uga                    468

<210> 23
<211> 468
<212> RNA
<213> Homo sapiens

<220>
<221> CDS
<222> 1..468
<223> /transl_table=1

<400> 23
aug gcc gcc ggc agc auc acc acc cug ccc gcc cug ccc gag gac ggc      48
ggc agc ggc gcc uuc ccc ccc ggc cac uuc aag gac ccc aag cgc cug      96
uac ugc aag aac ggc ggc uuc uuc cug cgc auc cac ccc gac ggc cgc     144
gug gac ggc gug cgc gag aag agc gac ccc cac auc aag cug cag cug     192
cag gcc gag gag cgc ggc gug gug agc auc aag ggc gug ugc gcc aac     240
cgc uac cug gcc aug aag gag gac ggc cgc cug cug gcc agc aag ugc     288
gug acc gac gag ugc uuc uuc uuc gag cgc cug gag agc aac aac uac     336
aac acc uac cgc agc cgc aag uac acc agc ugg uac gug gcc cug aag     384
cgc acc ggc cag uac aag cug ggc agc aag acc ggc ccc ggc cag aag     432
gcc auc cug uuc cug ccc aug agc gcc aag agc uaa                    468

<210> 24
<211> 468
<212> RNA
<213> Homo sapiens

<220>
<221> CDS
<222> 1..468

<223> /transl_table=1

<400> 24
aug gcc gcu ggc agc auc aca aca uug ccu gcu cug ccu gag gau ggc      48
ggc ucu ggu gcu uuu cca ccu ggc cac uuc aag gac ccc aag cgg cug      96
uac ugc aag aac ggc gga uuc uuc cug cgg auu cac ccc gac gga aga      144
gug gac ggc gug cgg gaa aaa agc gac ccu cac auc aag cuc cag cug      192
cag gcc gaa gag aga ggc guc guc agu auc aaa ggc gug ugc gcc aac      240
aga uac cug gcc aug aag gaa gau ggc cgg cug cug gcc ucu aag ugc      288
gug acc gau gag ugc uuc uuc uuc gaa cgg cug gaa agc aac aac uac      336
aac acc uac aga agc cgg aag uac acc ucu ugg uac gug gcc cug aag      384
cgg acc ggc cag uau aag cug ggc ucu aag aca ggc cca ggc cag aag      432
gcc auc cug uuu cug ccu aug agc gcc aag agc uga                      468

<210> 25
<211> 468
<212> RNA
<213> Homo sapiens

<220>
<221> CDS
<222> 1..468
<223> /transl_table=1

<400> 25
aug gca gca ggc agc auc acu acc uug ccc gcc cuu ccg gaa gau ggg      48
gga agc ggg gcc uuc ccc cca ggg cac uuu aag gau ccg aag cga cug      96
uau ugu aaa aac ggg ggc uuc uuu cuu cgg auc cau cca gau ggc cga      144
gua gac ggc guc cga gaa aag agu gac ccc cau auc aaa cuu cag cuc      192
cag gcc gag gaa agg ggu gug gug agu aua aag ggg gug ugc gcg aau      240
cga uac cuu gcu aug aag gag gac ggu cgc cuu cuc gcc agc aaa ugc      288
gug acu gac gag ugc uuc uuu uuc gag cga uug gaa ucc aac aau uac      336
aac aca uac cgg agu aga aaa uau acc ucc ugg uau gua gcg cug aaa      384
agg acc ggg cag uau aag cuc ggg agu aaa acc ggu ccg ggc caa aaa      432
gca aua cug uuu cuu ccc aug agc gcc aaa ucc uga                      468

<210> 26
<211> 468
<212> RNA
<213> Homo sapiens

<220>
<221> CDS
<222> 1..468
<223> /transl_table=1

<400> 26
aug gcc gcg ggc uca aua acc acg cuu ccu gcc cug ccc gag gac ggg       48
gga uca ggu gca uuc ccu cca ggc cac uuu aaa gau ccc aaa cga cug       96
uac ugc aaa aac ggc ggc uuu uuc uug cga auc cau ccc gac ggg aga      144
guu gau ggu guc aga gaa aaa agu gac ccg cac aua aag cuc caa cug      192
caa gcg gaa gaa agg ggc guu guc ucc auu aaa gga gug ugc gcg aau      240
agg uac cug gcu aug aag gag gac gga cga uug cuc gcc uca aag ugc      288
gua acc gau gag ugc uuu uuu uuc gag cgg cuc gaa uca aac aau uac     336
aac aca uac cga agc cgc aag uac acg ucu ugg uac guc gcc cug aag     384
agg acg gga cag uac aaa cuc ggg uca aaa acc ggc ccc gga caa aag     432
gcu auc cuc uuu cuc ccu aug ucc gca aaa ucu uga                     468

<210> 27
<211> 468
<212> RNA
<213> Homo sapiens

<220>
<221> CDS
<222> 1..468
<223> /transl_table=1

<400> 27
aug gca gcu ggc ucu auu acu acg cug ccg gcu cuc ccu gag gac gga      48
ggc ucc ggu gcc uuc ccc cca ggg cac uuu aaa gau cca aaa agg cuu      96
uau ugu aaa aac ggc ggg uuu uuu cuc cgg auc cac ccc gac ggc cgc     144
gua gau gga gug agg gaa aag agc gac ccu cau aua aaa cug cag cug     192
cag gcu gag gag cgg gga guc guu ucg auc aaa ggg guc ugc gca aac     240
cgc uac cuu gca aug aag gaa gac gga aga cuc cua gcg agu aaa ugu     288
gug aca gau gaa ugc uuc uuc uuu gag aga cug gag ucc aau aau uau     336
aac acc uac aga agc cga aag uau acu agu ugg uac gug gcc uug aag     384
cgu acc ggu caa uac aag cug ggc ucu aag aca ggu ccc ggg cag aag     432
gcc auu uua uuc uug ccu aug uca gcc aag uca uga                 468

<210> 28
<211> 468
<212> RNA
<213> Homo sapiens

<220>
<221> CDS
<222> 1..468
<223> /transl_table=1

<400> 28
aug gca gcc ggu ucg auu acu acc cua ccu gcc cuc ccg gaa gau ggu      48
gga agu ggc gca uuu ccu cca gga cau uuu aag gau cca aaa cgc cug      96
uac ugc aag aau ggu gga uuc uuu uua cgc auu cac ccc gau ggg cga     144
guc gac ggg guc cgu gaa aag ucc gac ccc cac auc aaa cuc cag uug     192
caa gcu gag gag aga ggc gug guu uca auc aag ggc gua ugc gcu aau     240
aga uau cuu gcc aug aag gag gac ggg cgg cuc cug gcc uca aaa ugu     288
gug acu gac gag ugu uuu uuc uuc gag cgg cug gaa ucc aac aac uac     336
aac aca uac agg agu aga aaa uac acc ucu ugg uau gug gca cuu aaa     384
agg acg gga cag uau aag uug ggg ucu aag aca ggc ccu ggc cag aaa     432
gcg aua cug uuc cug ccc aug agc gcu aag agc uga                 468

<210> 29
<211> 468
<212> RNA
<213> Homo sapiens

<220>
<221> CDS
<222> 1..468
<223> /transl_table=1

<400> 29
aug gcc gca ggc agc auu acc acu cuu ccu gcc uug ccu gag gac ggu      48
ggu uca ggu gcu uuu ccu cca ggu cau uuc aaa gac ccu aag cga cuc      96
uau ugc aag aac gga ggc uuc uuu cug agg aua cac cca gac ggc aga      144
guu gac ggu guc cgu gaa aag ucu gau ccu cac auc aag cug cag cuu      192
caa gcc gaa gag agg gga guu gug ucu auc aaa ggg gug ugu gcu aau      240
cgg uac cug gcu aug aaa gaa gau ggu aga cuc cug gca uca aag ugu      288
gug acg gau gag ugc uuc uuu uuc gag cgu uug gag ucc aac aau uac      336
aac acc uac cgu agc aga aag uac acc agu ugg uau gug gca cug aaa      384
cga aca ggu cag uau aaa cug ggu agc aaa aca gga cca gga cag aag      432
gcg auu uug uuu cuu ccg aug ucu gcu aag ucu uga                      468

<210> 30
<211> 468
<212> RNA
<213> Homo sapiens

<220>
<221> unsure
<222> join(9,24,147,423)
<223> /note="any nucleotide"

<400> 30
   auggchgcng gcwshauhac hacnyubcch gchyukccbg argayggvgg hwshggbgch      60
   uuycchcchg gscacuuyaa rgayccsaar mgvcuguayu gyaaraaygg bggvuuyuuy      120
   ytkcgvatyc ayccmgaygg vmghgtngay gghgtsmgvg araarwshga yccbcayatm      180
   aarcubcarc uscargcbga rgarmgvggb gubguswsya uhaarggvgu gugygcsaay 240
   mgvtayctkg cbatgaarga rgayggbmgv ytbctsgcyw shaartgygt rachgaygag 300
   ugcuuyuuyu uygarcgvyu sgarwsmaac aayuacaaca cmuaymgvag ymgvaaruay 360
   acswsyuggu ayguvgcvcu gaarmgsacb ggvcaguaya arcubggsws haaracmggh 420
   ccngghcara argchauhcu suuycubccy augwsygcma arwsyura 468

<210> 31
<211> 468
<212> RNA
<213> Homo sapiens

<220>
<221> unsure
<222>
join(9,15,24,27,30,48,72,87,111,123,147,153,159,210,219,228,237,252,267,279,2
94,318,348,363,375,387,390,393,423,426,435)
<223> /note="any nucleotide"

<400> 31
auggchgcng gywsnauhac hacnyunccn gchyubccbg argayggngg hwshggbgch 60

uuycchcchg gncayuuyaa rgayccnaar mgvyubuayu gyaaraaygg nggvuuyuuy 120

ytnmgvathc ayccmgaygg vmghgtngay ggngtsmgng araarwshga yccbcayatm 180

aarcubcary ubcargcbga rgarmgvggn gubgubwsna uhaarggngu vugygcnaay 240

mgvtayctkg cnatgaarga rgayggnmgv ytbctvgcnw shaartgygt racngaygar 300

ugyuuyuuyu uygarmgnyu sgarwsmaay aayuayaaca cmuaymgnag ymgvaaruay 360

acnwsyuggu aygungcvyu kaarmgnacn ggncaruaya aryubggbws haaracmggh 420

ccnggncara argcnauhyu vuuyyubccb augwshgcha arwshura 468

<210> 32
<211> 468
<212> RNA
<213> Homo sapiens

<220>
<221> unsure
<222>
join(9,15,24,27,30,48,66,72,87,111,123,147,153,159,186,198,210,219,228,237,24
3,252,267,273,279,294,318,348,363,375,387,390,393,408,423,426,435,441,450)
<223> /note="any nucleotide"

<400> 32
auggchgcng gbwsnauhac hacnyunccn gchyubccbg argayggngg hwshggbgch 60

uuyccncchg gncayuuyaa rgayccnaar mgvyubuayu gyaaraaygg nggvuuyuuy 120

ytnmgvathc ayccmgaygg vmghgtngay ggngtsmgng araarwshga yccbcayatm 180

aarcuncary ubcargcnga rgarmgvggn gubgubwsna uhaarggngu vugygcnaay 240

mgntayctkg cnatgaarga rgayggnmgv ytnctvgcnw shaartgygt dacngaygar 300

ugyuuyuuyu uygarmgnyu sgarwshaay aayuayaaya chuaymgnws hmgvaaruay 360

acnwsyuggu aygungcvyu kaarmgnacn ggncaruaya aryubggnws haaracmggh 420

ccnggncara argcnauhyu nuuyyubccn augwshgcha arwshura 468

<210> 33
<211> 155
<212> PRT
<213> Homo sapiens

<400> 33

<210> 34
<211> 19
<212> DNA
<213> Homo sapiens

<220>
<223> /PCR_primers="[fwd_name: RPL41, ]fwd_seq:agcgtggctgtctcctctc1"

<400> 34
agcgtggctg tctcctctc 19

<210> 35
<211> 20
<212> DNA
<213> Homo sapiens

<220>
<223> /PCR_primers="[rev_name: RPL41, ]rev_seq: gagccttgaatacagcaggc1"

<400> 35
gagccttgaa tacagcaggc 20

<210> 36
<211> 20
<212> DNA
<213> Mus musculus

<220>
<223> /PCR_primers="[fwd_name:ACTB1, ]fwd_seq: ggctgtattcccctccatcg1"

<400> 36
ggctgtattc ccctccatcg                                                    20


<210> 37
<211> 22
<212> DNA
<213> Mus musculus

<220>
<223> /PCR_primers="[rev_name: ACTB1, ]rev_seq:
      ccagttggtaacaatgccatgt1"

<400> 37
ccagttggta acaatgccat gt                                                 22


<210> 38
<211> 15
<212> RNA
<213> Homo sapiens

<220>
<221> unsure
<222> 5
<223> /note="any 0 to 10 nucleotides"

<220>
<221> unsure
<222> 10
<223> /note="any 0 to 10 pyrimidine (y) nucleotides"

<400> 38
yccancccwn ucycc                                                         15


<210> 39
<211> 194
<212> PRT
<213> Homo sapiens

<220>
<223> [CDS]:1..585 from SEQ ID NO 1

<400> 39
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser
        35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110

Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr

<210> 40
<211> 194
<212> PRT
<213> Homo sapiens

<220>
<223> [CDS]:1..585 from SEQ ID NO 2

<400> 40
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser
        35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110
Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr

<210> 41
<211> 194
<212> PRT
<213> Homo sapiens

<220>
<223> [CDS]:1..585 from SEQ ID NO 3

<400> 41
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser

35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110
Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr

<210> 42
<211> 194
<212> PRT
<213> Homo sapiens

<220>
<223> [CDS]:1..585 from SEQ ID NO 4

<400> 42
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser
        35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110
Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr

<210> 43
<211> 194
<212> PRT
<213> Homo sapiens

<220>
<223> [CDS]:1..585 from SEQ ID NO 5

<400> 43
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser
        35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110
Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr


<210> 44
<211> 194
<212> PRT
<213> Homo sapiens

<220>
<223> [CDS]:1..585 from SEQ ID NO 6

<400> 44
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser
        35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110
Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala

180                 185                 190
Ile Thr



<210> 45
<211> 194
<212> PRT
<213> Homo sapiens

<220>
<223> [CDS]:1..585 from SEQ ID NO 7

<400> 45
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser
        35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110
Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr



<210> 46
<211> 194
<212> PRT
<213> Homo sapiens

<220>
<223> [CDS]:1..585 from SEQ ID NO 8

<400> 46
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser
        35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110

Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr

<210> 47
<211> 194
<212> PRT
<213> Homo sapiens

<220>
<223> [CDS]:1..585 from SEQ ID NO 9

<400> 47
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser
        35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110
Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr

<210> 48
<211> 194
<212> PRT
<213> Homo sapiens

<220>
<223> [CDS]:1..585 from SEQ ID NO 10

<400> 48
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser

35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110
Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr

<210> 49
<211> 194
<212> PRT
<213> Homo sapiens

<220>
<223> [CDS]:1..585 from SEQ ID NO 11

<400> 49
Met His Lys Trp Ile Leu Thr Trp Ile Leu Pro Thr Leu Leu Tyr Arg
1               5                   10                  15
Ser Cys Phe His Ile Ile Cys Leu Val Gly Thr Ile Ser Leu Ala Cys
            20                  25                  30
Asn Asp Met Thr Pro Glu Gln Met Ala Thr Asn Val Asn Cys Ser Ser
        35                  40                  45
Pro Glu Arg His Thr Arg Ser Tyr Asp Tyr Met Glu Gly Gly Asp Ile
    50                  55                  60
Arg Val Arg Arg Leu Phe Cys Arg Thr Gln Trp Tyr Leu Arg Ile Asp
65                  70                  75                  80
Lys Arg Gly Lys Val Lys Gly Thr Gln Glu Met Lys Asn Asn Tyr Asn
                85                  90                  95
Ile Met Glu Ile Arg Thr Val Ala Val Gly Ile Val Ala Ile Lys Gly
            100                 105                 110
Val Glu Ser Glu Phe Tyr Leu Ala Met Asn Lys Glu Gly Lys Leu Tyr
        115                 120                 125
Ala Lys Lys Glu Cys Asn Glu Asp Cys Asn Phe Lys Glu Leu Ile Leu
    130                 135                 140
Glu Asn His Tyr Asn Thr Tyr Ala Ser Ala Lys Trp Thr His Asn Gly
145                 150                 155                 160
Gly Glu Met Phe Val Ala Leu Asn Gln Lys Gly Ile Pro Val Arg Gly
                165                 170                 175
Lys Lys Thr Lys Lys Glu Gln Lys Thr Ala His Phe Leu Pro Met Ala
            180                 185                 190
Ile Thr

<210> 50
<211> 155
<212> PRT
<213> Homo sapiens

<220>
<223> [CDS]:1..468 from SEQ ID NO 18

<400> 50

<210> 51
<211> 155
<212> PRT
<213> Homo sapiens

<220>
<223> [CDS]:1..468 from SEQ ID NO 19

<400> 51

<210> 52
<211> 155
<212> PRT
<213> Homo sapiens

<220>
<223> [CDS]:1..468 from SEQ ID NO 20

<400> 52

<210> 53
<211> 155
<212> PRT
<213> Homo sapiens

<220>
<223> [CDS]:1..468 from SEQ ID NO 21

<400> 53

<210> 54
<211> 155
<212> PRT
<213> Homo sapiens

<220>
<223> [CDS]:1..468 from SEQ ID NO 22

<400> 54

<210> 55
<211> 155
<212> PRT
<213> Homo sapiens

<220>
<223> [CDS]:1..468 from SEQ ID NO 23

<400> 55

<210> 56
<211> 155
<212> PRT
<213> Homo sapiens

<220>
<223> [CDS]:1..468 from SEQ ID NO 24

<400> 56

<210> 57
<211> 155
<212> PRT
<213> Homo sapiens

<220>
<223> [CDS]:1..468 from SEQ ID NO 25

<400> 57

<210> 58
<211> 155
<212> PRT
<213> Homo sapiens

<220>
<223> [CDS]:1..468 from SEQ ID NO 26

<400> 58

<210> 59
<211> 155
<212> PRT
<213> Homo sapiens

<220>
<223> [CDS]:1..468 from SEQ ID NO 27

<400> 59

<210> 60
<211> 155
<212> PRT
<213> Homo sapiens

<220>
<223> [CDS]:1..468 from SEQ ID NO 28

<400> 60

<210> 61
<211> 155
<212> PRT
<213> Homo sapiens

<220>
<223> [CDS]:1..468 from SEQ ID NO 29

<400> 61